# EUROPEAN PATENT APPLICATION

(11) **EP 3 805 203 A1**
(43) Date of publication of application: **14.04.2021**
(21) Application number: 19815827.1
(22) Date of filing: 06.06.2019
(51) Int. Cl.: C07D 205/04, A61K 31/397, A61P 37/02, C07D 401/04, C07D 405/12, C07D 413/12, C07D 417/04

(54) **AZETIDINE DERIVATIVE, AND PRODRUG THEREOF**

(30) Priority: 07.06.2018 JP 2018109308
(71) Applicant: Daiichi Sankyo Company, Limited, Tokyo 103-8426 (JP)
(72) Inventor: MARUOKA, Hiroshi, Tokyo 103-8426 (JP); NOJI, Toshiharu, Tokyo 103-8426 (JP); MURAFUJI, Hidenobu, Tokyo 103-8426 (JP); TSUNEMI, Tomoyuki, Tokyo 103-8426 (JP); TAKAHASHI, Junko, Tokyo 103-8426 (JP); UESUGI, Shuhei, Tokyo 103-8426 (JP); TOMITA, Urara, Tokyo 103-8426 (JP)
(74) Representative: Fairbairn, Angus Chisholm
(86) International application number: PCT/JP2019/022435
(87) International publication number: WO 2019/235553

(57) **Abstract**

An object of the present invention is to provide a compound useful as a therapeutic or prophylactic drug for a disease involving the immune system, by suppressing a function of immune cells by suppressing proliferation of activated T cells or suppressing production of interferon alpha (IFN-α) by activated plasmacytoid dendritic cells (pDC), particularly an autoimmune disease such as systemic lupus erythematosus (SLE) and lupus nephritis in SLE patients. The present invention provides a compound represented by general formula (I) : [wherein X, R¹, R², R³, R⁴, R⁵ and R⁶ are as described in the description], or a pharmaceutically acceptable salt thereof.

## Description

### Technical Field

The present invention relates to a compound or a pharmaceutically acceptable salt thereof useful as a therapeutic or prophylactic agent for a disease involving the immune system, by suppressing a function of immune cells by suppressing proliferation of activated T cells or by suppressing production of interferon alpha (sometimes abbreviated herein as IFN-α or IFNa) by activated plasmacytoid dendritic cells (sometimes abbreviated herein as pDC), particularly an autoimmune disease such as systemic lupus erythematosus (sometimes abbreviated herein as SLE) and lupus nephritis in SLE patients.

### Background Art

SLE is a systemic autoimmune disease and it is said that there are approximately 1.4 million SLE patients worldwide. Among them, SLE is frequent in Asian and Hispanic peoples, and its prevalence is about 50 per 100,000 people in Japan. The ratio in males to females is one to nine, the overwhelming majority of SLE patients being women. The peak of the onset is at the age of 20's to 30's, early onset being one of characteristics of SLE. Since the matching rate of SLE in identical twins is about 30%, it is believed that environmental factors such as ultraviolet light, viral infection, stress, and sex hormones contribute to a background of some genetic predisposition to cause the onset.

SLE is classified in accordance with the SLICC classification criteria (the systemic lupus international collaborating clinics classification criteria for systemic lupus erythematosus) presented by the American College of Rheumatology in 2012. SLE is diagnosed when both one or more of 11 clinical items and of 6 immunological items, to a total of 4 items, are met. Major clinical symptoms include acute and chronic skin lupus, synovitis (joint swelling or joint pain and stiffness), neurological symptoms, and nephropathy. The main immunological items include elevated autoantibodies such as anti-nuclear antibodies, anti-dsDNA antibodies, and anti-Sm antibodies, and reduced complements in the blood.

The onset mechanism of SLE is elicited by the deposition of immunoconjugates composed of autoantibodies produced in the blood and autoantigens on vessel walls. It is known that the production of autoantibodies requires B cell activation and T cell function to assist it and that interferon alpha (sometimes abbreviated herein as IFN-α or IFNa) produced from plasmacytoid dendritic cells (sometimes abbreviated herein as pDC) also plays an important role in SLE pathology, and it is believed that SLE develops through a complex network of various immune cells.

Important organ disorders in SLE patients are disorders of kidney, central nerve, and lung, and they are likely to cause poor life and function prognosis. Among them, nephropathy occurs in 50% or more of SLE patients, with high probability to lead to renal failure and death, and these are organ disorders having very high unmet medical needs. Patients with nephropathy are diagnosed with lupus nephritis according to tissue classification by the International Society of Nephrology. Lupus nephritis is defined when the classification criteria for SLE are met, and persistent urinary protein is >0.5 g/day or 3+ or more, and/or cell cylinders are present. Lupus nephritis is classified into type I to type VI based on pathological diagnosis. In the active lupus nephritis of types III, IV, and V, it is believed that strong treatment with a combination of a large quantity of steroids and immunosuppressive drugs is required and it is important that remission status is achieved early. The first selective drug for introducing a remission of lupus nephritis is mycophenolate mofetil (sometimes abbreviated herein as MMF), which is used in about 70% of patients. For the remaining 20 to 30% of patients, cyclophosphamide pulse therapy is used. MMF is an inhibitor of inosine monophosphate dehydrogenase. The main action mechanism of MMF is to act on lymphocyte T and B cells whose purine synthesis depends on this enzyme and to suppress purine synthesis in these target cells. Cyclophosphamides also have a mechanism to inhibit DNA synthesis in lymphocytes. However, since these agents have a mechanism of inhibiting nucleic acid synthesis, the problem of side effects is serious. MMF has serious side effects such as teratogenicity (contraindicated in pregnant women), gastrointestinal disorders, and susceptibility to infection. Cyclophosphamide has side effects such as carcinogenicity, bone marrow disorders, nausea and vomiting, and reproductive dysfunction. Thus, existing medicaments have not always adequately worked for treatment due to dose and use restrictions due to side effects. In addition, even when these drugs are able to work adequately, the response rate is not always high. Thus, drugs with novel mechanisms of action and drugs with higher safety are required in patients and medical settings.

One drug that has been developed for SLE is an anti-IFN-α receptor antibody, and this drug has obtained good results in treatment response rates in Phase II trials (Non Patent Literature 1). IFN-α is also a cytokine that is characteristically enhanced in patients with SLE and is considered to be an important factor as a therapeutic target, and thus an agent that more effectively suppresses IFN-α is required. In a Phase II trial targeting patients with lupus nephritis, voclosporin, a calcineurin inhibitor (CNI) and T cell suppressor, showed a significant remission introduction rate relative to a control group. This demonstrates again the validity of T cells as a treatment target (Non Patent Literature 2).

### Citation List

### Non Patent Literature

Non Patent Literature 1; Arthritis Rheumatol. 2017 Feb;69(2):376-386
Non Patent Literature 2: Dobronravov V, Dooley M, Haq S, et al. LB0002 48 week complete remission of active lupus nephritis with voclosporin. Annals of the Rheumatic Diseases 2017;76:153.

### Summary of Invention

### Technical Problem

The present inventors have found compounds that provide both proliferation suppression of activated T cells and production suppression of IFN-α, and considered that this profile is highly ideal as a therapeutic agent for diseases involving the immune system, particularly autoimmune diseases such as SLE and lupus nephritis in SLE patients. The present inventors conducted intensive researches, and as a result, have found that azetidine derivatives having a specific chemical structure exhibit excellent proliferation suppression of activated T cells and production suppression of IFN-α, and are effective for treating diseases involving the immune system, particularly autoimmune diseases such as SLE and lupus nephritis in SLE patients, and have completed the present invention.

### Solution to Problem

The present invention relates to the following.
[1] A compound represented by general formula (I): 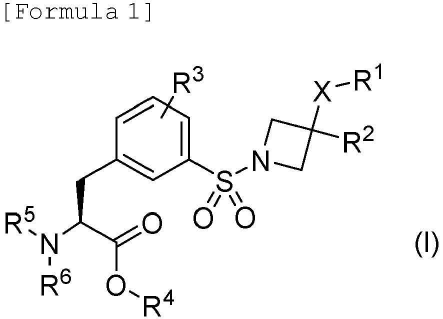 wherein
   X indicates a C₁-C₃ alkylene group, an oxygen atom, a sulfur atom, a group represented by formula -NH-, a group represented by formula -N(C₁-C₃ alkyl)-, or a single bond;
   R¹ indicates a C₃-C₆ cycloalkyl group, a phenyl group, or an aromatic heterocyclic ring group,
   wherein the aromatic heterocyclic ring is a 5- to 6-membered aromatic heterocyclic ring having in the ring 1 to 4 heteroatoms independently selected from an oxygen atom, a sulfur atom, and a nitrogen atom, and
   the phenyl group or the aromatic heterocyclic ring group may have 1 to 5 substituents independently selected from substituent group a below;
   R² indicates a hydrogen atom, a C₁-C₆ alkyl group, a C₁-C₆ alkyl group substituted with 1 to 3 halogen atoms, a C₃-C₆ cycloalkyl group, a phenyl group, or an aromatic heterocyclic ring group,
   wherein the aromatic heterocyclic ring is a 5- to 6-membered aromatic heterocyclic ring having in the ring 1 to 4 heteroatoms independently selected from an oxygen atom, a sulfur atom, and a nitrogen atom, and
   the phenyl group or the aromatic heterocyclic ring group may have 1 to 5 substituents independently selected from substituent group b below;
   R³ indicates a hydrogen atom or a halogen atom;
   R⁴ indicates a hydrogen atom, a C₁-C₆ alkyl group, a C₃-C₆ cycloalkyl group, or is taken together with R⁵ or R⁶ to represent a C₁-C₆ alkylene group,
   wherein the C₁-C₆ alkyl group, the C₃-C₆ cycloalkyl group, and the C₁-C₆ alkylene group may have 1 to 3 substituents independently selected from substituent group c below;
   R⁵ and R⁶ each independently indicate a hydrogen atom, or a group represented by formula A: 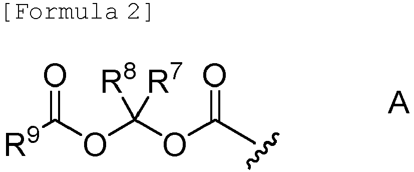
   wherein
   R⁷ and R⁸ each independently indicate a hydrogen atom, or a C₁-C₆ alkyl group;
   R⁹ indicates a C₁-C₆ alkyl group, a C₃-C₆ cycloalkyl group, a phenyl group, or a heterocyclic ring group,
   wherein the heterocyclic ring shows a saturated or unsaturated 5- to 6-membered heterocyclic ring having in the ring 1 to 4 heteroatoms selected from an oxygen atom, a sulfur atom, and a nitrogen atom, and
   the C₁-C₆ alkyl group, the C₃-C₆ cycloalkyl group, the phenyl group, or the heterocyclic ring group may have 1 to 3 substituents independently selected from the substituent group c below,
   substituent group a: a halogen atom, a C₁-C₆ alkyl group, a C₁-C₆ alkyl group substituted with 1 to 3 halogen atoms, a cyano group, a carbamoyl group, a deuterium atom, and a phenyl group, wherein the phenyl group is optionally substituted with 1 to 3 substituents independently selected from the group consisting of a C₁-C₆ alkyl group and a halogen atom;
   substituent group b: a halogen atom and a deuterium atom;
   substituent group c: a halogen atom, a C₁-C₆ alkyloxy group, a C₃-C₆ cycloalkyloxy group, a C₁-C₆ alkyloxy group substituted with 1 to 3 halogen atoms, a hydroxyl group, a C₁-C₇ acyl group, an amino group, a mono-C₁-C₆ alkylamino group, a di-(C₁-C₆ alkyl)amino group, a morpholino group, a cyano group, a carbamoyl group, a mono-C₁-C₆ alkylcarbamoyl group, a di-(C₁-C₆ alkyl)carbamoyl group, a phenyl group, and a medoxomil group (5-methyl-2-oxo-1,3-dioxol-4-yl group), or a pharmaceutically acceptable salt thereof.
[2] The compound according to [1] or a pharmaceutically acceptable salt thereof, wherein, in the general formula (I), X indicates a C₁-C₃ alkylene group, an oxygen atom, or a single bond.
[3] The compound according to [1] or [2] or a pharmaceutically acceptable salt thereof, wherein, in the general formula (I), R¹ represents a C₃-C₆ cycloalkyl group, a phenyl group, or a pyridyl group, wherein the phenyl group and the pyridyl group may have 1 to 5 substituents independently selected from the substituent group a.
[4] The compound according to any one of [1] to [3] or a pharmaceutically acceptable salt thereof, wherein, in the general formula (I), R² indicates a hydrogen atom, a C₁-C₆ alkyl group, a C₁-C₆ alkyl group substituted with 1 to 3 halogen atoms, a C₃-C₆ cycloalkyl group, a phenyl group which may have 1 to 5 substituents independently selected from the substituent group b, an unsubstituted pyridyl group, or an unsubstituted thiazolyl group.
[5] The compound according to any one of [1] to [4] or a pharmaceutically acceptable salt thereof, wherein, in the general formula (I), R³ indicates a hydrogen atom.
[6] The compound according to any one of [1] to [5] or a pharmaceutically acceptable salt thereof, wherein, in the general formula (I), R⁴ indicates a hydrogen atom or a C₁-C₆ alkyl group which may have 1 to 3 substituents independently selected from substituent group c1 below, or is taken together with R⁵ or R⁶ to represent a methylene, methylmethylene, dimethylmethylene or isopropylmethylene group,
   substituent group c1: a hydroxyl group, a mono-C₁-C₆ alkylamino group, a di-(C₁-C₆ alkyl) amino group, a morpholino group, and a medoxomil group (5-methyl-2-oxo-1,3-dioxol-4-yl group).
[7] The compound according to any one of [1] to [6] or a pharmaceutically acceptable salt thereof, wherein, in the general formula (I), one of R⁵ and R⁶ is a hydrogen atom and the other is formula A, or both R⁵ and R⁶ are hydrogen atoms.
[8] The compound according to any one of [1] to [7] or a pharmaceutically acceptable salt thereof, wherein, in the general formula (I), one of R⁵ and R⁶ is a hydrogen atom and the other is formula A, or both R⁵ and R⁶ are hydrogen atoms, and one of R⁷ and R⁸ in the formula A is a hydrogen atom and the other is a C₁-C₆ alkyl group.
[9] The compound according to any one of [1] to [8] or a pharmaceutically acceptable salt thereof, wherein, in the general formula (I), one of R⁵ and R⁶ is a hydrogen atom and the other is formula A, or both R⁵ and R⁶ are hydrogen atoms, wherein R⁹ in the formula A indicates a C₁-C₆ alkyl group, a C₃-C₆ cycloalkyl group, a 4-oxanyl group, or a 3-oxanyl group.
[10] The compound according to [1] or a pharmaceutically acceptable salt thereof, wherein, in the general formula (I),
   X indicates an oxygen atom;
   R¹ indicates a phenyl group which may have 1 to 2 substituents independently selected from substituent group a1:
   substituent group a1: a halogen atom, a C₁-C₆ alkyl group, and a C₁-C₆ alkyl group substituted with 1 to 3 halogen atoms;
   R² indicates a phenyl group optionally substituted with 1 to 2 halogen atoms;
   R³ indicates a hydrogen atom;
   R⁴ indicates a hydrogen atom;
   one of R⁵ and R⁶ is a hydrogen atom and the other is formula A, or both R⁵ and R⁶ are hydrogen atoms,
   wherein one of R⁷ and R⁸ in the formula A indicates a hydrogen atom, the other indicates a C₁-C₃ alkyl group, and R⁹ indicates a C₁-C₆ alkyl group,
   or a pharmaceutically acceptable salt thereof.
[11] The compound according to [1] or a pharmaceutically acceptable salt thereof, wherein, in the general formula (I),
   X indicates an oxygen atom;
   R¹ indicates a 3-fluorophenyl group;
   R² indicates an unsubstituted phenyl group;
   R³ indicates a hydrogen atom;
   R⁴ indicates a hydrogen atom or a C₁-C₆ alkyl group which may have 1 to 3 substituents independently selected from substituent group c1 below, or is taken together with R⁵ or R⁶ to represent a methylene, methylmethylene, dimethylmethylene or isopropylmethylene group,
   substituent group c1: a hydroxyl group, a mono-C₁-C₆ alkylamino group, a di-(C₁-C₆) amino group, a morpholino group, and a medoxomil group (5-methyl-2-oxo-1,3-dioxol-4-yl group);
   one of R⁵ and R⁶ is a hydrogen atom and the other is formula A, or both R⁵ and R⁶ are hydrogen atoms,
   wherein one of R⁷ and R⁸ in the formula A indicates a hydrogen atom and the other indicates a C₁-C₆ alkyl group, and R⁹ indicates a C₁-C₆ alkyl group, a C₃-C₆ cycloalkyl group, a 4-oxanyl group, or a 3-oxanyl group, or a pharmaceutically acceptable salt thereof.
[12] The compound according to [1] or a pharmaceutically acceptable salt thereof, wherein the compound is any one selected from the following group:
   (S)-2-Amino-3-(3-((3-(3-fluorophenoxy)-3-(4-fluorophenyl)azetidin-1-yl)sulfonyl)phenyl)propanoic acid;
   (S)-2-Amino-3-(3-((3-(4-fluorophenoxy)-3-phenylazetidin-1-yl)sulfonyl)phenyl)propanoic acid;
   (S)-2-Amino-3-(3-((3-(3-fluorophenoxy)-3-phenylazetidin-1-yl)sulfonyl)phenyl)propanoic acid;
   (2S)-3-(3-((3-(4-fluorophenoxy)-3-phenylazetidin-1-yl)sulfonyl)phenyl)-2-(((1-(pivaloyloxy)ethoxy)carbonyl)amino)propanoic acid;
   (S)-2-((((R)-1-acetoxyethoxy)carbonyl)amino)-3-(3-((3-(3-fluorophenoxy)-3-phenylazetidin-1-yl)sulfonyl)phenyl)propanoic acid;
   (S)-2-((((R)-1-acetoxyethoxy)carbonyl)amino)-3-(3-((3-(4-fluorophenoxy)-3-phenylazetidin-1-yl)sulfonyl)phenyl)propanoic acid;
   (S)-2-((((S)-1-acetoxyethoxy)carbonyl)amino)-3-(3-((3-(4-fluorophenoxy)-3-phenylazetidin-1-yl)sulfonyl)phenyl)propanoic acid;
   (S)-3-(3-((3-(3-fluorophenoxy)-3-phenylazetidin-1-yl)sulfonyl)phenyl)-2-((((R)-1-(pivaloyloxy)ethoxy)carbonyl)amino)propanoic acid;
   (S)-3-(3-((3-(3-fluorophenoxy)-3-phenylazetidin-1-yl)sulfonyl)phenyl)-2-((((S)-1-(pivaloyloxy)ethoxy)carbonyl)amino)propanoic acid;
   (S)-3-(3-((3-(3-fluorophenoxy)-3-(4-fluorophenyl)azetidin-1-yl)sulfonyl)phenyl)-2-((((R)-1-(pivaloyloxy)ethoxy)carbonyl)amino)propanoic acid;
   (S)-2-((((S)-1-acetoxyethoxy)carbonyl)amino)-3-(3-((3-(3-fluorophenoxy)-3-phenylazetidin-1-yl)sulfonyl)phenyl)propanoic acid;
   (S)-3-(3-((3-(4-fluorophenoxy)-3-phenylazetidin-1-yl)sulfonyl)phenyl)-2-((((S)-1-(isobutyryloxy)ethoxy)carbonyl)amino)propanoic acid;
   (S)-3-(3-((3-(4-fluorophenoxy)-3-phenylazetidin-1-yl)sulfonyl)phenyl)-2-((((R)-1-(isobutyryloxy)ethoxy)carbonyl)amino)propanoic acid;
   (S)-3-(3-((3-(3-fluorophenoxy)-3-phenylazetidin-1-yl)sulfonyl)phenyl)-2-((((R)-1-(isobutyryloxy)ethoxy)carbonyl)amino)propanoic acid; and
   (S)-3-(3-((3-(3-fluorophenoxy)-3-phenylazetidin-1-yl)sulfonyl)phenyl)-2-((((S)-1-(isobutyloxy)ethoxy)carbonyl)amino)propanoic acid.
[13] The compound according to [1] or a pharmaceutically acceptable salt thereof, wherein the compound is (S)-2-amino-3-(3-((3-(4-fluorophenoxy)-3-phenylazetidin-1-yl)sulfonyl)phenyl)propanoic acid.
[14] The compound according to [1] or a pharmaceutically acceptable salt thereof, wherein the compound is (S)-2-amino-3-(3-((3-(3-fluorophenoxy)-3-phenylazetidin-1-yl)sulfonyl)phenyl)propanoic acid.
[15] The compound according to [1] or a pharmaceutically acceptable salt thereof, wherein the compound is (2S)-3-(3-((3-(4-fluorophenoxy)-3-phenylazetidin-1-yl)sulfonyl)phenyl)-2-(((1-(pivaloyloxy)ethoxy)carbonyl)amino)propanoic acid.
[16] The compound according to [1] or a pharmaceutically acceptable salt thereof, wherein the compound is (S)-2-((((R)-1-acetoxyethoxy)carbonyl)amino)-3-(3-((3-(3-fluorophenoxy)-3-phenylazetidin-1-yl)sulfonyl)phenyl)propanoic acid.
[17] The compound according to [1] or a pharmaceutically acceptable salt thereof, wherein the compound is (S)-3-(3-((3-(3-fluorophenoxy)-3-phenylazetidin-1-yl)sulfonyl)phenyl)-2-((((S)-1-(pivaloyloxy)ethoxy)carbonyl)amino)propanoic acid.
[18] The compound according to [1] or a pharmaceutically acceptable salt thereof, wherein the compound is (S)-3-(3-((3-(4-fluorophenoxy)-3-phenylazetidin-1-yl)sulfonyl)phenyl)-2-((((S)-1-(isobutyryloxy)ethoxy)carbonyl)amino)propanoic acid.
[19] The compound according to [1] or a pharmaceutically acceptable salt thereof, wherein the compound is (S)-3-(3-((3-(4-fluorophenoxy)-3-phenylazetidin-1-yl)sulfonyl)phenyl)-2-((((R)-1-(isobutyryloxy)ethoxy)carbonyl)amino)propanoic acid.
[20] A medicament comprising the compound according to any one of [1] to [19] or a pharmaceutically acceptable salt thereof.
[21] An activated T cell-proliferation suppressor comprising the compound according to any one of [1] to [19] or a pharmaceutically acceptable salt thereof.
[22] An interferon α-production suppressor comprising the compound according to any one of [1] to [19] or a pharmaceutically acceptable salt thereof.
[23] A therapeutic or prophylactic agent for a disease whose symptoms are prevented, ameliorated or reduced by suppressing proliferation of activated T cells, or suppressing production of interferon α by activated plasmacytoid dendritic cells (pDC), the agent comprising the compound according to any one of [1] to [19] or a pharmaceutically acceptable salt thereof.
[24] A method for treating or preventing a disease whose symptoms are prevented, ameliorated or reduced by suppressing proliferation of activated T cells, or suppressing production of interferon α by activated plasmacytoid dendritic cells (pDC), the method comprising administering the compound according to any one of [1] to [19] or a pharmaceutically acceptable salt thereof to a patient.
[25] The compound according to any one of [1] to [19] or a pharmaceutically acceptable salt thereof for use in the treatment or prevention of a disease whose symptoms are prevented, ameliorated or reduced by suppressing proliferation of activated T cells, or suppressing production of interferon α by activated plasmacytoid dendritic cells (pDC).
[26] The therapeutic or prophylactic agent according to [23], wherein the disease is an autoimmune disease.
[27] The method for treating or preventing according to [24], wherein the disease is an autoimmune disease.
[28] The compound or a pharmaceutically acceptable salt according to [25], wherein the disease is an autoimmune disease.
[29] A therapeutic or prophylactic agent for systemic lupus erythematosus; a renal lesion, a central nerve disorder, a pulmonary disorder or vasculitis due to systemic lupus erythematosus; multiple myositis; ulcerative colitis; Sjogren's syndrome; graft-versus-host disease (GVHD) or psoriasis, the agent comprising the compound according to any one of [1] to [19] or a pharmaceutically acceptable salt thereof.
[30] A method for treating or preventing systemic lupus erythematosus; a renal lesion, a central nerve disorder, a pulmonary disorder or vasculitis due to systemic lupus erythematosus; multiple myositis; ulcerative colitis; Sjogren's syndrome; graft-versus-host disease (GVHD) or psoriasis, the method comprising administering the compound according to any one of [1] to [19] or a pharmaceutically acceptable salt thereof to a patient.
[31] The compound according to any one of [1] to [19] or a pharmaceutically acceptable salt thereof for use in the treatment or prevention of systemic lupus erythematosus; a renal lesion, a central nerve disorder, a pulmonary disorder or vasculitis due to systemic lupus erythematosus; multiple myositis; ulcerative colitis; Sjogren's syndrome; graft-versus-host disease (GVHD) or psoriasis.

In the present invention, the "halogen atom" is a fluorine atom, a chlorine atom, a bromine atom, or an iodine atom. Suitably, the "halogen atom" is a fluorine atom or a chlorine atom.

In the present invention, the "C₁-C₆ alkyl group" is a linear or branched alkyl group having 1 to 6 carbon atoms. Examples thereof include a methyl, ethyl, propyl, isopropyl, butyl, isobutyl, s-butyl, t-butyl, pentyl, isopentyl, 2-methylbutyl, neopentyl, 1-ethylpropyl, hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, or 1,2-dimethylbutyl group. The "C₁-C₆ alkyl group" is suitably a linear or branched alkyl group having 1 to 4 carbon atoms (C₁-C₄ alkyl group), more suitably a methyl group or an ethyl group (C₁-C₂ alkyl group), even more suitably a methyl group (C₁ alkyl group) .

In the present invention, the "C₁-C₃ alkyl group" is a linear or branched alkyl group having 1 to 3 carbon atoms. Examples thereof include a methyl, ethyl, propyl, or isopropyl group. The "C₁-C₃ alkyl group" is suitably a methyl group or an ethyl group (C₁-C₂ alkyl group), even more suitably, a methyl group (C₁ alkyl group).

In the present invention, the "C₃-C₆ cycloalkyl group" is a cyclopropyl group, a cyclobutyl group, a cyclopentyl group, or a cyclohexyl group. The "C₃-C₆ cycloalkyl group" is suitably a cyclopropyl group or a cyclohexyl group.

In the present invention, the "C₁-C₆ alkyl group substituted with 1 to 3 halogen atoms" is a group in which the same or different 1 to 3 "halogen atoms" described above are attached to the "C₁-C₆ alkyl group" described above. Examples thereof include a fluoromethyl, difluoromethyl, trifluoromethyl, trichloromethyl, 2,2,2-trifluoroethyl, 3,3,3-trifluoropropyl, or 2,2,2-trichloroethyl group. The "C₁-C₆ alkyl group substituted with 1 to 3 halogen atoms" is suitably a group in which the same or different 3 "halogen atoms" are attached to the "C₁-C₂ alkyl group" (trihalo C₁-C₂ alkyl group), more suitably a trihalomethyl group (trihalo C₁ alkyl group), and even more suitably a trifluoromethyl group.

In the present invention, the "C₁-C₃ alkylene group" is a linear or branched alkylene group having 1 to 3 carbon atoms. Examples thereof include a methylene, ethylene, propylene, or isopropylene group. The "C₁-C₃ alkylene group" is suitably a methylene group or an ethylene group (C₁-C₂ alkylene group), even more suitably a methylene group (C₁ alkylene group).

In the present invention, the "C₁-C₆ alkylene group" is a linear or branched alkylene group having 1 to 6 carbon atoms. Examples thereof include a methylene, ethylene, methylmethylene, propylene, dimethylmethylene (isopropylene), butylene, isopropylmethylene (isobutylene), s-butylene, t-butylene, pentylene, isopentylene, 2-methylbutylene, neopentylene, 1-ethylpropylene, hexylene, isohexylene, 4-methylpentylene, 3-methylpentylene, 2-methylpentylene, 1-methylpentylene, 3,3-dimethylbutylene, 2,2-dimethylbutylene, 1,1-dimethylbutylene, or 1,2-dimethylbutylene group. The "C₁-C₆ alkylene group" is suitably a C₁-C₄ alkylene group, such as a methylene, ethylene, methylmethylene, dimethylmethylene (isopropylene) or isopropylmethylene (isobutylene) group, even more suitably, a methylene group (C₁ alkylene group).

In the present invention, "R⁴ is taken together with R⁵ or R⁶ to represent a C₁-C₆ alkylene group" means that the oxygen atom to which R⁴ is attached and the nitrogen atom to which R⁵ or R⁶ is attached are crosslinked with a C₁-C₆ alkylene group to form a ring. The ring has suitably 5 to 7 members, more suitably 5 members. More specifically, suitable examples of the ring include 5-oxooxazolidine, 2-methyl-5-oxazolidine, 2-isopropyl-5-oxazolidine or 2,2-dimethyl-5-oxazolidine.

In the present invention, the "C₁-C₆ alkyloxy group" is a group in which the "C₁-C₆ alkyl group" is attached to an oxygen atom. Examples thereof include a methoxy, ethoxy, propoxy, isopropoxy, butoxy, isobutoxy, s-butoxy, t-butoxy, pentoxy, 2-methylbutoxy, 3-ethylpropoxy, neopentoxy, hexyloxy, or 2,3-dimethylbutoxy group. The "C₁-C₆ alkyloxy group" is suitably a linear or branched alkoxy group having 1 to 4 carbon atoms (C₁-C₄ alkoxy group), more suitably a methoxy group or an ethoxy group (C₁-C₂ alkoxy group), even more suitably a methoxy group (C₁ alkoxy group).

In the present invention, the "C₁-C₆ alkyloxy group substituted with 1 to 3 halogen atoms" is a group in which the same or different 1 to 3 "halogen atoms" described above are attached to the "C₁-C₆ alkyloxy group" described above. Examples thereof include a fluoromethyloxy, difluoromethyloxy, trichloromethyloxy, 2,2,2-trifluoroethyloxy, 3,3,3-trifluoropropyloxy, or 2,2,2-trichloroethyloxy group. The "C₁-C₆ alkyloxy group substituted with 1 to 3 halogen atoms" is suitably a group in which the same or different 3 "halogen atoms" are attached to the "C₁-C₂ alkyloxy group" (trihalo C₁-C₂ alkyloxy group), more suitably a trihalomethyloxy group (trihalo C₁ alkyloxy group), even more suitably a trifluoromethyloxy group.

In the present invention, the "C₃-C₆ cycloalkyloxy group" is a group in which the "C₃-C₆ cycloalkyl group" described above is attached to an oxygen atom and is a cyclopropyloxy group, a cyclobutyloxy group, a cyclopentyloxy group, or a cyclohexyloxy group. The "C₃-C₆ cycloalkyloxy group" is suitably a cyclopropyloxy group or a cyclohexyloxy group.

In the present invention, the "C₁-C₇ acyl group" is a group in which a hydrogen atom or the "C₁-C₆ alkyl group" described above is attached to a carbonyl group. Examples thereof include a formyl, acetyl, propionyl, butyryl, isobutyryl, valeryl, isovaleryl, pivaloyl, hexanoyl, or heptanoyl group. The "C₁-C₇ acyl group" is suitably an acetyl group or a propionyl group (C₂-C₃ acyl group) .

In the present invention, the "mono-C₁-C₆ alkylamino group" is an amino group to which one "C₁-C₆ alkyl group" described above is attached. Examples thereof include a methylamino, ethylamino, propylamino, isopropylamino, or butylamino group. The "mono-C₁-C₆ alkylamino group" is suitably an amino group to which one "C₁-C₄ alkyl group" is attached (mono-C₁-C₄ alkylamino group), more suitably a methylamino or an ethylamino group (mono-C₁-C₂ alkylamino group).

In the present invention, the "di-(C₁-C₆ alkyl) amino group" is an amino group to which the same or different two "C₁-C₆ alkyl groups" described above are attached. Examples thereof include a dimethylamino, diethylamino, dipropylamino, N-ethyl-N-methylamino, N-methyl-N-propylamino or N-butyl-N-methylamino group. The "di-(C₁-C₆ alkyl)amino group" is suitably an amino group to which the same or different two "C₁-C₄ alkyl groups" are attached (di-(C₁-C₄ alkyl)amino group), more suitably a dimethylamino group, a diethylamino group or an N-ethyl-N-methylamino group (di-(C₁-C₂ alkyl)amino group), even more suitably a dimethylamino group.

In the present invention, the "mono-C₁-C₆ alkylcarbamoyl group" is a carbamoyl group to which one "C₁-C₆ alkyl group" described above is attached. Examples thereof include a methylcarbamoyl, ethylcarbamoyl, propylcarbamoyl, isopropylcarbamoyl, or butylcarbamoyl group. The "mono-C₁-C₆ alkylcarbamoyl group" is suitably a carbamoyl group to which one "C₁-C₄ alkyl group" is attached (mono-C₁-C₄ alkylcarbamoyl group), more suitably a methylcarbamoyl group or an ethylcarbamoyl group (mono-C₁-C₂ alkylcarbamoyl group).

In the present invention, the "di-(C₁-C₆ alkyl)carbamoyl group" is a carbamoyl group to which the same or different two "C₁-C₆ alkyl groups" described above are attached. Examples thereof include a dimethylcarbamoyl, diethylcarbamoyl, dipropylcarbamoyl, N-ethyl-N-methylcarbamoyl, N-methyl-N-propylcarbamoyl or N-butyl-N-methylcarbamoyl group. The "di-(C₁-C₆ alkyl)carbamoyl group" is suitably a carbamoyl group to which the same or different two "C₁-C₄ alkyl groups" are attached (di-(C₁-C₄ alkyl)carbamoyl group), more suitably a dimethylcarbamoyl group, a diethyl carbamoyl group or a N-ethyl-N-methylcarbamoyl group (di-(C₁-C₂ alkyl)carbamoyl group), even more suitably a dimethylcarbamoyl group.

In the present invention, the "5- to 6-membered aromatic heterocyclic ring having in the ring 1 to 4 heteroatoms independently selected from an oxygen atom, a sulfur atom, and a nitrogen atom" indicates a 5- or 6-membered monocyclic aromatic compound containing 1 to 4 heteroatoms (nitrogen atom, oxygen atom, or sulfur atom) in addition to a carbon atom as constituent atoms of the ring. Examples thereof include pyrrole, pyrazole, imidazole, furan, thiophene, oxazole, isoxazole, thiazole, isothiazole, pyridine, pyrimidine, pyridazine, pyrazine, triazine, pyran, 1,2-oxazine, 1,3-oxazine, 1,4-oxazine, 1,2-thiazine, 1,3-thiazine, and 1,4-thiazine. The "5- to 6-membered aromatic heterocyclic ring having in the ring 1 to 4 heteroatoms independently selected from an oxygen atom, a sulfur atom, and a nitrogen atom" is suitably a 5- or 6-membered monocyclic aromatic compound containing 1 to 2 heteroatoms (nitrogen atom, oxygen atom, or sulfur atom) in the ring, more suitably pyridine or thiazole.

In the present invention, the "saturated or unsaturated 5- to 6-membered heterocyclic ring having in the ring 1 to 4 heteroatoms selected from an oxygen atom, a sulfur atom, and a nitrogen atom" indicates a 5- or 6-membered monocyclic compound containing 1 to 4 heteroatoms (nitrogen atom, oxygen atom, or sulfur atom) in addition to a carbon atom as constituent atoms of the ring. Examples thereof include pyrrolidine, pyrroline, imidazolidine, imidazoline, pyrazolidine, pyrazoline, piperidine, piperazine, oxolane, dioxolane, oxane, dioxane, morpholine, pyrrole, pyrazole, imidazole, furan, thiophene, oxazole, isoxazole, thiazole, isothiazole, pyridine, pyrimidine, pyridazine, pyrazine, triazine, pyran, 1,2-oxazine, 1,3-oxazine, 1,4-oxazine, 1,2-thiazine, 1,3-thiazine, and 1,4-thiazine. The "saturated or unsaturated 5- to 6-membered heterocyclic ring having in the ring 1 to 4 heteroatoms selected from an oxygen atom, a sulfur atom, and a nitrogen atom" is suitably a saturated 5- or 6-membered monocyclic compound containing 1 to 2 heteroatoms (nitrogen atom, oxygen atom, or sulfur atom) in the ring, more suitably oxane.

In the present invention, a suitable X is a C₁-C₃ alkylene group, an oxygen atom, or a single bond.

In the present invention, a suitable R¹ is a C₃-C₆ cycloalkyl group, or a phenyl group or a pyridyl group which may have 1 to 5 substituents independently selected from the substituent group a.

Herein, suitable examples of the substituent group a include a group independently selected from a fluorine atom, a chlorine atom, a bromine atom, a methyl group, a trifluoromethyl group, a cyano group, a carbamoyl group, a phenyl group, wherein the phenyl group is optionally substituted with 1 to 3 substituents independently selected from the group consisting of a methyl group, a fluorine atom, and a chlorine atom.

A more suitable R¹ is a phenyl group which may have 1 to 2 substituents independently selected from the substituent group a1 below.

Substituent group a1: a halogen atom (suitably a fluorine atom, a chlorine atom, a bromine atom), a C₁-C₆ alkyl group (suitably a methyl group), and a C₁-C₆ alkyl group substituted with 1 to 3 halogen atoms (suitably a trifluoromethyl group).

An even more suitable R¹ is an unsubstituted phenyl group or a phenyl group substituted with one fluorine atom.

In the present invention, a suitable R² is a hydrogen atom, a C₁-C₆ alkyl group, a C₁-C₆ alkyl group substituted with 1 to 3 halogen atoms, a C₃-C₆ cycloalkyl group, a phenyl group which may have 1 to 5 substituents independently selected from the substituent group b, an unsubstituted pyridyl group, or an unsubstituted thiazolyl group.

Herein, suitable examples of the substituent group b include a fluorine atom, a chlorine atom, or a deuterium atom.

A more suitable R² is a phenyl group which is optionally substituted with 1 to 2 halogen atoms. An even more suitable R² is an unsubstituted phenyl group or a phenyl group substituted with one fluorine atom.

In the present invention, a suitable R³ is a hydrogen atom, a fluorine atom or a chlorine atom, more suitably a hydrogen atom.

In the present invention, a suitable R⁴ is a hydrogen atom or a C₁-C₆ alkyl group which may have 1 to 3 substituents independently selected from the substituent group c1 below, or a group in which R⁴ is taken together with R⁵ or R⁶ to represent a methylene, methylmethylene, dimethylmethylene (isopropylene) or isopropylmethylene (isobutylene) group.

Substituent group c1: a hydroxyl group, a mono-C₁-C₆ alkylamino group (suitably, a methylamino group), a di-(C₁-C₆)amino group (suitably a dimethylamino group), a morpholino group, and a medoxomil group (5-methyl-2-oxo-1,3-dioxol-4-yl group).

A more suitable R⁴ is a hydrogen atom, a methyl group, an ethyl group, a propyl group, a hydroxymethyl group, a hydroxyethyl group, a morpholinomethyl group, a morpholinoethyl group, a dimethylaminomethyl group, a dimethylaminoethyl group, a medoxomilmethyl group, a medoxomilethyl group, or a group in which R⁴ is taken together with R⁵ or R⁶ to represent a methylene, methylmethylene, dimethylmethylene (isopropylene) or isopropylmethylene (isobutylene) group.

A more suitable R⁴ is a hydrogen atom.

It is preferred in the present invention that one or both of R⁵ and R⁶ are hydrogen atoms.

In the present invention, when one of R⁵ and R⁶ indicates formula A, it is preferred that one of R⁷ and R⁸ is a hydrogen atom and the other is a C₁-C₆ alkyl group. More suitably, one of R⁷ and R⁸ is a hydrogen atom and the other is a methyl group.

In the present invention, when one of R⁵ and R⁶ indicates formula A, R⁹ is preferably a C₁-C₆ alkyl group, a C₃-C₆ cycloalkyl group, a 4-oxanyl group, or a 3-oxanyl group. A more suitable R⁹ is a C₁-C₆ alkyl group, even more suitably a methyl group, an ethyl group, a propyl group, an isopropyl group, a butyl group, a s-butyl group or a t-butyl group (C₁-C₄ alkyl group).

In the present invention, examples of preferred combinations of the substituents in the formula (I) include the following aspects A and B.
Aspect A:
   X indicates an oxygen atom;
   R¹ indicates a phenyl group which may have 1 to 2 substituents independently selected from the substituent group a1:
      substituent group a1: a halogen atom, a C₁-C₆ alkyl group, and a C₁-C₆ alkyl group substituted with 1 to 3 halogen atoms;
      R² indicates a phenyl group optionally substituted with 1 to 2 halogen atoms;
      R³ indicates a hydrogen atom;
      R⁴ indicates a hydrogen atom;
      one of R⁵ and R⁶ is a hydrogen atom and the other is formula A, or both R⁵ and R⁶ are hydrogen atoms,
      wherein one of R⁷ and R⁸ in the formula A indicates a hydrogen atom, the other indicates a C₁-C₃ alkyl group, and R⁹ indicates a C₁-C₆ alkyl group.
Aspect B:
   X indicates an oxygen atom;
   R¹ indicates a 3-fluorophenyl group;
   R² indicates an unsubstituted phenyl group;
   R³ indicates a hydrogen atom;
   R⁴ indicates a hydrogen atom or a C₁-C₆ alkyl group which may have 1 to 3 substituents independently selected from the substituent group c1 below, or is taken together with R⁵ or R⁶ to represent a methylene, methylmethylene, dimethylmethylene or isopropylmethylene group,
   substituent group c1: a hydroxyl group, a mono-C₁-C₆ alkylamino group, a di-(C₁-C₆ alkyl)amino group, a morpholino group, and a medoxomil group (5-methyl-2-oxo-1,3-dioxol-4-yl group);
   one of R⁵ and R⁶ is a hydrogen atom and the other is formula A, or both R⁵ and R⁶ are hydrogen atoms,
   wherein one of R⁷ and R⁸ in the formula A indicates a hydrogen atom and the other indicates a C₁-C₆ alkyl group, and R⁹ indicates a C₁-C₆ alkyl group, a C₃-C₆ cycloalkyl group, a 4-oxanyl group, or a 3-oxanyl group.

In the general formula (I), a compound in which any one of R⁴, R⁵, and R⁶ is not a hydrogen atom (sometimes herein referred to as prodrug compound) is a prodrug, and is metabolized in vivo to a compound wherein all of R⁴, R⁵, and R⁶ are hydrogen atoms (sometimes herein referred to as active compound) to exhibit a pharmaceutical effect (see Test Example 6).

The compound represented by general formula (I) of the present invention or a pharmaceutically acceptable salt thereof may have all isomers (keto-enol isomers, diastereoisomers, optical isomers, rotary isomers, or the like).

The compound represented by general formula (I) of the present invention or a pharmaceutically acceptable salt thereof may have various isomers when an asymmetric carbon atom is present in the molecule. In the compound of the present invention, these isomers and mixtures of these isomers are all represented by a single formula, i.e., general formula (I). Thus, the present invention includes all of these isomers and mixtures of these isomers at any proportion. However, in general formula (I), the carbon atom to which R⁴-O-C(=O)- is attached shows a conformational structure as indicated by the formula.

The stereoisomers as described above can be obtained by using an optically active raw material compound, or by synthesizing the compound according to the present invention by methods of asymmetric synthesis or asymmetric induction, or by isolating the synthesized compound according to the present invention as desired by conventional optical resolution or separation methods.

The compound represented by general formula (I) of the present invention or a pharmaceutically acceptable salt thereof may also contain an atomic isotope at a non-natural proportion in one or more of the atoms that constitute such compounds. Examples of the atomic isotope include deuterium (²H), tritium (³H), iodine-125 (¹²⁵I), and carbon-14 (¹⁴C) . Furthermore, the compound may be radiolabeled with radioisotopes such as tritium (³H), iodine-125 (¹²⁵I) or carbon-14 (¹⁴C) . The radiolabeled compounds are useful as therapeutic or prophylactic agents, research reagents such as assay reagents, and diagnostic agents such as in vivo imaging agents. All isotopic variants of the compound of the present invention, whether they are radioactive or not, are encompassed within the scope of the present invention.

The term "pharmaceutically acceptable salt thereof" refers to a salt which is not significantly toxic and can be used as a medicament. The compound represented by general formula (I) of the present invention can be converted to a salt by reacting with an acid when the compound has a basic group, or with a base when the compound has an acidic group.

Examples of the salt based on the basic group include, but are not limited to, inorganic acid salts such as hydrohalides including hydrofluorides, hydrochlorides, hydrobromides, and hydroiodides, nitrates, perchlorates, sulfates and phosphates; organic acid salts such as C₁-C₆ alkyl sulfonates including methanesulfonates, trifluoromethanesulfonates and ethanesulfonates, aryl sulfonates including benzenesulfonates and p-toluenesulfonates, acetates, malates, fumarates, succinates, citrates, ascorbates, tartrates, oxalates, and maleates; and amino acid salts such as glycine salts, lysine salts, arginine salts, ornithine salts, glutamates, and aspartates.

Examples of the salt based on the acidic group include, but are not limited to, alkali metal salts including sodium salts, potassium salts, and lithium salts; alkaline earth metal salts including calcium salts and magnesium salts; metal salts including aluminum salts and iron salts; and inorganic salts such as ammonium salts; organic amine salts such as tert-butylamine salts, tert-octylamine salts, dibenzylamine salts, morpholine salts, glucosamine salts, phenylglycine alkyl ester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzylphenethylamine salts, piperazine salts, tetramethylammonium salts, and tris(hydroxymethyl) aminomethane salts; and amino acid salts such as glycine salts, lysine salts, arginine salts, ornithine salts, glutamates, and aspartates.

The compound represented by general formula (I) of the present invention or a pharmaceutically acceptable salt thereof may adsorb water or take up water molecules by being left in the air or by re-crystallization to form a hydrate. Such hydrates are also encompassed in the pharmaceutically acceptable salt of the present invention.

The compound represented by general formula (I) of the present invention or a pharmaceutically acceptable salt thereof may absorb a certain kind of solvent by being left in the solvent or by re-crystallization in the solvent to form a solvate. Such solvates are also encompassed in the salt of the present invention.

The solvent that can form a solvate is not particularly limited as long as it is not significantly toxic and can be used as a medicament. Examples of the solvent include ethanol, 1-propanol, 2-propanol, 1-butanol, 2-butanol, acetone, methyl ethyl ketone, methyl isobutyl ketone, dimethyl sulfoxide, ethyl formate, ethyl acetate, propyl acetate, diethyl ether, tetrahydrofuran, formic acid, acetic acid, pentane, heptane, cumene, and anisole.

### Advantageous Effects of Invention

The compound represented by general formula (I) of the present invention or a pharmaceutically acceptable salt thereof suppresses proliferation of activated T cells. It also suppresses IFN-α production by pDC activators. Thus, the compound of the present invention or a pharmacologically acceptable salt thereof is useful as a therapeutic or prophylactic agent for a disease whose symptoms are prevented, ameliorated or reduced by suppressing proliferation of activated T cells or suppressing production of IFN-α by activated pDC. Examples of such diseases include diseases involving the immune system, particularly autoimmune diseases. Specific examples of such diseases involving the immune system include systemic lupus erythematosus (SLE); a renal lesion, a central nerve disorder, a pulmonary disorder or vasculitis due to SLE; multiple myositis; ulcerative colitis; Sjogren's syndrome; rejection with organ transplantation; graft-versus-host disease (GVHD); dry eye; rheumatoid arthritis; Crohn's disease; psoriasis; dermatomyositis; atopic dermatitis; systemic scleroderma; and type I diabetes mellitus. Preferred diseases are SLE; a renal lesion, a central nerve disorder, a pulmonary disorder or vasculitis due to SLE; multiple myositis; ulcerative colitis; Sjogren's syndrome; graft-versus-host disease (GVHD) and psoriasis. Particularly preferred diseases are SLE, a renal lesion due to SLE (also referred to as lupus nephritis), and the like. The compound represented by general formula (I) of the present invention or a pharmaceutically acceptable salt thereof suppresses the function of immune cells by suppressing proliferation of activated T cells or suppressing production of IFN-α by activated pDC, and exerts an effect in the treatment and prevention of diseases involving the immune system described above.

### Brief Description of Drawings

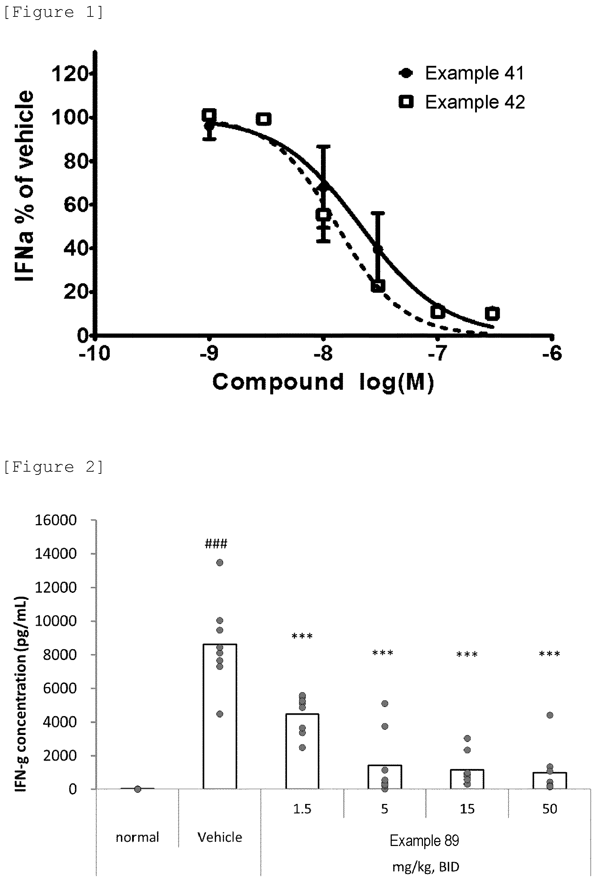

[Figure 1] Figure 1 shows the effect of test substances (compounds of Examples 41 and 42) on the production of IFN-α (shown as IFNa in the Figure) when mouse splenocytes were stimulated with CpG-ODN D35, a ligand of Toll-like receptor 9 (TLR9). The test substances were added at concentrations of 1 nM to 300 nM. The graph is the concentration reaction curve for each test substance when the IFN-α production amount in the test substancefree group is taken as 100% and the amount in the nonstimulation group as 0%, and shows the mean values and the standard deviations at N = 3.
[Figure 2] Figure 2 is a graph showing the effect of the compound of Example 89 in mouse acute GVHD models. The compound of Example 89 was orally administered at 1.5, 5, 15, or 50 mg/kg, BID. The vertical axis is the concentration of interferon gamma (sometimes abbreviated herein as IFN-γ or IFN-g) in blood on day 7 after elicitation in the model. The bar graph shows the mean values of each group at N = 8 and the plots show the values of each individual. A multiple comparison test of Dunnett relative to the Vehicle group was performed, and the group in which the p-value was 0.001 or less is shown by ***. ### indicates that the p-value by Student's t-test of the non-elicitation group (Normal group) and the Vehicle group is 0.001 or less.

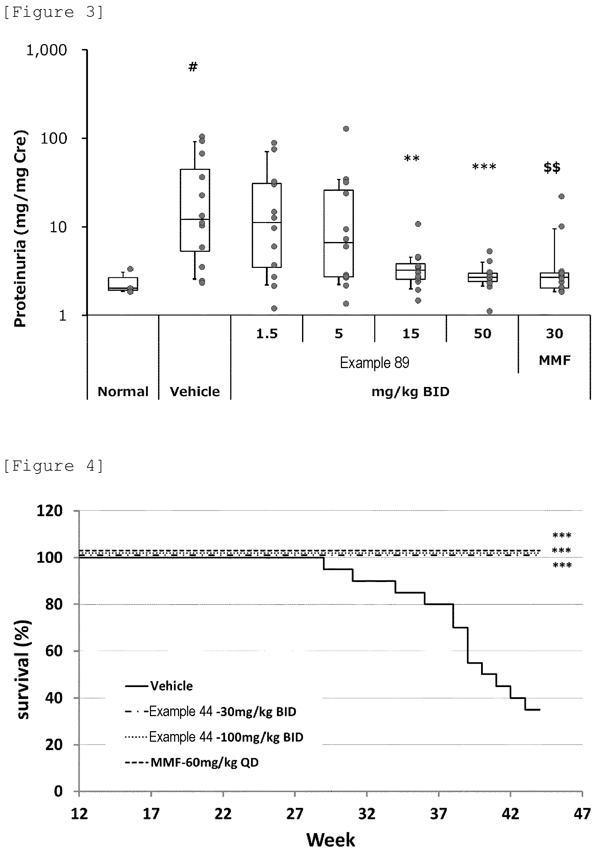

[Figure 3] Figure 3 is a box plot graph showing the effect of the compound of Example 89 on renal disorder in mouse chronic GVH models. The compound of Example 89 was orally administered at 1.5, 5, 15, or 50 mg/kg, BID, and MMF was orally administered at 30 mg/kg, BID. The vertical axis shows the urine protein/creatinine (Cre) ratio in urine on day 32 after elicitation in the model. The boxes in the graph represent box plots of the median and the quartile range for each group at N = 12. The plots show the values of each individual. Both between the non-elicitation group (Normal group) and the Vehicle group, and between the Vehicle group and the MMF group were compared by the Wilcoxon test (non-parametric, two-group comparison). # means that the p-value is 0.05 or less, and $$ means that the p-value is 0.01 or less, respectively. The Vehicle group and the compound group of Example 89 were analyzed by the Shirley-Williams test (non-parametric, multiple comparisons with dose response). The groups in which the p-value is 0.01 or less and 0.001 or less are shown by ** and ***, respectively.
[Figure 4] Figure 4 is a graph showing the effect of the compound of Example 44 in NZB/W F1 mice that naturally develop SLE-like symptoms and nephritis. To NZB/W F1 mice, the compound of Example 44 was orally administered at 30, 100 mg/kg, BID, and MMF was orally administered at 60 mg/kg, QD, starting at the age of 12 weeks. The vertical axis indicates the survival rate and the horizontal axis indicates the period. Both of the Vehicle group and the compound of Example 44 administration group was at N = 20, and the MMF administration group was at N = 15. The surviving days was analyzed by the Kaplan-Meier method, and the group in which the p-value was 0.001 or less compared to the Vehicle group by the log rank test is shown by ***.

### Description of Embodiments

Next, exemplary methods for producing a compound represented by general formula (I) are described. The compounds of the present invention can be produced by various production methods. The following production methods are examples and the present invention should be not construed as limited thereto. The compound represented by general formula (I) and production intermediates thereof can be produced using the various known reactions described below. During the reactions, the functional groups may be protected with appropriate protecting groups at the stage of the raw materials or intermediates. Examples of such functional groups include a hydroxyl group, a carboxy group, and an amino group. For types of protecting group and the conditions for introducing and removing those protecting groups, reference can be made, for example, to those described in Green's Protective Groups in Organic Synthesis, Fourth Edition (P. G. M. Wuts and T. W. Green, John Wiley & Sons, Inc., New York).

### [Production Method 1]

Among the compounds represented by the formula (I), compound 1a shown below can be produced, for example, by the reaction formula below,
wherein X, R¹, R² are the same as defined above, Y¹ represents a halogen group, Ar¹ represents an aryl group, and P¹, P² each represent a protecting group.

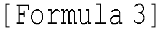

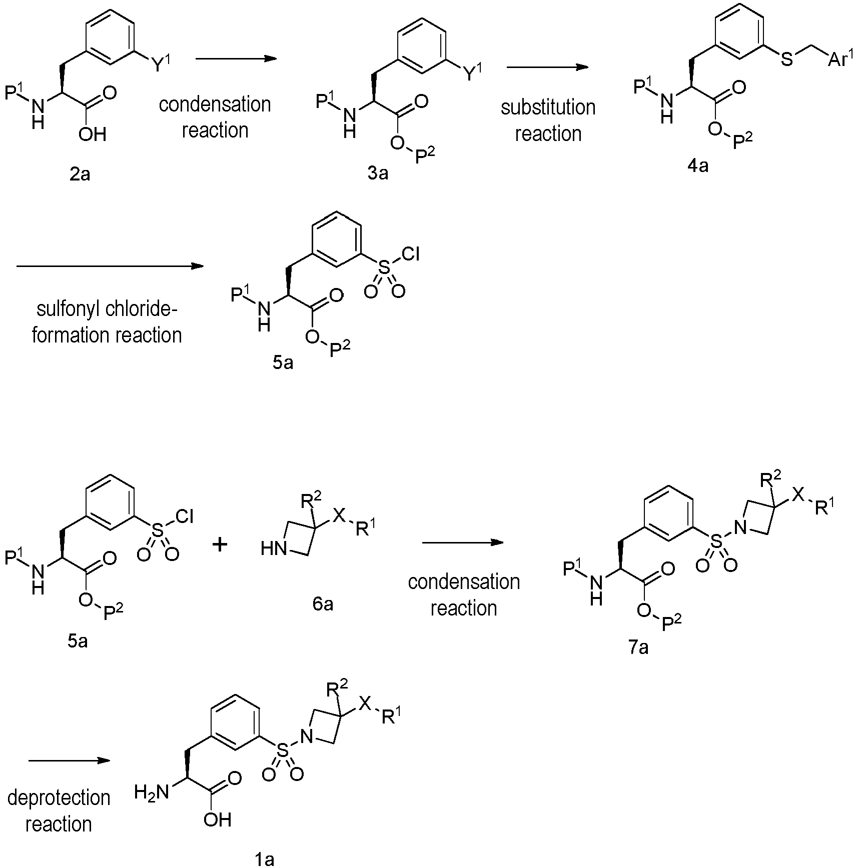

### (1A) Conversion of compound 2a to compound 3a

The conversion of compound 2a to compound 3a has different reaction conditions depending on the type of P². For example, when P² is a protecting group that can be introduced under general condensation reaction conditions, the conversion can be carried out by reacting, in an appropriate solvent (e.g., benzene, toluene, dichloromethane, chloroform, diethyl ether, tetrahydrofuran, N,N-dimethylformamide, or a mixed solvents thereof) that does not adversely affect the reaction, at from -10°C to the boiling point of the solvent used in the reaction, preferably from 0°C to 50°C, with an appropriate condensing agent (e.g., 1-ethyl-3-(3-dimethylaminopropyl)carbodiimide hydrochloride (EDC), N,N'-dicyclohexylcarbodiimide (DCC), benzotriazol-1-yloxy-trisdimethylaminophosphonium (BOP), 1H-benzotriazol-1-yloxytripyrrolidinophosphonium hexafluorophosphate (PyBOP), 1-[bis(dimethylamino)methylene]-1H-1,2,3-triazolo[4,5-b]pyridinium-3-oxide hexafluorophosphate (HATU), diphenylphosphate azide (DPPA), or 4-(4,6-dimethoxy-1,3,5-triazin-2-yl)-4-methylmorpholinium chloride (DMT-MM)), and, if necessary, an appropriate auxiliary agent or a base (such as 1-hydroxybenzotriazole (HOBt), 3H-1,2,3-triazolo[4,5-b]pyridin-3-ol (HOAt), 4-dimethylaminopyridine (DMAP), triethylamine, or N,N-diisopropylethylamine). The reaction time is preferably 5 minutes to 72 hours, and more preferably 30 minutes to 24 hours.

Compound 2a, which is a raw material for production, is commercially available or can be synthesized according to known methods.

### (1B) Conversion of compound 2a to compound 3a

The conversion of compound 2a to compound 3a can also be carried out, when P² is a tert-butyl group, by reacting, in an appropriate solvent (e.g., benzene, toluene, diethyl ether, dichloromethane, tetrahydrofuran, N,N-dimethylformamide, or a mixed solvents thereof) that does not adversely affect the reaction, at from -10°C to the boiling point of the solvent used in the reaction, preferably from 20°C to the boiling point of the solvent, with an appropriate reagent (e.g., tert-butanol, isobutene, or O-tert-butyl-N,N'-diisopropylisourea) and, if necessary, an appropriate reagent (e.g., N,N-dimethylformamide dineopentylacetal, sulfuric acid, or hydrochloric acid). The reaction time is preferably 30 minutes to 72 hours, and more preferably 2 hours to 24 hours.

### (2) Conversion of compound 3a to compound 4a

The conversion of compound 3a to compound 4a can also be carried out, by reacting, in an appropriate solvent (e.g., cyclopentylmethyl ether, tetrahydrofuran, 1,4-dioxane, dichloromethane, dimethylsulfoxide, N,N-dimethylformamide, or a mixed solvents thereof) that does not adversely affect the reaction, at from -20°C to the boiling point of the solvent used in the reaction, preferably from 20°C to the boiling point of the solvent used in the reaction, in the presence of an appropriate metal catalyst (e.g., copper(I) iodide, copper(I) bromide, copper(I) oxide, tris(dibenzylideneacetone)dipalladium(0), or tetrakis(triphenylphosphine)palladium(0)), and, if necessary, an appropriate phosphine reagent (e.g., xanthophos, or 1,10-phenanthroline) and a base (e.g., cesium carbonate, potassium carbonate, sodium carbonate, potassium phosphate, triethylamine, or N,N-diisopropylethylamine), with an appropriate mercaptan reagent (e.g., benzyl mercaptan, 4-tert-butyl benzyl mercaptan, or 4-(trimethylsilyl)benzyl mercaptan). The reaction time is preferably 30 minutes to 72 hours, and more preferably 2 hours to 24 hours.

### (3) Conversion of compound 4a to compound 5a

The conversion of compound 4a to compound 5a can be carried out by reacting, in an appropriate solvent (e.g., cyclopentylmethyl ether, 1,4-dioxane, tetrahydrofuran, acetonitrile, acetic acid, water, dimethylsulfoxide, N,N-dimethylformamide, or a mixed solvents thereof) that does not adversely affect the reaction, at from -50°C to the boiling point of the solvent used in the reaction, preferably from -30°C to 50°C, with an appropriate reagent (e.g., 1,3-dichloro-5,5-dimethylhydantoin, N-chlorosuccinimide, or trichloroisocyanuric acid). The reaction time is preferably 5 minutes to 72 hours, and more preferably 10 minutes to 6 hours.

### (4) Conversion of compound 5a to compound 7a

The conversion of compound 5a and compound 6a to compound 7a can be carried out by reacting, in an appropriate solvent (e.g., dichloromethane, chloroform, benzene, toluene, diethyl ether, cyclopentylmethyl ether, tetrahydrofuran, N,N-dimethylformamide, or a mixed solvents thereof) that does not adversely affect the reaction, at from -50°C to the boiling point of the solvent used in the reaction, preferably from -20°C to 50°C, in the presence of an appropriate base (e.g., triethylamine, or N,N-diisopropylethylamine). The reaction time is preferably 5 minutes to 72 hours, and more preferably 10 minutes to 6 hours.

The compound 6a, which is a raw material for production, is commercially available or can be synthesized according to the production methods 2 to 5 below.

### (5A) Conversion of compound 7a to compound 1a

The conversion of compound 7a to compound 1a has different deprotection reaction conditions depending on the type of P¹ and P². For example, when P¹ and P² can be deprotected under the same acidic conditions, the conversion can be carried out by reacting, in an appropriate solvent (e.g., dichloromethane, chloroform, diethyl ether, cyclopentylmethyl ether, tetrahydrofuran, 1,4-dioxane, ethyl acetate, acetic acid, N,N-dimethylformamide, or a mixed solvents thereof) that does not adversely affect the reaction, at from -80°C to the boiling point of the solvent used in the reaction, preferably from -30°C to 50°C, with an acid (e.g., hydrochloric acid, or trifluoroacetic acid). The reaction time for each is preferably 5 minutes to 72 hours, and more preferably 5 minutes to 6 hours.

### (5B) Conversion of compound 7a to compound 1a

The conversion of compound 7a to compound 1a has different deprotection reaction conditions depending on the type of P¹ and P². When P¹ and P² are deprotected under different reaction conditions, the deprotection of P¹ and P² can be carried out sequentially, for example, in an appropriate solvent (e.g., methanol, ethanol, isopropanol, diethyl ether, cyclopentylmethyl ether, tetrahydrofuran, 1,4-dioxane, water, N,N-dimethylformamide, or a mixed solvents thereof) that does not adversely affect the reaction, at from -80°C to the boiling point of the solvent used in the reaction, preferably from -30°C to 50°C, by first deprotecting one protecting group by reacting with a base (e.g., sodium hydroxide, potassium hydroxide, or lithium hydroxide); and subsequently deprotecting the other protecting group under general reaction conditions similar to the method described in (5A) of the [Production Method 1] above. The reaction time for each is preferably 5 minutes to 72 hours, and more preferably 5 minutes to 6 hours.

### [Production Method 2]

Among the raw material 6a of the [production method 1] above, compound 6aa shown below can be produced, for example, by the reaction formula below, wherein R² is the same as defined above, P³ represents a protecting group, and Ar² represents an aryl group].

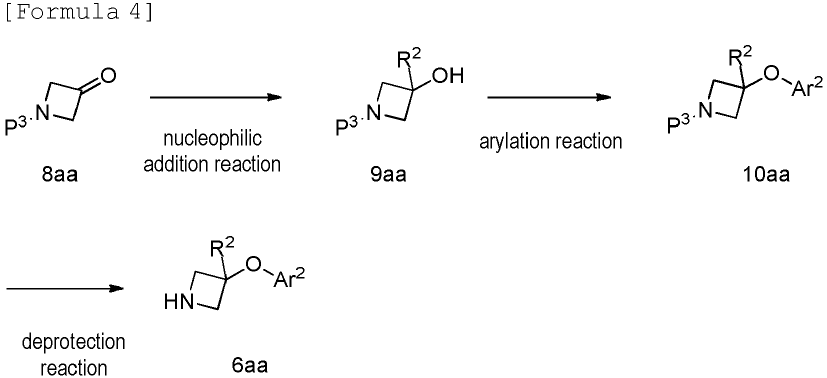

### (1) Conversion of compound 8aa to compound 9aa

The conversion of compound 8aa to compound 9aa can be carried out by reacting, in an appropriate solvent (e.g., benzene, toluene, diethyl ether, dichloromethane, tetrahydrofuran, N,N-dimethylformamide, or a mixed solvents thereof) that does not adversely affect the reaction, at from -80°C to the boiling point of the solvent used in the reaction, preferably from -80°C to 50°C, with an appropriate nucleophile agent (e.g., a Grignard reagent or an organic lithium reagent). The above-mentioned nucleophile agent may be used as is after it has been prepared in the reaction system. The reaction time is preferably 5 minutes to 72 hours, and more preferably 1 hour to 24 hours.

Compound 8aa, which is a raw material for production, is commercially available or can be synthesized according to known methods.

### (2A) Conversion of compound 9aa to compound 10aa

The conversion of compound 9aa to compound 10aa can be carried out by reacting, in an appropriate solvent (e.g., benzene, toluene, diethyl ether, cyclopentylmethyl ether, dichloromethane, tetrahydrofuran, N,N-dimethylformamide, or a mixed solvents thereof) that does not adversely affect the reaction, at from -80°C to the boiling point of the solvent used in the reaction, preferably from -30°C to 50°C, in the presence of an aryl alcohol and an appropriate phosphine reagent (e.g., triphenylphosphine, tributylphosphine, or PS-TPP), with an appropriate Mitsunobu reagent (e.g., diethyl azodicarboxylate, diisopropyl azodicarboxylate, di-tert-butyl azodicarboxylate, (azodicarbonyl)dipiperidine, or azobis(N,N-dimethylformamide)). The reaction time is preferably 5 minutes to 72 hours, and more preferably 10 minutes to 6 hours. The reaction can also be performed by processing in a sealed tube or under microwave irradiation.

The conversion of compound 9aa to compound 10aa can also be carried out by the different method 2B or 2C shown below.

### (2B)

This method can be carried out by reacting, in an appropriate solvent (e.g., benzene, toluene, dichloromethane, chloroform, diethyl ether, cyclopentylmethyl ether, tetrahydrofuran, or a mixed solvents thereof) that does not adversely affect the reaction, at from -80°C to the boiling point of the solvent used in the reaction, preferably from -30°C to 50°C, in the presence of an appropriate metal reagent (e.g., copper(II) acetate, copper(I) chloride, copper(II) chloride, or copper (powder)) and a base (e.g., triethylamine, N,N-diisopropylethylamine, or pyridine), with an appropriate bismuth reagent (e.g., triphenyl bismuth diacetate, triphenyl bismuth dichloride, triphenyl bismuthine, tris(3-fluorophenyl)bismuthine, or tris(4-fluorophenyl)bismuthine). This reaction can also be carried out under an oxygen atmosphere, as needed. The reaction time is preferably 1 hour to 72 hours, and more preferably 2 hours to 24 hours.

### (2C)

This method can be carried out by reacting, in an appropriate solvent (e.g., benzene, toluene, diethyl ether, cyclopentylmethyl ether, dichloromethane, tetrahydrofuran, N,N-dimethylformamide, or a mixed solvents thereof) that does not adversely affect the reaction, at from -80°C to the boiling point of the solvent used in the reaction, preferably from -30°C to 50°C, with an appropriate base (e.g., sodium hydride or potassium tert-butoxide), subsequently with an appropriate electrophile (e.g., 4-fluorobenzonitrile). The reaction time is preferably 5 minutes to 72 hours, and more preferably 10 minutes to 6 hours.

### (3) Conversion of compound 10aa to compound 6aa

The conversion of compound 10aa to compound 6aa has different deprotection reaction conditions depending on the type of P³. For example, when P³ can be deprotected under acidic conditions, the conversion can be carried out by the same general deprotection reaction as the method described in (5A) of the [Production Method 1] above.

### [Production Method 3]

Among the raw material 6a of the [production method 1] above, compound 6ab shown below can be produced, for example, by the reaction formula below, wherein P⁴ indicates a protecting group, Y² indicates a halogen group, and Ar³ indicates an aryl group].

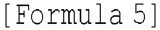

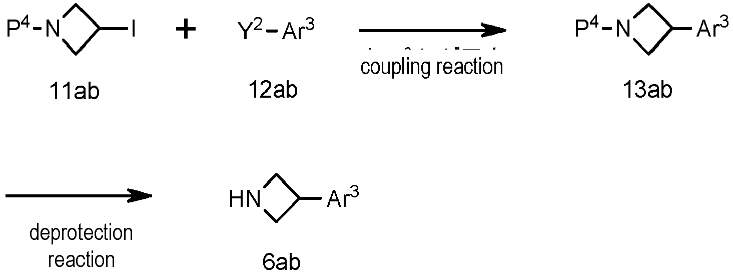

### (1) Conversion of compound 11ab to compound 13ab

The conversion of compound 11ab to compound 13ab can be carried out by adding compound 11ab to the reaction of zinc powder with 1,2-dibromoethane and chlorotrimethylsilane, in an appropriate solvent (e.g., benzene, toluene, dichloromethane, chloroform, diethyl ether, cyclopentylmethyl ether, tetrahydrofuran, N,N-dimethylformamide, or a mixed solvents thereof) that does not adversely affect the reaction; subsequently reacting with an appropriate metal reagent (e.g., tris(dibenzylideneacetone) dipalladium(0)) and an appropriate ligand (e.g., tri(2-furyl) phosphine); and then further reacting with compound 12ab. The reaction temperature is preferably from -80°C to the boiling point of the solvent used in the reaction, and more preferably from -30°C to 70°C. The reaction time is preferably 1 hour to 72 hours, and more preferably 2 hours to 24 hours. After this coupling reaction, structural conversion such as conversion of the substituent on the aromatic ring of Ar³ can be performed as needed.

### (2) Conversion of compound 13ab to compound 6ab

The conversion of compound 13ab to compound 6ab has different deprotection reaction conditions depending on the type of P⁴. For example, when P⁴ can be deprotected under acidic conditions, the conversion can be carried out by the same general deprotection reaction as the method described in (5A) of the [Production Method 1] above.

### [Production Method 4]

Among the raw material 6a of the [production method 1] above, compound 6ac shown below can be produced, for example, by the reaction formula below,
wherein P⁵ indicates a protecting group and Ar⁴ indicates an aryl group.

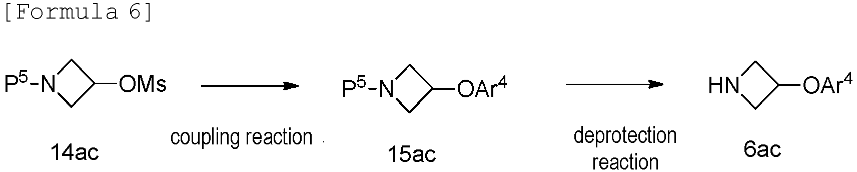

### (1) Conversion of compound 14ac to compound 15ac

The conversion of compound 14ac to compound 15ac can be carried out by reacting, in an appropriate solvent (e.g., N,N-dimethylformamide, acetone, or a mixed solvents thereof) that does not adversely affect the reaction, at from room temperature to the boiling point of the solvent used in the reaction, preferably from 50°C to 100°C, in the presence of an appropriate base (e.g., potassium carbonate, cesium carbonate, sodium hydride, or potassium tert-butoxide), with a corresponding phenol derivative. The reaction time is preferably 1 hour to 72 hours, and more preferably 8 hours to 24 hours. After the coupling reaction, structural conversion such as conversion of the substituent on the aromatic ring of Ar⁴ can be performed as needed.

### (2) Conversion of compound 15ac to compound 6ac

The conversion of compound 15ac to compound 6ac has different deprotection reaction conditions depending on the type of P⁵. For example, when P⁵ can be deprotected under acidic conditions, the conversion can be carried out by the same general deprotection reaction as the method described in (5A) of the [Production Method 1] above.

### [Production Method 5]

Among the raw material 6a of the [production method 1] above, compound 6ad shown below can be produced, for example, by the reaction formula below, wherein R² is the same as defined above, P⁶ indicates a protecting group, and R^{1a} indicates a C₁-C₆ alkyl group, a C₁-C₆ alkyl group substituted with 1 to 3 halogen atoms, a C₃-C₆ cycloalkyl group].

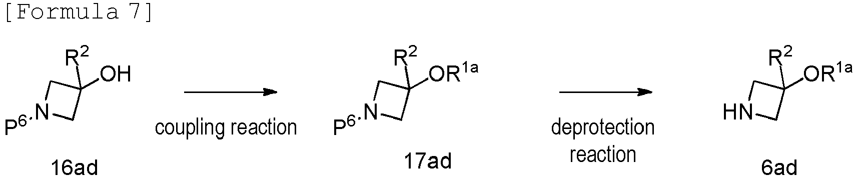

### (1) Conversion of compound 16ad to compound 17ad

The conversion of compound 16ad to compound 17ad can be carried out by the same coupling reaction as the method described in (2A) of the [Production method 2] above. After the coupling reaction, structural conversion of R^{1a} can be performed as needed.

### (2) Conversion of compound 17ad to compound 6ad

The conversion of compound 17ad to compound 6ad has different deprotection reaction conditions depending on the type of P⁶. For example, when P⁶ can be deprotected under acidic conditions, the conversion can be carried out by the same general deprotection reaction as the method described in (5A) of the [Production Method 1] above.

### [Production Method 6]

Among the compounds represented by formula (I), compound 1b shown below can be produced, for example, by the reaction formula below,
wherein X, R¹, R², R⁷, R⁸, and R⁹ are the same as defined above, R¹⁰ represents an appropriate leaving group such as a 4-nitrophenoxy group or a succinimidyloxy group.

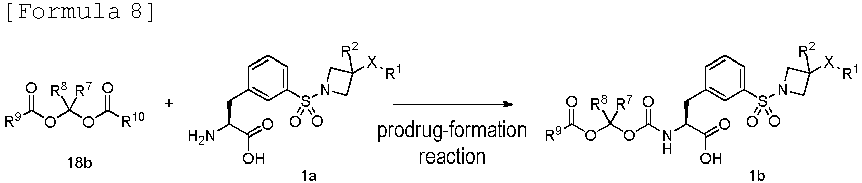

### (1) Conversion of compound 1a to compound 1b

The conversion of compound 1a to compound 1b can be carried out by reacting, in an appropriate solvent (e.g., diethyl ether, cyclopentylmethyl ether, tetrahydrofuran, 1,4-dioxane, dichloromethane, chloroform, N,N-dimethylformamide, or a mixed solvents thereof) that does not adversely affect the reaction, at from -20°C to the boiling point of the solvent used in the reaction, preferably from 0°C to 50°C, in the presence of an appropriate base (e.g., triethylamine, N,N-diisopropylethylamine, cesium carbonate, potassium carbonate, or sodium carbonate). The reaction time is preferably 10 minutes to 72 hours, and more preferably 30 minutes to 24 hours. It should be noted that compound 18b, which is a raw material, is commercially available or can be synthesized according to known methods. Compound 18b can also be used in the reaction after being separated with a chiral column to form an optically active material.

### [Production Method 7]

Among the compounds represented by formula (I), compound 1c shown below can be produced, for example, by the reaction formula below,
wherein X, R¹, R², and R⁴ are the same as defined above.

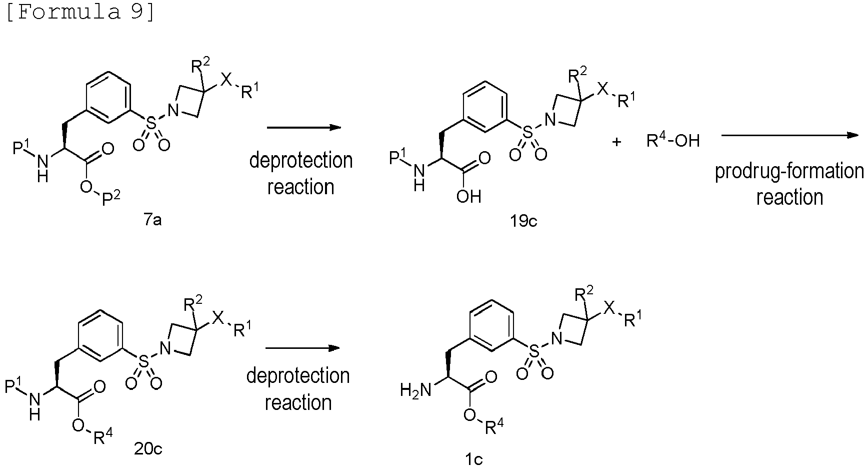

### (1) Conversion of compound 7a to compound 19c

The conversion of compound 7a to compound 19c has different deprotection reaction conditions depending on the type of P². For example, when P² can be deprotected under alkaline hydrolysis conditions and P¹ is stable under the same conditions, the conversion can be carried out by reacting, in an appropriate solvent (e.g., methanol, ethanol, isopropanol, diethyl ether, cyclopentylmethyl ether, tetrahydrofuran, 1,4-dioxane, water, N,N-dimethylformamide, or a mixed solvents thereof) that does not adversely affect the reaction, at from -80°C to the boiling point of the solvent used in the reaction, preferably from -30°C to 50°C, with a base (e.g., sodium hydroxide, potassium hydroxide, or lithium hydroxide). The reaction time is preferably 5 minutes to 72 hours, and more preferably 5 minutes to 6 hours.

### (2) Conversion of compound 19c to compound 20c

The conversion of compound 19c to compound 20c can be carried out by the same esterification reaction as the method described in (1A) of the [Production Method 1] above. It should be noted that, when R⁴ of compound 20c has a protecting group, deprotection can be carried out after this reaction.

### (3) Conversion of compound 20c to compound 1c

The conversion of compound 20c to compound 1c has different deprotection reaction conditions depending on the type of P¹. For example, when P¹ can be deprotected under acidic conditions, the conversion can be carried out by the same general deprotection reaction as the method described in (5A) of the [Production Method 1] above.

### [Production Method 8]

Among the compounds represented by formula (I), compound 1d shown below can be produced, for example, by the reaction formula below,
wherein X, R¹, R², R⁴, R⁷, R⁸, and R⁹ are the same as defined above.

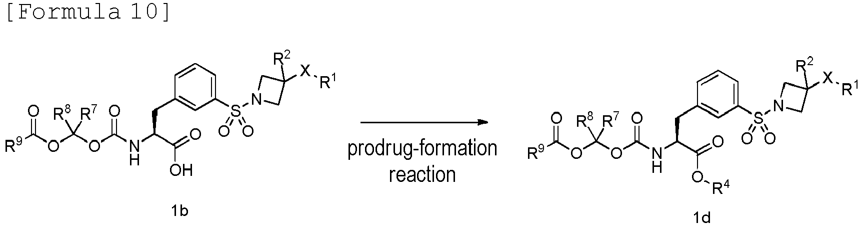

### (1) Conversion of compound 1b to compound 1d

The conversion of compound 1b to compound 1d can be carried out by the same esterification reaction as the method described in (1A) of the [Production Method 1] above. It should be noted that, when R⁴ of compound 1d has a protecting group, deprotection can be carried out after the reaction.

### [Production Method 9]

Among the compound represented by formula (I), compound 1d shown below can be produced, for example, by the reaction formula below,
wherein X, R¹, R², R⁴, R⁷, R⁸, and R⁹ are the same as defined above.

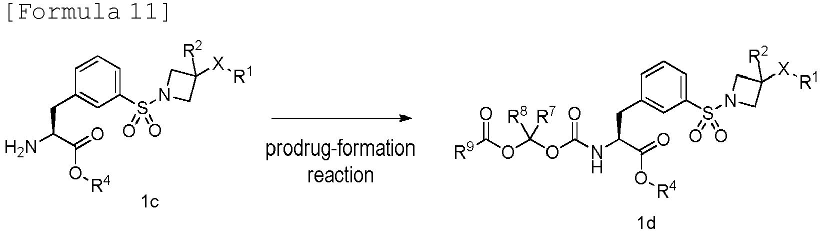

### (1) Conversion of compound 1c to compound 1d

The conversion of compound 1c to compound 1d can be carried out by the same prodrug-formation reaction as the method described in (1) of the [Production Method 6] above. It should be noted that, when R⁴ of compound 1d has a protecting group, deprotection can be carried out after this reaction.

### [Production Method 10]

Among the compounds represented by formula (I), compound 1e shown below can be produced, for example, by the reaction formula below,
wherein X, R¹, R², R⁷, R⁸, and R⁹ are the same as defined above, and R¹¹ and R¹² indicate a hydrogen atom or a C₁-C₆ alkyl group.

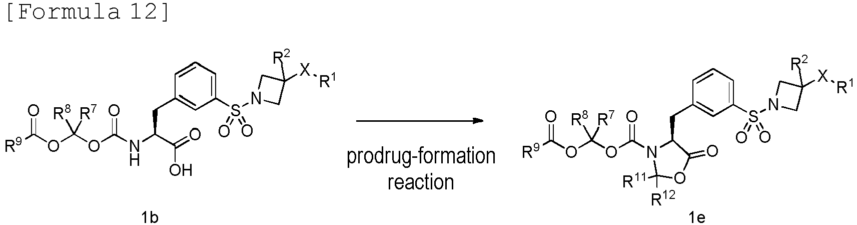

### (1) Conversion of compound 1b to compound 1e

The conversion of compound 1b to compound 1e is carried out by reacting, in an appropriate solvent (e.g., benzene, toluene, dichloromethane, chloroform, diethyl ether, tetrahydrofuran, N,N-dimethylformamide, or a mixed solvents thereof) that does not adversely affect the reaction, at from -50°C to the boiling point of the solvent used in the reaction, preferably from -20°C to 50°C, an appropriate aldehyde, ketone, or an corresponding acetal (paraformaldehyde, metaldehyde, isobutylaldehyde, acetone, or the like) and an acid (p-toluenesulfonic acid, methanesulfonic acid, trimethylsilyl trifluoromethanesulfonate, or the like). The reaction time is preferably 5 minutes to 72 hours, and more preferably 1 hour to 24 hours.

The compound represented by general formula (I) of the present invention or a pharmaceutically acceptable salt thereof includes the compound of a hydrate, which may be formed by taking up water molecules by being left in the air or by re-crystallization.

The compound represented by general formula (I) of the present invention or a salt thereof includes the compound of a solvate, which may be formed by absorbing certain kinds of solvent by being left in the solvent or by re-crystallization in the solvent.

The compound of the present invention or a pharmacologically acceptable salt thereof can be administered in various forms. Examples of such administration form include oral administration with tablets, capsules, granules, emulsions, pills, powders, and syrups (liquids), or parenteral administration with injectables (intravenous, intramuscular, subcutaneous, or intraperitoneal administration), drops, and suppositories (rectal administration). These various formulations can be formulated in accordance with conventional methods using the main drugs and auxiliary agents that are commonly usable in the pharmaceutical formulation technical field such as excipients, binders, disintegrants, lubricants, flavor modifiers, dissolution aids, suspending agents, and coatings.

When the formulation is used as tablets, examples of the usable carrier include excipients such as lactose, white sugar, sodium chloride, glucose, urea, starch, calcium carbonate, kaolin, crystalline cellulose, and silicic acid; binders such as water, ethanol, propanol, simple syrup, liquid glucose, liquid starch, gelatin solution, carboxymethylcellulose, shellac, methylcellulose, potassium phosphate, and polyvinylpyrrolidone; disintegrants such as dry starch, sodium alginate, agar powder, laminaran powder, sodium hydrogen carbonate, calcium carbonate, polyoxyethylene sorbitan fatty acid ester, sodium lauryl sulfate, monoglyceride stearate, starch, and lactose; disintegration inhibiting agents such as white sugar, stearin, cocoa butter, and hydrogenated oil; absorption promoters such as quaternary ammonium salts and sodium lauryl sulfate; moisturizing agents such as glycerin and starch; adsorbents such as starch, lactose, kaolin, bentonite, and colloidal silica; and lubricants such as purified talc, stearate, boric acid powder, and polyethylene glycol. When necessary, tablets that have been coated with a normal coating, such as sugar-coated tablets, gelatin-coated tablets, enteric-coated tablets, film-coated tablets, or double-layer tablets, or multilayer tablets, can be used.

When the formulation is used as pills, examples of the usable carrier include excipients such as glucose, lactose, cocoa butter, starch, hardened vegetable oil, kaolin, and talc; binders such as arabic rubber powder, tragacanth powder, gelatin, and ethanol; and disintegrants such as laminalan and agar.

When the formulation is used as suppositories, materials that are conventionally known in the art as carriers can be widely used, and examples thereof include polyethylene glycol, cocoa butter, higher alcohol, esters of higher alcohol, gelatin, and semi-synthetic glyceride.

When the formulation is used as injections, it may be used as a liquid, emulsion or suspension agent. It is preferred that such liquids, emulsions or suspension agents are sterilized, and isotonic with blood. The solvent used in the manufacture of the liquid, emulsion or suspension agent is not particularly limited as long as it can be used as a medical diluent, and examples thereof include water, ethanol, propylene glycol, ethoxylated isostearyl alcohol, polyoxylated isostearyl alcohol, and polyoxyethylene sorbitan fatty acid esters. In this case, the formulation may contain a sufficient amount of salt, glucose or glycerin to prepare an isotonic solution, and may contain a conventional dissolution aid, a buffer, or a soothing agent.

The above formulation can also contain a colorant, a preservative, a fragrance, a flavor, a sweetener, or the like, as well as other pharmaceuticals, as needed.

The amount of the active ingredient compound contained in the formulation is not particularly limited and is selected in a wide range as appropriate, but typically contains 0.5 to 70% by weight, preferably 1 to 30% by weight, in the total composition.

The amount used varies depending on the symptoms, age, or the like of the patient (warm-blooded animal, especially human). It is desirable that the formulation is administered, in the case of oral administration, at 0.01 mg/kg body weight (preferably 0.1 mg/kg body weight) as the lower limit and 500 mg/kg body weight (preferably 100 mg/kg body weight) as the upper limit per administration, and in the case of intravenous administration, at 0.002 mg/kg body weight (preferably 0.02 mg/kg body weight) as the lower limit and 100 mg/kg body weight (preferably 20 mg/kg body weight) as the upper limit per administration, once or several times per day according to symptoms.

### Examples

Hereinafter, the present invention will be more specifically described by way of Examples and Test Examples. However, the scope of the present invention is not limited to these.

Elution in column chromatography of Reference Examples and Examples were observed by Thin Layer Chromatography (TLC). In the TLC observation, a silica gel 60F254 manufactured by Merck & Co., Inc. was employed as a TLC plate, a solvent used as an eluting solvent in the column chromatography was employed as a developing solvent, and a UV detector was employed as a detection method. In the column chromatography, an automated purification device manufactured by YAMAZEN CORPORATION or an automated purification device manufactured by Shoko Science Co., Ltd. was used as appropriate. For the eluting solvent, the solvents specified in each Reference Example and Example were used. Note that the abbreviations used in the Reference Examples and Examples have the following meanings.

mg: milligram, g: gram, µl: microliter, ml: milliliter, L: liter, M: molar concentration, MHz: megahertz, Me: methyl, Et: ethyl, tBu: tert-butyl, Boc: tert-butoxycarbonyl, Fmoc: 9-fluorenylmethoxycarbonyl, TFA: trifluoroacetic acid.

In the following Examples, nuclear magnetic resonance (hereinafter referred to as ¹H NMR: 400 MHz) spectra were obtained using tetramethylsilane as a standard substance, and chemical shift values were described by δ values (ppm). In a splitting pattern, a singlet was represented by s, a doublet by d, a triplet by t, a quartet by q, a multiplet by m, and a broad by br.

### [Example 1]

### (S)-2-Amino-3-(3-((3-(3-fluorophenoxy)-3-(4-fluorophenyl)azetidin-1-yl)sulfonyl)phenyl)propanoic acid

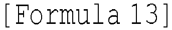

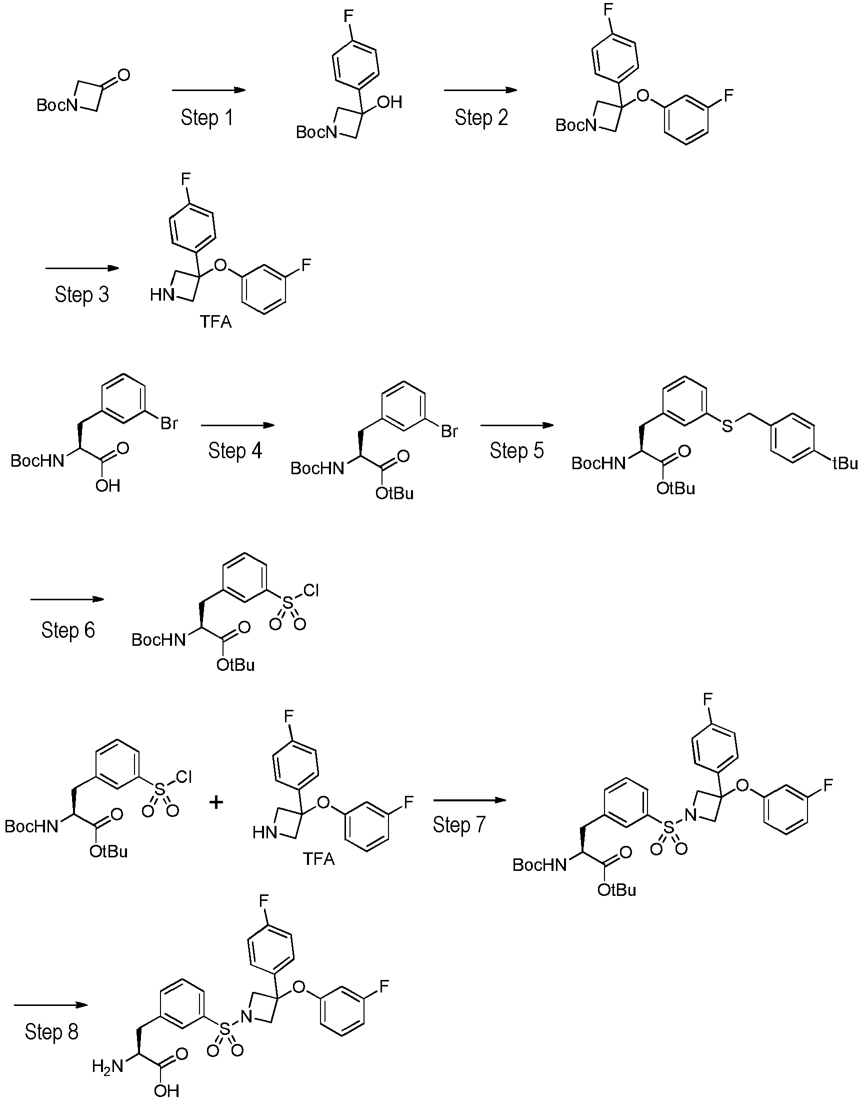

### [Step 1]

### tert-Butyl 3-(4-fluorophenyl)-3-hydroxyazetidin-1-carboxylate

To a solution of tert-butyl 3-oxoazetidin-1-carboxylate (1 g, FUJIFILM Wako Pure Chemical Corporation) in tetrahydrofuran (18 ml), 4-fluorophenyl magnesium bromide (2.0 M/diethyl ether solution, 4.26 ml) was added dropwise under ice-cooling, and then the mixture was stirred under ice-cooling for 15 minutes and at room temperature for 24 hours. The reaction solution was diluted with water, and made acidic with 1 N hydrochloric acid, then the mixture was extracted with diethyl ether. The organic layer was dried over sodium sulfate, then the sodium sulfate was filtered off, and the filtrate was concentrated under reduced pressure. The residue was purified by silica gel column chromatography (chloroform/methanol) to obtain the title compound (1.53 g).
¹H-NMR (CDCl₃) δ: 1.47 (9H, s), 2.47 (1H, s), 4.16 (1H, d, J = 9.3 Hz), 4.16 (1H, d, J = 9.3 Hz), 4.24 (2H, d, J = 9.3 Hz), 7.05-7.12 (2H, m), 7.45-7.51 (2H, m). MS (m/z) : 290 (M+Na)⁺.

### [Step 2]

### tert-Butyl 3-(3-fluorophenoxy)-3-(4-fluorophenyl)azetidin-1-carboxylate

To a solution of the compound obtained by the method of Step 1 (1.00 g), 3-fluorophenol (0.678 ml) and triphenylphosphine (1.08 g) in toluene (10 ml), diisopropyl azodicarboxylate (0.800 ml) was added under ice-cooling, and the mixture was stirred at room temperature for 3 hours. The reaction solution was concentrated under reduced pressure and the residue was purified by silica gel column chromatography (n-hexane/ethyl acetate) to obtain the title compound (730 mg) .
¹H-NMR (CDCl₃) δ: 1.47 (9H, s), 4.25 (1H, d, J = 9.3 Hz), 4.25 (1H, d, J = 9.3 Hz), 4.36 (2H, d, J = 9.5 Hz), 6.27-6.32 (2H, m), 6.59-6.66 (1H, m), 7.03-7.15 (3H, m), 7.39-7.45 (2H, m).
MS (m/z) : 384 (M+Na)⁺.

### [Step 3]

### 3-(3-Fluorophenoxy)-3-(4-fluorophenyl)azetidine trifluoroacetate

To a solution of the compound obtained by the method of Step 2 (364 mg) in dichloromethane (3 ml), trifluoroacetic acid (0.8 ml) was added, and the mixture was stirred at room temperature for 1 hour. The reaction solution was concentrated under reduced pressure to obtain the title compound (378 mg) and used in Step 7 without purification.
MS (m/z) : 262 (M+H)⁺.

### [Step 4]

### (S)-tert-Butyl 3-(3-bromophenyl)-2-((tert-butoxycarbonyl)amino)propanoate

To a solution of N-tert-butoxycarbonyl-L-3-bromophenylalanine (9.5 g, Amatek, Inc.) in toluene (120 ml), tert-butanol (60 ml) was added, and the mixture was heated to reflux for 15 minutes. While the mixture was heated to reflux, N,N-dimethylformamide dineopentyl acetal (35 ml) was divided into three portions and slowly added dropwise over 3 hours, and then the mixture was stirred for another 1 hour. The reaction solution was left to cool to room temperature, and then diluted with ethyl acetate. The diluted solution was washed with saturated aqueous sodium hydrogen carbonate solution, water and saturated saline sequentially, and dried over sodium sulfate. The sodium sulfate was filtered off, and the filtrate was concentrated under reduced pressure. The residue was purified by silica gel column chromatography (n-hexane/ethyl acetate) to obtain the title compound (10.2 g).
¹H-NMR (CDCl₃) δ: 1.41 (9H, s), 1.43 (9H, s), 2.96-3.09 (2H, m), 4.38-4.47 (1H, m), 5.03 (1H, d, J = 6.6 Hz), 7.09-7.13 (1H, m), 7.16 (1H, t, J = 7.8 Hz), 7.31-7.34 (1H, m), 7.34-7.39 (1H, m).
MS (m/z) : 422 (M+Na)⁺.

### [Step 5]

### (S)-tert-Butyl 2-((tert-butoxycarbonyl)amino)-3-(3-((4-(tert-butyl)benzyl)thio)phenyl)propanoate

To a solution of the compound (5.2 g) obtained by the method of Step 4 in 1,4-dioxane (50 ml), 4-tert-butylbenzylmercaptan (3.78 ml), 9,9-dimethyl-4,5-bis(diphenylphosphino)xanthene (0.785 g), tris(dibenzylideneacetone)dipalladium(0) (0.622 g) and N,N-diisopropylethylamine (3.53 ml) were added, and then the mixture was stirred at 110°C for 3 hours under an argon atmosphere. The reaction solution was left to cool to room temperature, and then diluted with ethyl acetate. The diluted solution was washed with 0.5 N hydrochloric acid, saturated aqueous sodium hydrogen carbonate solution, water and saturated saline sequentially, and then dried over sodium sulfate. The sodium sulfate was filtered off, and the filtrate was concentrated under reduced pressure. The residue was purified by silica gel column chromatography (n-hexane/ethyl acetate) to obtain the title compound (6.13 g).
¹H-NMR (CDCl₃) δ: 1.30 (9H, s), 1.40 (9H, s), 1.43 (9H, s), 2.81-3.07 (2H, m), 4.09 (2H, s), 4.42 (1H, dd, J = 13.6, 6.5 Hz), 4.98 (1H, d, J = 7.5 Hz), 6.96-7.02 (1H, m), 7.11-7.14 (1H, m), 7.16-7.20 (2H, m), 7.21-7.25 (2H, m), 7.29-7.34 (2H, m).
MS (m/z) : 522 (M+Na)⁺.

### [Step 6]

### (S)-tert-Butyl 2-((tert-butoxycarbonyl)amino)-3-(3-(chlorosulfonyl)phenyl)propanoate

To a solution of the compound obtained by the method of Step 5 (6.13 g) in acetonitrile (90 ml), water (2.21 ml) was added and then acetic acid (2.81 ml) and 1,3-dichloro-5,5-dimethylhydantoin (5.08 g) were added under ice-cooling, and the mixture was stirred for 15 minutes. The reaction solution was diluted with saturated saline, and then extracted with ethyl acetate. The organic layer was washed with saturated saline, then the mixture was dried over sodium sulfate. After the sodium sulfate was filtered off, the filtrate was concentrated under reduced pressure and the residue was purified by silica gel column chromatography (n-hexane/ethyl acetate) to obtain the title compound (4.6 g).
¹H-NMR (CDCl₃) δ: 1.43 (9H, s), 1.43 (9H, s), 3.15 (1H, dd, J = 13.8, 5.3 Hz), 3.26 (1H, dd, J = 13.9, 6.1 Hz), 4.44-4.54 (1H, m), 5.05-5.16 (1H, m), 7.51-7.60 (2H, m), 7.82-7.85 (1H, m), 7.90-7.95 (1H, m).
MS (m/z) : 442 (M+Na)⁺.

### [Step 7]

### (S)-tert-Butyl 2-(tert-butoxycarbonyl)amino)-3-(3-((3-(3-fluorophenoxy)-3-(4-fluorophenyl)azetidin-1-yl)sulfonyl)phenyl)propanoate

To a solution of the compound (378 mg) obtained by the method of Step 3 in dichloromethane (8 ml), the compound (423 mg) obtained by the method of Step 6 and N,N-diisopropylethylamine (0.880 ml) were added, and the mixture was stirred at room temperature for 1 hour. The reaction solution was diluted with ethyl acetate, and washed with 1 N hydrochloric acid and saturated saline sequentially, and then dried over sodium sulfate. The sodium sulfate was filtered off, and the filtrate was concentrated under reduced pressure. The residue was purified by silica gel column chromatography (n-hexane/ethyl acetate) to obtain the title compound (488 mg) .
¹H-NMR (CDCl₃) δ: 1.43 (9H, s), 1.44 (9H, s), 3.12 (1H, dd, J = 13.7, 5.4 Hz), 3.25 (1H, dd, J = 13.4, 6.4 Hz), 4.13-4.21 (4H, m), 4.42-4.52 (1H, m), 5.00 (1H, d, J = 6.3 Hz), 6.11-6.17 (2H, m), 6.58-6.64 (1H, m), 6.99-7.09 (3H, m), 7.25-7.32 (2H, m), 7.50 (2H, d, J = 5.0 Hz), 7.67 (1H, s), 7.70-7.76 (1H, m).
MS (m/z) : 667 (M+Na)⁺.

### [Step 8]

### (S)-2-Amino-3-(3-((3-(3-fluorophenoxy)-3-(4-fluorophenyl)azetidin-1-yl)sulfonyl)phenyl)propanoic acid

The compound (1.21 g) obtained by the method of Step 7 was dissolved in a solution (13 ml) of 1 N hydrochloric acid/acetic acid, and the mixture was stirred at 45°C for 2.5 hours, then concentrated under reduced pressure. To the residue, n-hexane/dichloromethane (2/1) was added to form a suspension, and the precipitate was collected by filtration to obtain the title compound (935 mg) containing a small amount of impurities. A portion (100 mg) of the solid was dissolved in a small amount of acetic acid under heating, then the solution was left to cool to room temperature. Water (1.6 ml) was added thereto, and the precipitate was collected by filtration. The obtained solid was again dissolved in a small amount of acetic acid under heating, then the solution was left to cool to room temperature. The precipitate was collected by filtration to obtain the title compound (69 mg).
¹H-NMR (DMSO-d₆) δ: 2.97 (1H, dd, J = 14.2, 7.4 Hz), 3.20 (1H, dd, J = 14.2, 5.1 Hz), 3.46 (1H, dd, J = 7.3, 5.3 Hz), 4.11 (1H, d, J = 9.8 Hz), 4.12 (1H, d, J = 9.5 Hz), 4.35 (1H, d, J = 9.5 Hz), 4.36 (1H, d, J = 9.7 Hz), 6.25-6.30 (1H, m), 6.32 (1H, dt, J = 10.8, 2.3 Hz), 6.69-6.76 (1H, m), 7.12-7.20 (3H, m), 7.28-7.35 (2H, m), 7.57 (1H, t, J = 7.8 Hz), 7.66-7.72 (2H, m), 7.77-7.80 (1H, m). MS (m/z) : 489 (M+H)⁺.

The same procedures as in Example 1 were performed to obtain the compounds below. However, for Example 16, Step 7 and later steps were performed using commercially available 3-benzyl-3-phenylazetidine hydrochloride.

**[Table 1]**

| Example | Name and Structure | Equipment data |
|---|---|---|
| 2 | (S)-2-Amino-3-(3-((3-phenyl-3-(2-(trifluoromethyl)phenoxy)azetidin-1-yl)sulfonyl)phenyl)propanoic acid | ¹H-NMR (DMSO-d₆) δ: 3.02 (1H, dd, J = 14.2, 7.4 Hz), 3.22 (1H, dd, J = 14.2, 5.6 Hz), 3.67 (1H, br s), 4.09 (1H, d, J = 9.8 Hz), 4.10 (1H, d, J = 9.8 Hz), 4.39 (2H, d, J = 9.8 Hz), 6.30 (1H, d, J = 8.5 Hz), 7.03 (1H, t, J = 7.7 Hz), 7.25-7.38 (6H, m), 7.59 (1H, t, J = 7.8 Hz), 7.61 (1H, dd, J = 7.8, 1.5 Hz), 7.66-7.75 (2H, m), 7.79-7.82 (1H, m). |
| | 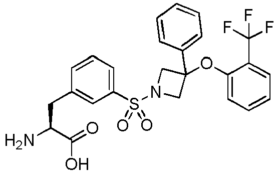 | |
| | | MS (m/z): 543 (M+Na)⁺. |
| 3 | (S)-2-Amino-3-(3-((3-(3-chlorophenoxy)-3 -phenylazetidin- 1 - yl)sulfonyl)phenyl)propanoic acid | ¹H-NMR (DMSO-d₆) δ: 2.95 (1H, dd, J = 14.1, 7.5 Hz), 3.20 (1H, dd, J = 14.2, 4.9 Hz), 3.41 (1H, dd, J = 7.7, 5.1 Hz), 4. 10 (1H, d, J = 9.8 Hz), 4. 11 (1H, d, J |
| | 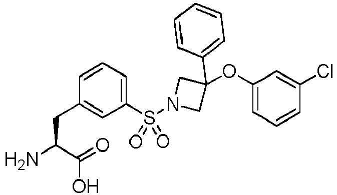 | = 9.8 Hz), 4.35 (2H, d, J = 10.0 Hz), 6.37 (1H, ddd, J = 8.5, 2.5, 0.8 Hz), 6.52 (1H, t, J = 2.1 Hz), 6.94 (1H, ddd, J = 8.0, 2.0, 0.8 Hz), 7.14 (1H, t, J = 8.2 Hz), 7.25-7.36 (5H, m), 7.58 (1H, t, J = 7.8 Hz), 7.67-7.73 (2H, m), 7.77-7.80 (1H, m). |
| | | MS (m/z) : 487 (M+H)⁺. |
| 4 | (S)-2-Amino-3-(3-((3-(2-fluorophenoxy)-3-(4-fluorophenyl)azetidin-1-yl)sulfonyl)phenyl)propanoic acid | ¹H-NMR (DMSO-d₆) δ: 2.96 (1H, dd, J = 14.2, 7.4 Hz), 3.20 (1H, dd, J = 14.2, 5.1 Hz), 3.42 (1H, dd, J = 7.5, 5.3 Hz), 4.16 (1H, d, J = 10.0 Hz), 4.16 (1H, d, J = 10.0 Hz), 4.36 (2H, d, J = 10.0 Hz), 6.38 (1H, td, J = 8.3, 1.8 Hz), 6.83-6.93 (2H, m), 7.11-7.19 (3H, m), 7.31-7.37 (2H, m), 7.55 (1H, t, J = 7.7 Hz), 7.64-7.70 (2H, m), 7.76-7.79 (1H, m). |
| | 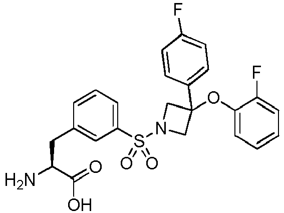 | |
| | | MS (m/z) : 511 (M+Na)⁺. |

**[Table 2]**

| | | |
|---|---|---|
| 5 | (S)-2-Amino-3-(3-((3-(2-bromophenoxy)-3 -phenylazetidin- 1 - yl)sulfonyl)phenyl)propanoic acid | ¹H-NMR (DMSO-d₆) δ: 2.99 (1H, dd, J = 14.2, 7.4 Hz), 3.22 (1H, dd, J = 14.4, 5.1 Hz), 3.47-3.59 (1H, m), 4.13 (1H, d, J = 10.3 Hz), 4.14 (1H, d, J = 10.0 Hz), 4.37 (2H, d, J = 9.8 Hz), 6.22 (1H, dd, J = 8.3, 1.3 Hz), 6.82 (1H, td, J = 7.7, 1.3 Hz), 7.02-7.06 (1H, m), 7.25-7.35 (5H, m), 7.57 (2H, dt, J = 14.0, 5.8 Hz), 7.70 (2H, t, J = 8.9 Hz), 7.80 (1H, br s). |
| | 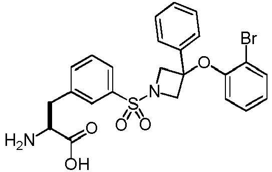 | |
| | | MS (m/z): 555 (M+Na)⁺. |
| 6 | (S)-2-Amino-3-(3-((3-(4-fluorophenoxy)-3-(4-fluorophenyl)azetidin-1-yl)sulfonyl)phenyl)propanoic acid | ¹H-NMR (DMSO-d₆) δ: 2.97 (1H, dd, J = 14.3, 7.5 Hz), 3.20 (1H, dd, J = 14.3, 5.0 Hz), 3.44 (1H, dd, J = 7.3, 5.3 Hz), 4.09 (1H, d, J = 9.7 Hz), 4.10 (1H, d, J = 9.8 Hz), 4.30 (2H, d, J = 9.8 Hz), 6.43-6.50 (2H, m), 6.93-7.01 (2H, m), 7.12-7.19 (2H, m), 7.28-7.34 (2H, m), 7.57 (1H, t, J = 7.7 Hz), 7.66-7.71 (2H, m), 7.76-7.79 (1H, m). |
| | 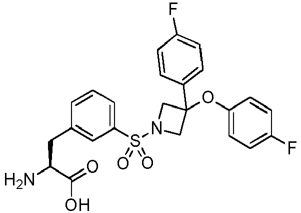 | |
| | | MS (m/z) : 511 (M+Na)⁺. |
| 7 | (S)-2-Amino-3-(3-((3-(2-chlorophenoxy)-3-phenylazetidin-1-yl)sulfonyl)phenyl)propanoic acid | ¹H-NMR (DMSO-d₆) δ: 2.95 (1H, dd, J = 14.3, 7.7 Hz), 3.21 (1H, dd, J = 14.3, 5.1 Hz), 3.42 (1H, dd, J = 7.7, 5.1 Hz), 4.14 (2H, d, J = 8.5 Hz), 4.37 (2H, d, J = 9.8 Hz), 6.26 (1H, dd, J = 8.3, 1.5 Hz), 6.86-6.92 (1H, m), 6.97-7.03 (1H, m), 7.22-7.36 (5H, m), 7.40 (1H, dd, J = 7.9, 1.6 Hz), 7.57 (1H, t, J = 7.8 Hz), 7.66-7.72 (2H, m), 7.78-7.81 (1H, m). |
| | 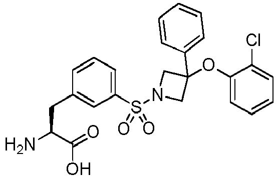 | |
| | | MS (m/z) : 487 (M+H)⁺. |

**[Table 3]**

| | | |
|---|---|---|
| 8 | (S)-2-Amino-3 -(3 -((3 -(3 -bromophenoxy)-3-phenylazetidin-1-yl)sulfonyl)phenyl)propanoic acid | ¹H-NMR (DMSO-d₆) δ: 2.96 (1H, dd, J = 14.2, 7.6 Hz), 3.22 (1H, dd, J = 14.2, 5.0 Hz), 3.44 (1H, dd, J = 7.6, 5.0 Hz), 4.06-4.15 (2H, m), 4.34 (2H, d, J = 10.0 Hz), 6.35-6.42 (1H, m), 6.64-6.68 (1H, m), 7.05-7.10 (2H, m), 7.24-7.37 (5H, m), 7.58 (1H, t, J = 7.7), 7.67-7.73 (2H, m), 7.77-7.80 (1H, m). |
| | 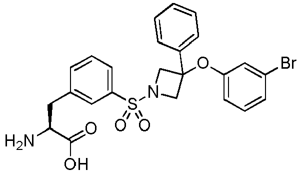 | |
| | | MS (m/z): 531 (M+H)⁺. |
| 9 | (S)-2-Amino-3-(3-((3-phenyl-3-((p-tolyloxy)azetidin-1-yl)sulfonyl)phenyl)propanoic acid | ¹H-NMR (DMSO-d₆) δ: 2.14 (3H, s), 2.95 (1H, dd, J = 14.3, 7.5 Hz), 3.21 (1H, dd, J = 14.3, 5.0 Hz), 3.41 (1H, dd, J = 7.5, 5.0 Hz), 4.06 (1H, d, J = 9.8 Hz), 4.08 (1H, d, J = 9.8 Hz), 4.28 (2H, d, J = 9.8 Hz), 6.30-6.35 (2H, m), 6.88-6.94 (2H, m), 7.22-7.34 (5H, m), 7.58 (1H, t, J = 7.7 Hz), 7.66-7.71 (2H, m), 7.76-7.79 (1H, m). |
| | 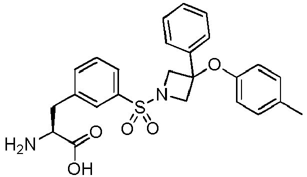 | |
| | | MS (m/z) : 467 (M+H)⁺. |
| 10 | (S)-2-Amino-3 -(3 -((3 -(3,4-difluorophenoxy)-3 -phenylazetidin- 1 - yl)sulfonyl)phenyl)propanoic acid | ¹H-NMR (DMSO-d₆) δ: 2.96 (1H, dd, J = 14.2, 7.7 Hz), 3.21 (1H, dd, J = 14.2, 5.1 Hz), 3.42 (1H, dd, J = 7.5, 5.3 Hz), 4.10 (1H, d, J = 9.5 Hz), 4.11 (1H, d, J = 9.8 Hz), 4.33 (1H, d, J = 9.5 Hz), 4.34 (1H, d, J = 9.3 Hz), 6.22-6.28 (1H, m), 6.54-6.62 (1H, m), 7.15-7.36 (8H, m), 7.58 (1H, t, J = 7.7 Hz), 7.66-7.72 (2H, m), 7.78 (1H, br s). |
| | 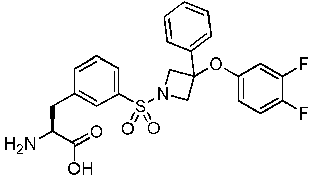 | |
| | | MS (m/z) : 490 (M+H)⁺. |

**[Table 4]**

| | | |
|---|---|---|
| 11 | (S)-2-Amino-3 -(3 -((3 -(3 -chloro-4-fluorophenoxy)-3 -phenylazetidin- 1 - yl)sulfonyl)phenyl)propanoic acid | ¹H-NMR (DMSO-d₆) δ: 2.96 (1H, dd, J = 14.2, 7.4 Hz), 3.21 (1H, dd, J = 13.9, 5.1 Hz), 3.44 (1H, dd, J = 7.2, 5.1 Hz), 4.10 (1H, d, J = 9.5 Hz), 4.11 (1H, d, J = 9.3 Hz), 4.34 (2H, d, J = 10.5 Hz), 6.41 (1H, dt, J = 8.9, 3.4 Hz), 6.66 (1H, dd, J = 6.3, 3.0 Hz), 7.17 (1H, t, J = 9.2 Hz), 7.23-7.39 (5H, m), 7.58 (1H, t, J = 7.7 Hz), 7.69 (2H, t, J = 6.8 Hz), 7.78 (1H, br s). |
| | 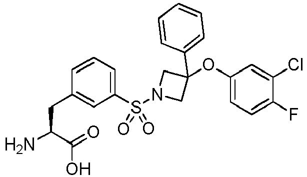 | |
| | | MS (m/z) : 528 (M+Na)⁺. |
| 12 | (S)-2-Amino-3-(3-((3-phenyl-3-(4-(trifluoromethyl)phenoxy)azetidin-1-yl)sulfonyl)phenyl)propanoic acid | ¹H-NMR (DMSO-d₆) δ: 2.96 (1H, dd, J = 14.3, 7.5 Hz), 3.21 (1H, dd, J = 14.1, 5.0 Hz), 3.43 (1H, dd, J = 7.5, 5.3 Hz), 4.14 (1H, d, J = 9.5 Hz), 4.17 (1H, d, J = 9.5 Hz), 4.37 (1H, d, J = 9.3 Hz), 4.37 (1H, d, J = 9.3 Hz), 6.65 (2H, d, J = 8.8 Hz), 7.23-7.28 (2H, m), 7.28-7.36 (3H, m), 7.50 (2H, d, J = 8.8 Hz), 7.58 (1H, t, J = 7.8 Hz), 7.67-7.73 (2H, m), 7.78-7.81 (1H, m). |
| | 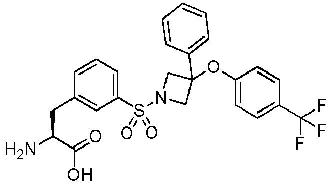 | |
| | | MS (m/z): 521 (M+H)⁺. |
| 13 | (S)-2-Amino-3-(3-((3-(3-fluorophenoxy)-3-(2-fluorophenyl)azetidin-1-yl)sulfonyl)phenyl)propanoic acid | ¹H-NMR (DMSO-d₆) δ: 2.91 (1H, dd, J = 14.1, 7.8 Hz), 3.17 (1H, dd, J = 14.3, 5.0 Hz), 3.40 (1H, dd, J = 7.5, 5.3 Hz), 4.23 (1H, d, J = 10.0 Hz), 4.24 (1H, d, J = 10.0 Hz), 4.53 (2H, d, J = 10.0 Hz), 6.37-6.44 (2H, m), 6.69-6.76 (1H, m), 7.10-7.18 (3H, m), 7.32-7.40 (1H, m), 7.52 (1H, t, J = 7.8 Hz), 7.56-7.63 (2H, m), 7.68 (1H, d, J = 7.5 Hz), 7.75 (1H, s). |
| | 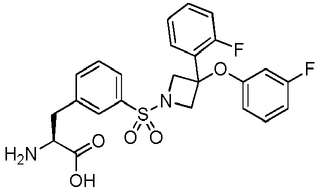 | |
| | | MS (m/z) : 489 (M+H)⁺. |

**[Table 5]**

| | | |
|---|---|---|
| 14 | (S)-2-Amino-3-(3-((3-(4-fluorophenoxy)-3-(2-fluorophenyl)azetidin-1-yl)sulfonyl)phenyl)propanoic acid | ¹H-NMR (DMSO-d₆) δ: 2.92 (1H, dd, J = 14.2, 7.4 Hz), 3.18 (1H, dd, J = 14.3, 5.0 Hz), 3.40 (1H, dd, J = 7.4, 5.1 Hz), 4.22 (1H, d, J = 10.0 Hz), 4.24 (1H, d, J = 9.8 Hz), 4.46 (2H, d, J = 9.8 Hz), 6.54-6.60 (2H, m), 6.92-6.96 (2H, m), 7.06-7.20 (2H, m), 7.30-7.39 (1H, m), 7.46-7.53 (2H, m), 7.60 (1H, d, J = 7.8 Hz), 7.67 (1H, d, J = 8.0 Hz), 7.75 (1H, |
| | 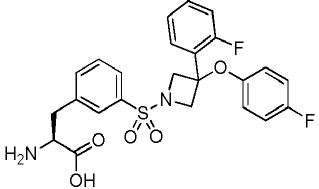 | br s). |
| | | MS (m/z) : 489 (M+H)⁺. |
| 15 | (S)-2-Amino-3-(3-((3-(3-carbamoylphenoxy)-3-phenylazetidin-1-yl)sulfonyl)phenyl)propanoic acid | ¹H-NMR (DMSO-d₆) δ: 2.94 (1H, br s), 3.13 (1H, dd, J = 13.6, 4.8 Hz), 4.10 (1H, d, J = 9.6 Hz), 4.11 (1H, d, J = 9.5 Hz), 4.28-4.37 (2H, m), 6.48 (1H, dd, J = 8.2, 2.1 Hz), 7.05 (1H, s), 7.16 (1H, t, J = 8.0 Hz), 7.19-7.34 (6H, m), 7.37 (1H, d, J = 8.0 Hz), 7.51 (1H, t, J = 7.7 Hz), 7.60-7.68 (2H, m), 7.77 (1H, s), 8.16 (1H, br s), signal of one proton was overlapped with H₂O signal. |
| | 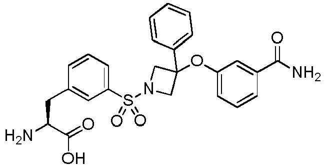 | |
| | | MS (m/z) : 496 (M+H)⁺. |
| 16 | (S)-2-Amino-3 -(3 -((3 -benzyl-3 - phenylazetidin-1-yl)sulfonyl)phenyl)propanoic acid | ¹H-NMR (DMSO-d₆) δ: 2.86-2.95 (3H, m), 3.18 (1H, dd, J = 14.1, 5.0 Hz), 3.37 (1H, dd, J = 7.5, 5.0 Hz), 3.92 (1H, d, J = 8.0 Hz), 3.92 (1H, d, J = 8.0 Hz), 3.97 (1H, d, J = 8.3 Hz), 4.00 (1H, d, J = 8.3 Hz), 6.60-6.65 (2H, m), 6.83-6.89 (2H, m), 7.03-7.23 (6H, m), 7.51 (1H, t, J = 7.7 Hz), 7.55-7.59 (1H, m), 7.62 (1H, dt, J = 7.6, 1.6 Hz), 7.70-7.73 (1H, m). |
| | 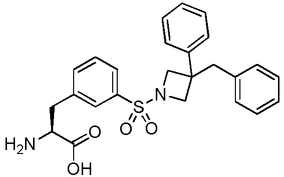 | |
| | | MS (m/z): 451 (M+H)⁺. |

### [Example 17]

### (S)-2-Amino-3-(3-((3-(4-carbamoylphenoxy)-3-phenylazetidin-1-yl)sulfonyl)phenyl)propanoic acid hydrochloride

The same procedures as in Example 1 were performed to obtain the compound of Example 17. However, the following procedures were performed for Step 8.

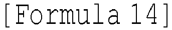

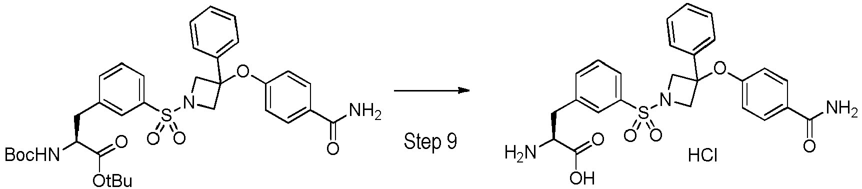

### [Step 9]

(S)-tert-Butyl 2-((tert-butoxycarbonyl)amino)-3-(3-((3-(4-carbamoylphenoxy)-3-phenylazetidin-1-yl)sulfonyl)phenyl)propanoate (58 mg) was dissolved in a solution (2 ml) of 1 N hydrochloric acid/acetic acid, and stirred at room temperature for 6 hours, and then concentrated under reduced pressure. The residue was dissolved by addition of acetic acid, then ethyl acetate was added thereto to form a suspension. The precipitate was collected by filtration to obtain the title compound (42 mg).
¹H-NMR (DMSO-d₆) δ: 3.16 (1H, dd, J = 14. 6, 6.8 Hz), 3.23 (1H, dd, J = 14.3, 6.8 Hz), 4.10 (1H, d, J = 9.3 Hz), 4.12 (1H, d, J = 9.0 Hz), 4.20 (1H, br s), 4.35 (1H, d, J = 8.5 Hz), 4.38 (1H, d, J = 8.5 Hz), 6.45-6.50 (2H, m), 7.17 (1H, s), 7.24-7.36 (5H, m), 7.61-7.67 (3H, m), 7.68-7.73 (1H, m), 7.73-7.83 (3H, m), 7.95-8.48 (3H, br m). MS (m/z) : 496 (M+H)⁺.

### [Example 18]

### (S)-2-Amino-3-(3-((3-(2-cyanophenoxy)-3-phenylazetidin-1-yl)sulfonyl)phenyl)propanoic acid

The same procedures as in Example 1 were performed to obtain the compound of Example 18. However, the following procedures were performed for Step 3.

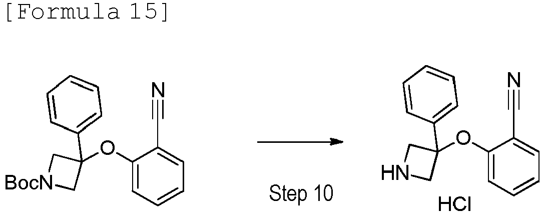

### [Step 10]

### 2-((3-Phenylazetidin-3-yl)oxy)benzonitrile hydrochloride

To a tert-butyl 3-(2-cyanophenoxy)-3-phenylazetidin-1-carboxylate (515 mg), a solution (1.8 ml) of 4 N hydrochloric acid/ethyl acetate was added, and the mixture was stirred at room temperature for 1 hour. The solvent was distilled off under reduced pressure, and to the obtained residue, ethyl acetate/n-hexane was added, and the precipitate was collected by filtration to obtain the title compound (383 mg).
¹H-NMR (CDCl₃) δ: 4.49 (2H, d, J = 12.8 Hz), 4.68 (2H, d, J = 12.8 Hz), 6.49 (1H, d, J = 8.3 Hz), 7.07-7.12 (1H, m), 7.36-7.42 (1H, m), 7.43-7.49 (3H, m), 7.56-7.61 (2H, m), 7.81 (1H, dd, J = 7.7, 1.6 Hz).
MS (m/z) : 251 (M+H)⁺.

**[Table 6]**

| Example | Name and Structure | Equipment data |
|---|---|---|
| 18 | (S)-2-Amino-3-(3-((3-(2-cyanophenoxy)-3-phenylazetidin-1-yl)sulfonyl)phenyl)propanoic acid | ¹H-NMR (DMSO-d₆) δ: 2.94 (1H, dd, J = 14.1, 7.8 Hz), 3.20 (1H, dd, J = 14.2, 4.6 Hz), 3.37-3.43 (1H, m), 4.17 (1H, d, J = 9.5 Hz), 4.18 (1H, d, J = 9.8 Hz), 4.39-4.45 (2H, m), 6.34 (1H, d, J = 8.3 Hz), 7.01-7.06 (1H, m), 7.23-7.40 (7H, m), 7.56 (1H, t, J = 7.7 Hz), 7.67-7.74 (3H, m), 7.80 (1H, br s). |
| | 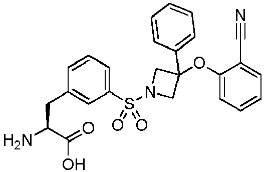 | |
| | | MS (m/z): 478 (M+H)⁺. |

The same procedures as in Example 18 above were performed to obtain the compounds below.

**[Table 7]**

| Example | Name and Structure | Equipment data |
|---|---|---|
| 19 | (S)-2-Amino-3 -(3 -((3 -(4-bromophenoxy)-3-phenylazetidin-1-yl)sulfonyl)phenyl)propanoic acid | ¹H-NMR (DMSO-d₆) δ: 2.95 (1H, dd, J = 14.2, 7.7 Hz), 3.21 (1H, dd, J = 14.1, 5.0 Hz), 3.42 (1H, dd, J = 7.4, 5.1 Hz), 4.09 (1H, d, J = 9.5 Hz), 4.11 (1H, d, J = 9.5 Hz), 4.31 (2H, d, J = 9.8 Hz), 6.42 (2H, dd, J = 6.8, 2.3 Hz), 7.23-7.34 (9H, m), 7.58 (1H, t, J = 7.7 Hz), 7.69 (2H, d, J = 7.0 Hz), 7.78 (1H, br s). |
| | 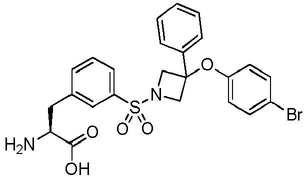 | |
| | | MS (m/z): 553 (M+Na)⁺. |
| 20 | (S)-2-Amino-3 -(3 -((3 -(3 -cyanophenoxy)-3-phenylazetidin-1-yl)sulfonyl)phenyl)propanoic acid | ¹H-NMR (DMSO-d₆) δ: 2.95 (1H, dd, J = 14.1, 7.5 Hz), 3.20 (1H, dd, J = 14.2, 5.1 Hz), 3.42 (1H, dd, J = 7.4, 5.1 Hz), 4.13 (1H, d, J = 9.8 Hz), 4.14 (1H, d, J = 9.8 Hz), 4.36-4.41 (2H, m), 6.77-6.83 (1H, m), 6.87-6.90 (1H, m), 7.24-7.36 (7H, m), 7.58 (1H, t, J = 7.7 Hz), 7.67-7.73 (2H, m), 7.78 (1H, s). |
| | 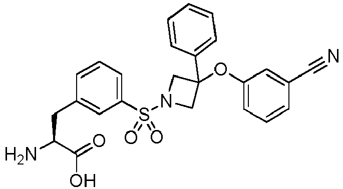 | |
| | | MS (m/z): 478 (M+H)⁺. |
| 21 | (S)-2-Amino-3-(3-((3-phenyl-3-(3-(trifluoromethyl)phenoxy)azetidin-1-yl)sulfonyl)phenyl)propanoic acid | ¹H-NMR (DMSO-d₆) δ: 2.94 (1H, dd, J = 14.1, 7.8 Hz), 3.19 (1H, dd, J = 14.1, 5.3 Hz), 3.41 (1H, dd, J = 7.5, 5.3 Hz), 4.13 (1H, d, J = 9.8 Hz), 4.15 (1H, d, J = 9.8 Hz), 4.38 (2H, d, J = 10.0 Hz), |
| | 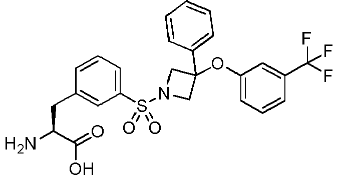 | 6.64 (1H, dd, J = 8.3, 2.3 Hz), 6.79 (1H, br s), 7.20-7.25 (1H, m), 7.26-7.37 (6H, m), 7.57 (1H, t, J = 7.8 Hz), 7.65-7.73 (2H, m), 7.79 (1H, br s). |
| | | MS (m/z): 543 (M+Na)⁺. |

The same procedures as in Example 1 were performed to obtain the compounds below. However, the same procedures as in Steps 10 and 9 were performed for Steps 3 and 8, respectively.

**[Table 8]**

| Example | Name and Structure | Equipment data |
|---|---|---|
| 22 | (S)-2-Amino-3-(3-((3-phenyl-3-(pyridin-3-yloxy)azetidin-1-yl)sulfonyl)phenyl)propanoic acid dihydrochloride | ¹H-NMR (CD₃OD) δ: 3.28-3.42 (2H, m), 4.30-4.38 (3H, m), 4.44 (2H, br d, J = 9.8 Hz), 7.31-7.40 (5H, m), 7.58-7.81 (4H, m), 7.86-7.91 (2H, m), 8.26 (1H, br s), 8.36-8.40 (1H, m). |
| | 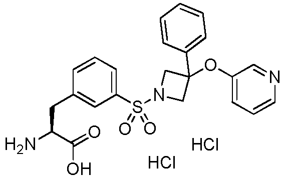 | |
| | | MS (m/z) : 454 (M+H)⁺. |
| 23 | (S)-2-Amino-3-(3-((3-phenyl-3-(pyridin-4-yloxy)azetidin-1-yl)sulfonyl)phenyl)propanoic acid dihydrochloride | ¹H-NMR (CD₃OD) δ: 3.28-3.43 (2H, m), 4.31 (1H, br t, J = 6.7 Hz), 4.39 (1H, d, J = 10 . 0 Hz), 4.40 (1H, d, J = 10.0 Hz), 4.48 (2H, br d, J = 10.5 Hz), 7.11-7.16 (2H, m), 7.27-7.41 (5H, m), 7.65-7.75 (2H, m), 7.86-7.92 (2H, m), 8.50-8.55 (2H, m). |
| | 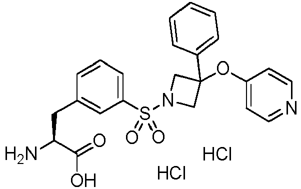 | |
| | | MS (m/z) : 454 (M+H)⁺. |

### [Example 24]

The same procedures as in Example 1 were performed to obtain the compound of Example 24. However, the following procedures were performed for Step 2.

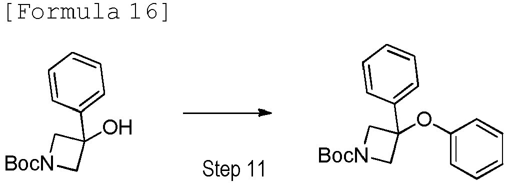

### [Step 11]

### tert-Butyl 3-phenoxy-3-phenylazetidin-1-carboxylate

To a solution of tert-butyl 3-hydroxy-3-phenylazetidin-1-carboxylate (200 mg) in dichloromethane (4.0 ml), N,N-dicyclohexylmethylamine (0.341 ml), copper(II) acetate (29.5 mg) and triphenylbismuth(V) diacetate (900 mg) were added, and the mixture was stirred at room temperature overnight. The reaction solution was diluted with ethyl acetate, and washed with 1 N hydrochloric acid, water, and saturated saline, sequentially. The organic layer was dried over sodium sulfate, then the sodium sulfate was filtered off, and the filtrate was concentrated under reduced pressure. The obtained residue was purified by silica gel column chromatography (n-hexane/ethyl acetate) to obtain the title compound (227 mg).
¹H-NMR (CDCl₃) δ: 1.47 (9H, s), 4.28 (2H, d, J = 8.5 Hz), 4.37 (2H, d, J = 10.0 Hz), 6.56 (2H, dt, J = 7.4, 1.3 Hz), 6.88-6.92 (1H, m), 7.15-7.19 (2H, m), 7.30 (1H, dt, J = 7.4, 1.8 Hz), 7.34-7.38 (2H, m), 7.44-7.49 (2H, m). MS (m/z) : 226 (M-99)⁺.

**[Table 9]**

| Example | Name and Structure | Equipment data |
|---|---|---|
| 24 | (S)-2-Amino-3-(3-((3-(3-phenoxy-3 - phenylazetidin-1-yl)sulfonyl)phenyl)propanoic acid | ¹H-NMR (DMSO-d₆) δ: 2.94 (1H, dd, J = 13.9, 7.7 Hz), 3.16-3.24 (1H, m), 4.09 ( 1H, d, J = 9.7 Hz), 4.10 (1H, d, J = 9.7 Hz), 4.31 (2H, d, J = 9.8 Hz), 6.44 (2H, dd, J = 8.7, 0.9 Hz), 6.86 (1H, t, J = 7.4 Hz), 7.09-7.16 (2H, m), 7.23-7.34 (5H, m), 7.58 (1H, t, J = 7.8 Hz), 7.67-7.73 (2H, m), 7.78 (1H, br s), signal of one proton was overlapped with H₂O signal. |
| | 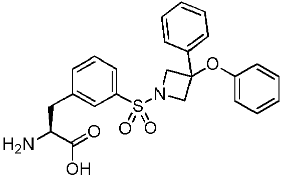 | |
| | | MS (m/z) : 453 (M+H)⁺. |

The same procedures as in Example 24 above were performed to obtain the compounds below. However, for Step 11 of Example 28, the same procedures as in Step 2 were performed. Furthermore, for Example 29, Step 3 and later steps were performed using commercially available tert-butyl 3-hydroxy-3-(trifluoromethyl)azetidin-1-carboxylate as a raw material.

**[Table 10]**

| Example | Name and Structure | Equipment data |
|---|---|---|
| 25 | (S)-2-Amino-3 -(3 -((3 -neopentyl-3 - phenoxyazetidin-1-yl)sulfonyl)phenyl)propanoic acid | ¹H-NMR (DMSO-d₆) δ: 0.76 (9H, s), 1.74 (2H, s), 2.93 (1H, dd, J = 14.2, 7.4 Hz), 3.17 (1H, dd, J = 14.1, 5.0 Hz), 3.42 (1H, dd, J = 7.4, 5.1 Hz), 3.89 (2H, d, J = 9.5 Hz), 4.01 (1H, d, J = 9.3 Hz), 4.01 (1H, d, J = 9.5 Hz), 6.61 (2H, |
| | 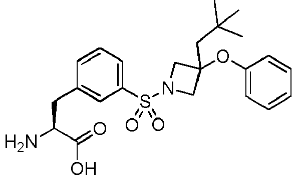 | dd, J = 8.8, 1.0 Hz), 6.91 (1H, t, J = 7.4 Hz), 7.20-7.24 (2H, m), 7.57 (1H, t, J = 7.7 Hz), 7.66 (2H, dt, J = 8.2, 2.8 Hz), 7.71 (1H, s). |
| | | MS (m/z) : 447 (M+H)⁺. |
| 26 | (S)-2-Amino-3-(3-((3-(3-fluorophenyl)-3-phenoxyazetidin-1-yl)sulfonyl)phenyl)propanoic acid | ¹H-NMR (DMSO-d₆) δ: 2.95 (1H, dd, J = 14.2, 7.4 Hz), 3.20 (1H, dd, J = 14.2, 5.1 Hz), 3.42 (1H, dd, J = 7.5, 5.3 Hz), 4.10 (2H, d, J = 9.8 Hz), 4.34 (1H, d, J = 9.8 Hz), 4.36 (1H, d, J = 9.5 Hz), 6.44-6.47 (2H, m), 6.88 (1H, t, J = 7.3 Hz), 7.04 (1H, dt, J = 10.3, 2.1 Hz), 7.08-7.19 (4H, m), 7.32-7.41 (1H, m), 7.57 (1H, t, J = 7.7 Hz), 7.66-7.72 (2H, m), 7.77-7.80 (1H,m). |
| | 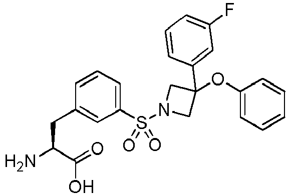 | |
| | | MS (m/z): 471 (M+H)⁺. |
| 27 | (S)-2-Amino-3-(3-((3-(2-fluorophenyl)-3-phenoxyazetidin-1-yl)sulfonyl)phenyl)propanoic acid | ¹H-NMR (DMSO-d₆) δ: 2.91 (1H, dd, J = 13.9, 7.7 Hz), 3.17 (1H, dd, J = 14.2, 5.1 Hz), 3.40 (1H, dd, J = 7.5, 5.0 Hz), 4.20-4.23 (1H, m), 4.21-4.24 (1H, m), 4.50 (2H, d, J = 9.8 Hz), 6.51-6.57 (2H, m), 6.88 (1H, t, J = 7.3 Hz), 7.08-7.17 (4H, m), 7.33-7.61 (6H, m), 7.67 (1H, d, J = 8.0 Hz), 7.74 (1H, br s). |
| | 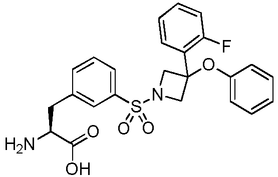 | |
| | | MS (m/z) : 471 (M+H)⁺. |

**[Table 11]**

| | | |
|---|---|---|
| 28 | (S)-2-Amino-3 -(3 -((3 -(3 -fluorophenoxy)-3-phenylazetidin-1-yl)sulfonyl)phenyl)propanoic acid-ds | ¹H-NMR (CD₃OD) δ: 3.11 (1H, dd, J = 14.6, 8.3 Hz), 3.37 (1H, dd, J = 14.7, 4.9 Hz), 3.81 (1H, dd, J = 8.3, 4.8 Hz), 4.14-4.21 (2H, m), 4.31 (2H, br d, J = 9.8 Hz), 6.16-6.24 (2H, m), 6.61 (1H, tdd, J = 8.4, 2.4, 0.8 Hz), 7.06-7.13 (1H, m), 7.64 (1H, t, J = 7.7 Hz), 7.69-7.74 (1H, m), 7.82 (1H, dt, J = 7.7, 1.4 Hz), 7.86-7.89 (1H, m). |
| | 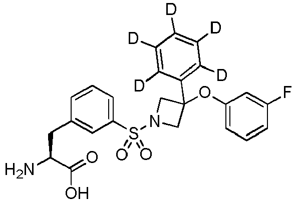 | |
| | | MS (m/z): 476 (M+H)⁺. |
| 29 | (S)-2-Amino-3 -(3 -((3 -phenoxy-3 - (trifluoromethyl)azetidin-1-yl)sulfonyl)phenyl)propanoic acid | ¹H-NMR (DMSO-d₆) δ: 2.91 (1H, dd, J = 14.2, 7.4 Hz), 3.16 (1H, dd, J = 14.3, 5.3 Hz), 3.40 (1H, dd, J = 7.3, 5.3 Hz), 3.96 (2H, d, J = 10.5 Hz), 4.23 (1H, d, J = 10.5 Hz), 4.24 (1H, d, J = 10.5 Hz), 6.73-6.78 (2H, m), 7.11-7.16 (1H, m), |
| | 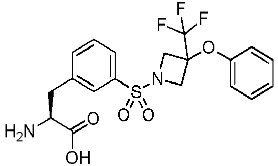 | 7.24-7.30 (2H, m), 7.48-7.54 (1H, m), 7.57 (1H, dt, J = 7.8, 1.5 Hz), 7.63-7.67 (2H, m). |
| | | MS (m/z): 445 (M+H)⁺. |

The same procedures as in Example 1 were performed to obtain the compound below. However, the same procedures as in Steps 11 and 9 were performed for Steps 2 and 8, respectively.

**[Table 12]**

| Example | Name and Structure | Equipment data |
|---|---|---|
| 30 | (S)-2-Amino-3 -(3 -((3 -methyl-3 - phenoxyazetidin-1-yl)sulfonyl)phenyl)propanoic acid hydrochloride | ¹H-NMR (DMSO-d₆) δ: 1.40 (3H, s), 3.19-3.38 (2H, m), 3.80-3.88 (2H, m), 3.93 (1H, d, J = 8.0 Hz), 3.95 (1H, d, J = 8.8 Hz), 4.17-4.26 (1H, m), 6.62-6.68 (2H, m), 6.93-6.99 (1H, m), 7.20-7.27 (2H, m), 7.60-7.72 (2H, m), 7.72-7.78 (2H, m), 8.41 (2H, br s). |
| | 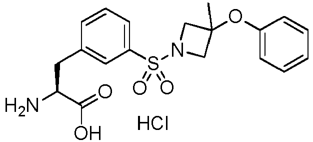 | |
| | | MS (m/z) : 391 (M+H)⁺. |

The same procedures as in Example 1 were performed to obtain the compound below. However, the same procedures as in Steps 11 and 10 were performed for Steps 2 and 3, respectively.

**[Table 13]**

| Example | Name and Structure | Equipment data |
|---|---|---|
| 31 | (S)-2-Amino-3-(3-((3-cyclopropyl-3-phenoxyazetidin-1-yl)sulfonyl)phenyl)propanoic acid | ¹H-NMR (DMSO-d₆) δ: 0.22-0.28 (2H, m), 0.41-0.47 (2H, m), 1.27-1.35 (1H, m), 2.93 (1H, dd, J = 14.2, 7.7 Hz), 3.20 |
| | 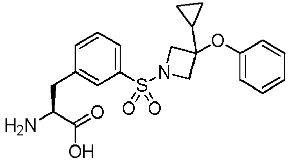 | (1H, dd, J = 14.2, 5.1 Hz), 3.40 (1H, dd, J = 7.5, 5.0 Hz), 3.71-3.78 (4H, m), 6.71-6.76 (2H, m), 6.94-6.99 (1H, m), 7.20-7.26 (2H, m), 7.57 (1H, t, J = 7.5 Hz), MS (m/z) : 417 (M+H)⁺. (1H, m). |

### [Example 32]

The same procedures as in Example 1 were performed to obtain the compound of Example 32. However, the following procedures were performed for Step 1, and the same procedures as in Steps 11 and 10 were performed for Steps 2 and 3, respectively.

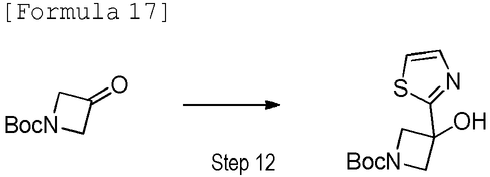

### [Step 12]

### tert-Butyl 3-hydroxy-3-(thiazol-2-yl)azetidin-1-carboxylate

Under an argon atmosphere, to a solution of n-butyl lithium (1.55 M/hexane solution, 1.73 ml) in diethyl ether (6 ml), 2-bromothiazole (0.24 ml) was added at - 78°C. After the mixture was stirred at the same temperature for 15 minutes, a solution of 1-(tert-butoxycarbonyl)-3-azetidinone (400 mg) in diethyl ether (4 ml) was slowly added. The reaction solution was warmed to room temperature and stirred for another 24 hours. To the reaction solution, a saturated aqueous ammonium chloride solution was added, and the mixture was diluted with ethyl acetate and water. The organic layer was washed with 1 N hydrochloric acid and saturated saline sequentially, and then dried over sodium sulfate. The sodium sulfate was filtered off, and the filtrate was concentrated under reduced pressure. The residue was purified by silica gel column chromatography (chloroform/methanol) to obtain the title compound (468 mg) .
¹H-NMR (CDCl₃) δ: 1.47 (9H, s), 3.88 (1H, s), 4.22-4.26 (2H, m), 4.34 (1H, d, J = 9.3 Hz), 4.34 (1H, d, J = 9.3 Hz), 7.39 (1H, d, J = 3.3 Hz), 7.76 (1H, d, J = 3.3 Hz). MS (m/z) : 279 (M+Na)⁺.

**[Table 14]**

| Example | Name and Structure | Equipment data |
|---|---|---|
| 32 | (S)-2-Amino-3 -(3 -((3 -phenoxy-3 - (thiazol-2-yl)azetidin-1-yl)sulfonyl)phenyl)propanoic acid | ¹H-NMR (DMSO-d₆) δ: 2.98 (1H, dd, J = 14.2, 7.4 Hz), 3.21 (1H, dd, J = 14.3, 5.0 Hz), 3.49 (1H, dd, J = 7.4, 4.9 Hz), 4.18 (1H, d, J = 9.4 Hz), 4.20 (1H, d, J = 9.3 Hz), 4.48 (1H, d, J = 9.3 Hz), 4.49 (1H, d, J = 9.3 Hz), 6.48-6.53 (2H, m), 6.90-6.96 (1H, m), 7.15-7.21 (2H, m), 7.56 (1H, t, J = 7.7 Hz), 7.65-7.71 (2H, m), 7.76-7.78 (1H, m), 7.77 (1H, d, J = 3.3 Hz), 7.79 (1H, d, J = 3.3 Hz). |
| | 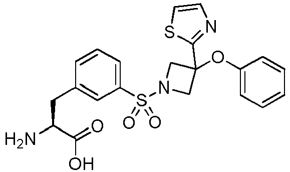 | |
| | | MS (m/z) : 460 (M+H)⁺. |

The same procedures as in Example 32 above were performed to obtain the compound below. However, the same procedures as in Step 9 were performed for Step 8.

**[Table 15]**

| Example | Name and Structure | Equipment data |
|---|---|---|
| 33 | (S)-2-Amino-3 -(3 -((3 -phenoxy-3 - (pyridin-2-yl)azetidin-1 - yl)sulfonyl)phenyl)propanoic acid dihydrochloride | ¹H-NMR (DMSO-d₆) δ: 3.11-3.25 (2H, m), 4.07 (1H, d, J = 8.8 Hz), 4.09 (1H, d, J = 8.8 Hz), 4.20-4.30 (1H, br m), 4.51 (1H. d, J = 8.5 Hz), 4.53 (1H, d, J = 8.8 Hz), 6.36-6.42 (2H, m), 6.87 (1H, t, J = 7.4 Hz), 7.11-7.17 (2H, m), 7.24-7.28 (1H, m), 7.29-7.34 (1H, m), 7.60-7.70 (2H, m), 7.70-7.80 (3H, m), 8.26-8.40 (3H, br m), 8.50-8.53 (1H, m). |
| | 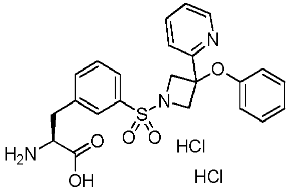 | |
| | | MS (m/z) : 454 (M+H)⁺. |

The same procedures as in Example 1 were performed to obtain the compounds below. However, the procedures as in Steps 12, 10 and 9 were performed for Steps 1, 3 and 8, respectively.

**[Table 16]**

| Example | Name and Structure | Equipment data |
|---|---|---|
| 34 | (S)-2-Amino-3-(3-((3-(2-fluorophenoxy)-3 -(pyridin-2-yl)azetidin-1-yl)sulfonyl)phenyl)propanoic acid dihydrochloride | ¹H-NMR (DMSO-d₆) δ: 3.12-3.26 (2H, m), 4.12 (1H, d, J = 9.0 Hz), 4.15 (1H, d, J = 8.8 Hz), 4.19-4.28 (1H, br m), 4.53 (1H, d, J = 9.3 Hz), 4.55 (1H, d, J = 9.0 Hz), 6.22-6.29 (1H, m), 6.82-6.94 (2H, m), 7.15-7.22 (1H, m), 7.29-7.35 (2H, m), 7.61 (1H, t, J = 7.8 Hz), 7.64-7.70 (1H, m), 7.73-7.80 (3H, m), 8.28-8.46 (3H, m), 8.51 (1H, d, J = 4.5 Hz). |
| | 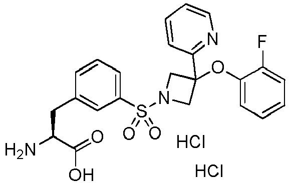 | |
| | | MS (m/z): 472 (M+H)⁺. |
| 35 | (S)-2-Amino-3-(3-((3-(3-fluorophenoxy)-3-(pyridin-2-yl)azetidin-1-yl)sulfonyl)phenyl)propanoic acid dihydrochloride | ¹H-NMR (DMSO-d₆) δ: 3.12-3.25 (2H, m), 4.08 (1H, d, J = 9.5 Hz), 4.11 (1H, d, J = 9.8 Hz), 4.20-4.29 (1H, m), 4.53 (1H, d, J = 8.5 Hz), 4.55 (1H, d, J = 8.8 Hz), 6.17-6.22 (1H, m), 6.26-6.32 (1H, m), 6.70-6.76 (1H, m), 7.13-7.20 (1H, m), 7.26-7.30 (1H, m), 7.30-7.36 (1H, m), 7.60-7.71 (2H, m), 7.72-7.81 (3H, m), 8.28-8.44 (3H, m), 8.51-8.55 (1H, m). |
| | 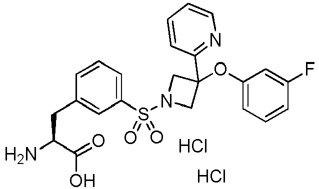 | MS (m/z): 472 (M+H)⁺. |

### [Example 36]

The same procedures as in Example 1 were performed to obtain the compound of Example 36. However, the following procedures were performed in place of Steps 1 and 2.

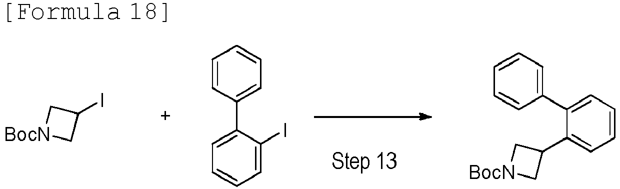

### [Step 13]

### tert-Butyl 3-([1,1'-biphenyl]-2-yl)azetidin-1-carboxylate

Under a nitrogen atmosphere, 1,2-dibromoethane (59 µl) was added to a suspension of zinc powder (0.381 g) in N,N-dimethylformamide (10 ml), and then the mixture was stirred at 70°C for 3 minutes and at room temperature for 30 minutes. To the reaction solution, chlorotrimethylsilane (88 µl) was added. After the mixture was stirred for 15 minutes, 1-(tert-butoxycarbonyl)-3-(iodo)azetidine (1.5 g) in N,N-dimethylformamide (10 ml) was slowly added, and then the reaction solution was stirred for 60 minutes. To the reaction solution, a solution of tris(dibenzylideneacetone) dipalladium(0) (97 mg), tri(2-furyl) phosphine (74 mg) in N,N-dimethylformamide (2 ml) was added, and the mixture was stirred for 10 minutes. After the addition of a solution of 2-iodobiphenyl (1.48 g) in N,N-dimethylformamide (10 ml), the mixture was stirred at 65°C for 4 hours and at room temperature overnight. The reaction solution was diluted with diethyl ether, then washed with a saturated aqueous ammonium chloride solution and saturated saline sequentially, and then dried over sodium sulfate. After the sodium sulfate was filtered off, the filtrate was concentrated under reduced pressure, and the obtained residue was purified by silica gel column chromatography (n-hexane/ethyl acetate) to obtain the title compound (583 mg).
¹H-NMR (CDCl₃) δ: 1.43 (9H, s), 3.88-3.95 (3H, m), 4.00-4.09 (2H, m), 7.18-7.22 (2H, m), 7.24 (1H, dd, J = 7.5, 1.3 Hz), 7.31 (1H, td, J = 7.4, 1.3 Hz), 7.33-7.46 (4H, m), 7.57 (1H, dd, J = 7.8, 1.0 Hz).
MS (m/z) : 254 (M-55)⁺.

**[Table 17]**

| Example | Name and Structure | Equipment data |
|---|---|---|
| 36 | (S)-3-(3-((3-([1,1'-biphenyl]-2-yl)azetidin-1-yl)sulfonyl)phenyl)-2-aminopropanoic acid | ¹H-NMR (DMSO-d₆) δ: 2.98 (1H, dd, J = 14.1, 7.5 Hz), 3.22 (1H, dd, J = 14.2, 5.1 Hz), 3.42 (1H, dd, J = 7.5, 5.0 Hz), 3.60-3.66 (2H, m), 3.68-3.77 (1H, m), 3.80 (1H, d, J = 7.3 Hz), 3.84 (1H, d, J = 7.5 Hz), 7.09-7.20 (4H, m), 7.30 (2H, ttd, J = 7.3, 7.3, 1.8 Hz), 7.34-7.45 (3H, m), 7.58 (1H, t, J = 7.5 Hz), 7.62 (1H, dt, J = 7.8, 1.6 Hz), 7.64-7.69 (1H, m), 7.69-7.72 (1H, m). |
| | 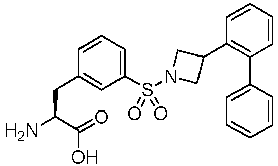 | |
| | | MS (m/z) : 437 (M+H)⁺. |

### [Example 37]

The same procedures as in Example 1 were performed to obtain the compound of Example 37. However, the following procedures were performed in place of Steps 1 and 2.

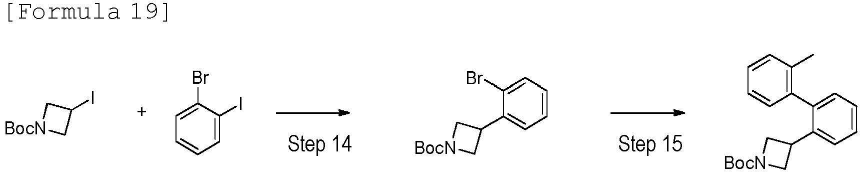

### [Step 14]

### tert-Butyl 3-(2-bromophenyl)azetidin-1-carboxylate

Under a nitrogen atmosphere, 1,2-dibromoethane (59 µl) was added to a suspension of zinc powder (0.450 g) in N,N-dimethylformamide (10 ml), and then the mixture was stirred at 70°C for 3 minutes and at room temperature for 30 minutes. To the reaction solution, chlorotrimethylsilane (88 µl) was added. After the mixture was stirred for 15 minutes, 1-(tert-butoxycarbonyl)-3-(iodo)azetidine (1.5 g) in N,N-dimethylformamide (10 ml) was slowly added, and then the reaction solution was stirred for 60 minutes. To the reaction solution, a solution of tris(dibenzylideneacetone) dipalladium(0) (0.291 g), tri(2-furyl) phosphine (0.221 g) in N,N-dimethylformamide (2 ml) was added, and the mixture was stirred for 10 minutes. After the addition of a solution of 1-bromo-2-iodobenzene (0.795 ml) in N,N-dimethylformamide (10 ml), the mixture was stirred at 65°C for 4 hours and at room temperature overnight. The reaction solution was diluted with diethyl ether, then washed with saturated aqueous ammonium chloride solution and saturated saline sequentially, and then dried over sodium sulfate. After the sodium sulfate was filtered off, the filtrate was concentrated under reduced pressure and the obtained residue was purified by silica gel column chromatography (n-hexane/ethyl acetate) to obtain the title compound (1.01 g).
¹H-NMR (CDCl₃) δ: 1.46 (9H, s), 3.95-4.14 (3H, m), 4.36 (2H, dd, J = 8.3, 8.3 Hz), 7.13 (1H, td, J = 7.5, 1.8 Hz), 7.35 (1H, td, J = 7.5, 1.1 Hz), 7.39-7.43 (1H, m), 7.56 (1H, dd, J = 8.0, 1.3 Hz) .
MS (m/z) : 256 (M-55)⁺⁺.

### [Step 15]

### tert-Butyl 3-(2'-methyl-[1,1'-biphenyl]-2-yl)azetidin-1-carboxylate

A mixed solution of the compound obtained by the method of Step 14 (355 mg), o-tolyl boronic acid (186 mg), tetrakis(triphenylphosphine)palladium(0) (131 mg), cesium carbonate (741 mg), 1,4-dioxane (1.5 ml), and water (0.4 ml) was stirred under microwave irradiation at 120°C for 40 minutes. The reaction solution was diluted with ethyl acetate, washed with water and saturated saline, and dried over sodium sulfate. After the sodium sulfate was filtered off, the filtrate was concentrated under reduced pressure, and the obtained residue was purified by silica gel column chromatography (n-hexane/ethyl acetate) to obtain the title compound (304 mg).
¹H-NMR (CDCl₃) δ: 1.42 (9H, s), 2.00 (3H, s), 3.57 (1H, tt, J = 8.8, 6.5 Hz), 3.83-4.04 (4H, m), 7.02 (1H, br d, J = 7.3 Hz), 7.12 (1H, dd, J = 7.5, 1.0 Hz), 7.17-7.32 (4H, m), 7.42 (1H, td, J = 7.5, 1.3 Hz), 7.56-7.61 (1H, m) .
MS (m/z) : 346 (M+Na)⁺.

**[Table 18]**

| Example | Name and Structure | Equipment data |
|---|---|---|
| 37 | (S)-2-Amino-3-(3-((3-(2'-methy1-[1,1'-biphenyl]-2-yl)azetidin-1-yl)sulfonyl)phenyl)propanoic acid | ¹H-NMR (DMSO-d₆) δ: 1.87 (3H, s), 2.87-2.96 (1H, m), 3.19 (1H, dd, J = 13.9, 4.6 Hz), 3.37-3.45 (1H, m), 3.52-3.62 (2H, m), 3.64-3.73 (2H, m), 6.92-6.97 (1H, m), 7.02 (1H, dd, J = 7.2, 1.6 Hz), 7.04-7.09 (1H, m), 7.18-7.32 (5H, m), 7.53-7.61 (2H, m), 7.62-7.69 (2H, m), signal of one proton was overlapped with H₂O signal. |
| | 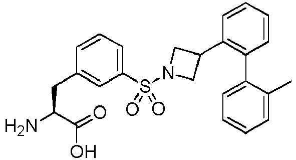 | MS (m/z): 451 (M+H)⁺. |

The same procedures as in Example 1 were performed to obtain the compound of Example 38. However, the following procedures were performed in place of Steps 1 and 2.

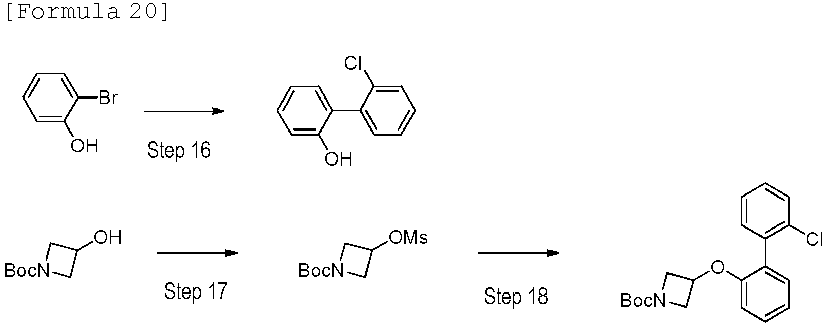

### [Step 16]

### 2-Chloro-[1,1'-biphenyl]-2-ol

To a mixed solution of 2-bromophenol (200 mg) in 1,4-dioxane (3 ml) and water (840 µl), tetrakis(triphenylphosphine)palladium(0) (134 mg), cesium carbonate (753 mg) and 2-chlorophenylboronic acid (271 mg) were added at room temperature, and the mixture was stirred at 120°C for 3 hours. Water was added to the reaction solution, and the mixture was extracted with ethyl acetate. The extract was washed with saturated saline, and dried over sodium sulfate. After the sodium sulfate was filtered off, the filtrate was concentrated under reduced pressure, and the obtained residue was purified by silica gel chromatography (n-hexane/ethyl acetate) to obtain the title compound (206 mg).
¹H-NMR (CDCl₃) δ: 4.77 (1H, s), 6.98-7.04 (2H, m), 7.17 (1H, dd, J = 8.0, 1.5 Hz), 7.29-7.39 (4H, m), 7.51-7.56 (1H, m).
MS (m/z): 205 (M+H)⁺.

### [Step 17]

### tert-Butyl 3-((methylsulfonyl)oxy)azetidin-1-carboxylate

To a solution of tert-butyl 3-hydroxyazetidin-1-carboxylate (500 mg) and triethylamine (805 µl) in dichloromethane (5 ml), methanesulfonyl chloride (247 µl) was added at 0°C, and the mixture was stirred at room temperature for 3 days. The reaction solution was diluted with chloroform, then the organic layer was washed with 1 N hydrochloric acid and dried over sodium sulfate. After the sodium sulfate was filtered off, the filtrate was concentrated under reduced pressure, and the obtained residue was purified by silica gel chromatography (chloroform/methanol) to obtain the title compound (774 mg).
¹H-NMR (CDCl₃) δ: 1.44 (9H, s), 3.06 (3H, s), 4.10 (2H, ddd, J = 10.4, 4.1, 1.1 Hz), 4.27 (2H, ddd, J = 10.4, 6.6, 1.2 Hz), 5.15-5.23 (1H, m).
MS (m/z): 274 (M+Na)⁺.

### [Step 18]

### tert-Butyl 3-((2'-chloro-[1,1'-biphenyl]-2-yl)oxy)azetidin-1-carboxylate

To a solution of the compound obtained by the method of Step 17 (108 mg) in N,N-dimethylformamide (2 ml), the compound obtained by the method of Step 16 (73 mg) and cesium carbonate (128 mg) were added at room temperature, and the mixture was stirred at 120°C for 2 days. To the reaction solution, saturated saline was added, and then the mixture was extracted with ethyl acetate. The extract was washed with saturated saline, and dried over sodium sulfate. After the sodium sulfate was filtered off, the filtrate was concentrated under reduced pressure, and the obtained residue was purified by silica gel chromatography (n-hexane/ethyl acetate) to obtain the title compound (100 mg).
¹H-NMR (CDCl₃) δ: 1.42 (9H, s), 3.88-3.94 (2H, m) , 4.19-4.26 (2H, m), 4.83-4.90 (1H, m), 6.61 (1H, dd, J = 8.3, 1.0 Hz), 7.07 (1H, td, J = 7.5, 1.1 Hz), 7.23 (1H, dd, J = 7.5, 1.8 Hz), 7.28-7.36 (4H, m), 7.43-7.48 (1H, m). MS (m/z): 382 (M+Na)⁺.

**[Table 19]**

| Example | Name and Structure | Equipment data |
|---|---|---|
| 38 | (S)-2-Amino-3-(3-((3-((2'-ch1oro-[1,1'-biphenyl]-2-yl)oxy)azetidin-1-yl)sulfonyl)phenyl)propanoic acid | ¹H-NMR (DMSO-d₆) δ: 2.96-3.07 (1H, m), 3.21 (1H, dd, J = 14.1, 4.8 Hz), 3.40-3.55 (3H, m), 4.05-4.14 (1H, m), 4.17-4.26 (1H, m), 4.85-4.91 (1H, m), 6.85 (1H, d, J = 8.3 Hz), 7.02-7.06 (1H, m), 7.12-7.20 (2H, m), 7.29-7.39 (3H, m), 7.45-7.47 (1H, m), 7.56-7.64 (2H, m), 7.67-7.73 (2H, m). |
| | 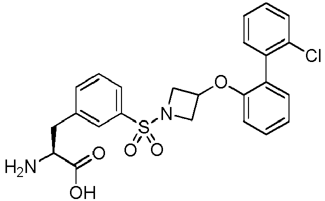 | MS (m/z) : 487 (M+H)⁺. |

The same procedures as in Example 1 were performed to obtain the compound of Example 39. However, the following procedures were performed for Step 2.

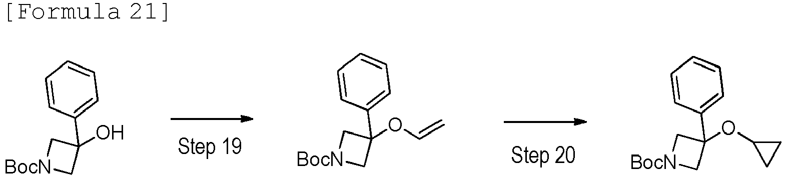

### [Step 19]

### tert-Butyl 3-phenyl-3-(vinyloxy)azetidin-1-carboxylate

To a solution of chloro(1,5-cyclooctadiene) iridium(I) dimer (10.8 mg) and sodium carbonate (102 mg) in toluene (2 ml), tert-butyl 3-hydroxy-3-phenylazetidin-1-carboxylate (400 mg) and vinyl acetate (297 µl) were added at room temperature, and the mixture was stirred at 100°C for 5.5 hours. Water was added to the reaction solution, and the mixture was extracted with diethyl ether. The extract was washed with saturated saline, and dried over sodium sulfate. After the sodium sulfate was filtered off, the filtrate was concentrated under reduced pressure, and the obtained residue was purified by silica gel chromatography (n-hexane/ethyl acetate) to obtain the title compound (324 mg).
¹H-NMR (CDCl₃) δ: 1.47 (9H, s) , 4.14 (1H, dd, J = 6.5, 1.8 Hz), 4.19-4.23 (3H, m), 4.29 (2H, dd, J = 9.4, 0.9 Hz), 6.14 (1H, dd, J = 14.1, 6.5 Hz), 7.30-7.36 (1H, m), 7.37-7.45 (4H, m).
MS (m/z): 298 (M+Na)⁺.

### [Step 20]

### tert-Butyl 3-cyclopropoxy-3-phenylazetidin-1-carboxylate

Under a nitrogen atmosphere, to a solution of the compound obtained by the method of Step 19 (324 mg) and chloroiodomethane (273 µl) in 1,2-dichloroethane (3.5 ml), zinc diethyl (1.0 M/hexane solution, 1.88 ml) was added at 0°C, and the mixture was stirred at 0°C for 10 minutes. The reaction solution was warmed to room temperature, then stirred for 4 hours. In addition, chloroiodomethane (137 µl) and zinc diethyl (1.0 M/hexane solution, 941 µl) were added to the reaction solution at room temperature, and the mixture was stirred for 3 hours. To the reaction solution, a saturated aqueous ammonium chloride solution was added, and the mixture was extracted with chloroform. The extract was dried over sodium sulfate. After the sodium sulfate was filtered off, the filtrate was concentrated under reduced pressure and the obtained residue was purified by silica gel chromatography (n-hexane/ethyl acetate) to obtain the title compound (59.2 mg) .
¹H-NMR (CDCl₃) **δ:** 0.33-0.40 (2H, m) , 0.50-0.59 (2H, m), 1.45 (9H, s), 2.96-3.01 (1H, m), 4.23 (2H, d, J = 8.5 Hz), 4.31 (2H, d, J = 9.0 Hz), 7.31-7.36 (1H, m), 7.38-7.45 (4H, m).
MS (m/z): 312 (M+Na)⁺.

**[Table 20]**

| Example | Name and Structure | Equipment data |
|---|---|---|
| 39 | (S)-2-Amino-3-(3-((3-cyclopropoxy-3-phenylazetidin-1-yl)sulfonyl)phenyl)propanoic acid | ¹H-NMR (DMSO-d₆) δ: 0.20-0.22 (4H, m), 2.76-2.82 (1H, m), 2.96 (1H, dd, J = 14.2, 7.2 Hz), 3.22 (1H, dd, J = 13.9, 5.1 Hz), 3.41 (1H, dd, J = 7.5, 5.3 Hz), 4.07 (4H, br s), 7.24-7.52 (7H, m), 7.57 (1H, t, J = 7.8 Hz), 7.63-7.69 (2H, m), 7.77 (1H, br s). |
| | 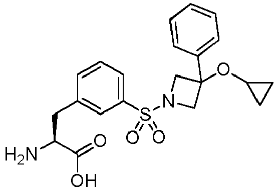 | MS (m/z) : 439 (M+Na)⁺. |

The same procedures as in Example 1 were performed to obtain the compound of Example 40. However, the following procedures were performed for Step 2.

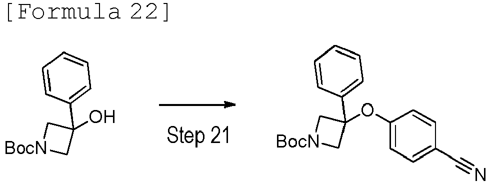

### [Step 21]

### tert-Butyl 3-(4-cyanophenoxy)-3-phenylazetidin-1-carboxylate

To a solution of tert-butyl 3-hydroxy-3-phenylazetidin-1-carboxylate (502 mg) in N,N-dimethylformamide (4 ml), sodium hydride (48.3 mg) was added at 0°C, and the mixture was stirred at 0°C for 20 minutes. To the reaction solution, 4-fluorobenzonitrile (296 mg) was added, and the mixture was stirred at 60°C for 2 hours. Water and a saturated aqueous ammonium chloride solution were added to the reaction product, and the mixture was extracted with ethyl acetate. The extract was washed with saturated saline, and dried over sodium sulfate. After the sodium sulfate was filtered off, the filtrate was concentrated under reduced pressure and the obtained residue was purified by silica gel chromatography (n-hexane/ethyl acetate) to obtain the title compound (497 mg).
1H-NMR (CDCl₃) δ: 1.47 (9H, s) , 4.32 (2H, d, J = 9.5 Hz), 4.36 (2H, d, J = 9.5 Hz), 6.63 (2H, d, J = 8.8 Hz), 7.30-7.35 (1H, m), 7.36-7.43 (4H, m), 7.47 (2H, d, J = 9.0 Hz). MS (m/z) : 373 (M+Na)⁺.

**[Table 21]**

| Example | Name and Structure | Equipment data |
|---|---|---|
| 40 | (S)-2-Amino-3-(3-((3-(4-cyanophenoxy)-3-phenylazetidin-1-yl)sulfonyl)phenyl)propanoic acid | ¹H-NMR (DMSO-d₆) δ: 2.89-3.00 (1H, m), 3.16-3.23 (1H, m), 3.38-3.45 (1H, m), 4.13 (1H, d, J = 9.8 Hz), 4.15 (1H, d, J = 9.8 Hz), 4.36 (1H, d, J = 9.8 Hz), 4.38 (1H, d, J = 9.8 Hz), 6.59-6.65 (2H, m), 7.21-7.36 (5H, m), 7.54-7.65 (3H, m), 7.66-7.72 (2H, m), 7.79 (1H, s). |
| | 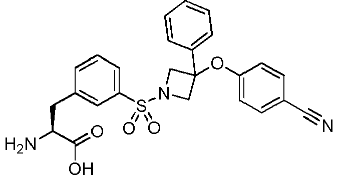 | MS (m/z): 478 (M+H)⁺. |

### [Example 41]

### (S)-2-Amino-3-(3-((3-(4-fluorophenoxy)-3-phenylazetidin-1-yl)sulfonyl)phenyl)propanoic acid

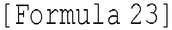

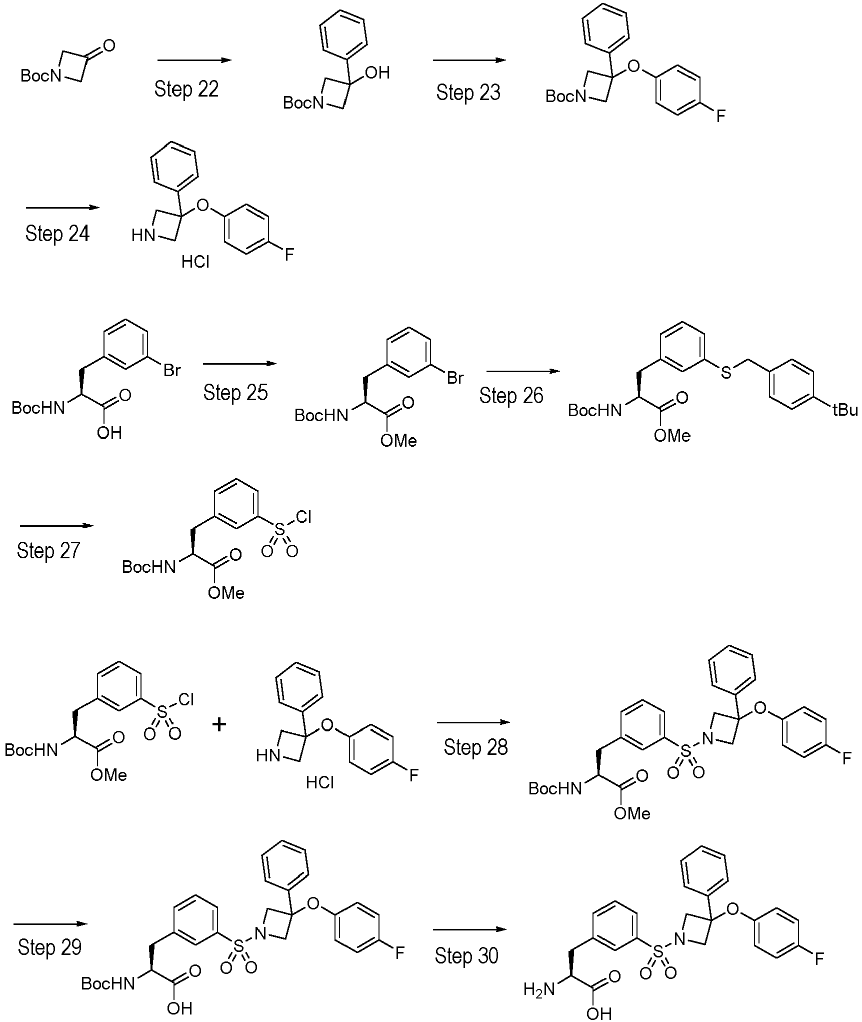

### [Step 22]

### tert-Butyl 3-hydroxy-3-phenylazetidin-1-carboxylate

To a solution of 1-(tert-butoxycarbonyl)-3-azetidinone (5.2 g, PharmaBlock, Inc.) in tetrahydrofuran (75 ml), phenylmagnesium bromide (1.0 M/tetrahydrofuran solution, 45.6 ml) was added under ice-cooling, and the mixture was stirred at room temperature overnight. Under ice cooling, the mixture was neutralized by addition of 1 N hydrochloric acid, and the mixture was extracted with ethyl acetate. The combined organic layer was washed with saturated saline, and dried over sodium sulfate. After the sodium sulfate was filtered off, the filtrate was concentrated under reduced pressure, and the obtained residue was purified by silica gel column chromatography (n-hexane/ethyl acetate) to obtain the title compound (7.0 g).
¹H-NMR (CDCl₃) δ: 1.47 (9H, s), 2.38 (1H, s), 4.17 (2H, d, J = 9.3 Hz), 4.29 (2H, d, J = 9.3 Hz), 7.30-7.36 (1H, m), 7.38-7.44 (2H, m), 7.48-7.52 (2H, m).
MS (m/z) : 272 (M+Na)⁺.

### [Step 23]

### tert-Butyl 3-(4-fluorophenoxy)-3-phenylazetidin-1-carboxylate

To the compound obtained by the method of Step 22 (1.20 g), triphenylphosphine (1.39 g), 4-fluorophenol (1.08 g) and cyclopentylmethyl ether (12 ml) were added, and the mixture was stirred at room temperature to form a homogeneous solution. Diisopropyl azodicarboxylate (1.07 g) was added to the solution under ice-cold stirring, and the mixture was stirred at room temperature for 1.5 hours, then the solvent was distilled off under reduced pressure. The obtained residue was purified by silica gel column chromatography (n-hexane/ethyl acetate) to obtain the title compound (890 mg).
¹H-NMR (CDCl₃) δ: 1.46 (9H, s) , 4.27 (2H, d, J = 9.8 Hz), 4.34 (2H, d, J = 10.0 Hz), 6.46-6.52 (2H, m), 6.81-6.89 (2H, m), 7.28-7.33 (1H, m), 7.34-7.40 (2H, m), 7.42-7.46 (2H, m).
MS (m/z) : 244 (M-99)⁺.

### [Step 24]

### 3-(4-Fluorophenoxy)-3-phenylazetidine hydrochloride

To the compound obtained by the method of Step 23 (463 mg), isopropyl alcohol (4 ml) and a solution (2.8 ml) of 4 N hydrochloric acid/ethyl acetate were added, and the mixture was stirred at room temperature overnight. The reaction solution was concentrated under reduced pressure, and ethyl acetate (5 ml) was added thereto, then the mixture was stirred at room temperature. The solid produced was collected by filtration to obtain the title compound (220 mg).
¹H-NMR (DMSO-d₆) δ: 4.38 (2H, d, J = 12.8 Hz), 4.56 (2H, d, J = 12.5 Hz), 6.62-6.68 (2H, m), 7.00-7.08 (2H, m), 7.34-7.40 (1H, m), 7.40-7.46 (2H, m), 7.53-7.58 (2H, m), 9.81 (2H, br s).
MS (m/z) : 244 (M+H)⁺.

### [Step 25]

### (S)-Methyl 3-(3-bromophenyl)-2-((tert-butoxycarbonyl)amino)propanoate

N-tert-butoxycarbonyl-L-3-bromophenylalanine (59 g, Amatek, Inc.), 1-(3-dimethylaminopropyl)-3-ethylcarbodiimide hydrochloride (49.3 g), N,N-dimethylaminopyridine (2.09 g), methanol (69.4 ml) and dichloromethane (590 ml) were mixed, and the mixture was stirred at room temperature for 1.5 hours. To the reaction solution, 0.5 N hydrochloric acid (1000 ml) was added, the mixture was separated in solutions, and the aqueous layer was then re-extracted with dichloromethane (200 ml) . The dichloromethane layer was mixed and washed with saturated saline, and then dried over sodium sulfate. The sodium sulfate was filtered off, and the filtrate was concentrated under reduced pressure to obtain the title compound (55 g).
¹H-NMR (CDCl₃) δ: 1.43 (9H, s), 3.00 (1H, dd, J = 13.9, 5.9 Hz), 3.11 (1H, dd, J = 13.3, 5.5 Hz), 3.73 (3H, s), 4.52-4.62 (1H, m), 4.93-5.04 (1H, m), 7.06 (1H, br d, J = 7.5 Hz), 7.16 (1H, t, J = 7.8 Hz), 7.28 (1H, br s), 7.38 (1H, br d, J = 8.0 Hz).
MS (m/z) : 380 (M+Na)⁺.

### [Step 26]

### (S)-Methyl 2-((tert-butoxycarbonyl)amino)-3-(3-((4-(tert-butyl)benzyl)thio)phenyl)propanoate

To a solution of the crude (55 g) obtained by the method of Step 25 in 1,4-dioxane (500 ml), 4-tert-butylbenzyl mercaptan (40.1 ml), 9,9-dimethyl-4,5-bis(diphenylphosphino)xanthene (8.88 g), tris(dibenzylideneacetone)dipalladium(0) (7.03 g) and N,N-diisopropylethylamine (53.6 ml) were added, and then the mixture was stirred at 110°C for 3 hours under an argon atmosphere. The reaction solution was left to cool to room temperature, and then diluted with n-hexane/ethyl acetate. The diluted solution was washed with water and saturated saline sequentially, and then dried over sodium sulfate. The sodium sulfate was filtered off, and the filtrate was concentrated under reduced pressure. The residue was purified by silica gel column chromatography (n-hexane/ethyl acetate) to obtain the title compound (68.9 g) .
¹H-NMR (CDCl₃) δ: 1.30 (9H, s), 1.43 (9H, s), 2.98 (2H, dd, J = 13.4, 6.1 Hz), 3.07 (2H, dd, J = 13.8, 6.0 Hz), 3.69 (3H, s), 4.09 (2H, s), 4.50-4.61 (1H, m), 4.90-4.99 (1H, m), 6.92-6.96 (1H, m), 7.07 (1H, br s), 7.17-7.21 (2H, m) , 7.21-7.25 (2H, m) , 7.30-7.34 (2H, m) .
MS (m/z) : 458 (M+H)⁺.

### [Step 27]

### (S)-Methyl 2-((tert-butoxycarbonyl)amino)-3-(3-(chlorosulfonyl)phenyl)propanoate

To a solution of the compound obtained by the method of Step 26 (62.6 g) in acetonitrile (600 ml), water (24.6 ml) was added, and then acetic acid (31.3 ml) and 1,3-dichloro-5,5-dimethylhydantoin (37.6 g) were added under ice-cooling, and the mixture was stirred for 15 minutes. Water was added to the reaction solution, and then the solvent was distilled off under reduced pressure. To the residue, ethyl acetate (200 ml) and n-hexane (500 ml) were added, and the mixture was washed with water and saturated saline sequentially, and then dried over sodium sulfate. After the sodium sulfate was filtered off, the solvent was distilled off under reduced pressure to obtain a crude (66 g) of the title compound.
¹H-NMR (CDCl₃) δ: 1.42 (9H, s), 3.10-3.20 (1H, m), 3.26-3.36 (1H, m), 3.76 (3H, s), 4.57-4.70 (1H, m), 5.02-5.12 (1H, m), 7.51-7.59 (2H, m), 7.79 (1H, br s), 7.91-7.95 (1H, m).
MS (m/z) : 400 (M+Na)⁺.

### [Step 28]

### (S)-Methyl 2-((tert-butoxycarbonyl)amino)-3-(3-((3-(4-fluorophenoxy)-3-phenylazetidin-1-yl)sulfonyl)phenyl)propanoate

To a solution of the compound obtained by the method of Step 24 (30 g) in N-methyl-2-pyrrolidone (75 ml), 1-methylimidazole (29.5 ml) was added. To the mixture, a solution of the crude obtained by the method of Step 27 (51.3 g) in N-methyl-2-pyrrolidone (75 ml) was added dropwise, and the mixture was stirred at room temperature for 1 hour. The reaction solution was diluted with ethyl acetate, and washed with water, an aqueous potassium hydrogen sulfate solution and saturated saline sequentially, and then dried over sodium sulfate. The sodium sulfate was filtered off, and the filtrate was concentrated under reduced pressure. The residue was purified by silica gel column chromatography (n-hexane/ethyl acetate) to obtain the title compound (44.5 g) .
¹H-NMR (CDCl₃) δ: 1.42 (9H, s) , 3.14 (1H, dd, J = 13.4, 5.4 Hz), 3.26 (1H, dd, J = 13.8, 5.8 Hz), 3.72 (3H, s), 4.12-4.22 (4H, m), 4.56-4.67 (1H, m), 4.93-5.06 (1H, m), 6.31-6.35 (2H, m), 6.76-6.83 (2H, m), 7.28-7.36 (5H, m), 7.44-7.48 (1H, m), 7.51 (1H, t, J = 7.7 Hz), 7.62 (1H, br s), 7.73-7.77 (1H, m).
MS (m/z) : 607 (M+Na)⁺.

### [Step 29]

### (S)-2-((tert-Butoxycarbonyl)amino)-3-(3-((3-(4-fluorophenoxy)-3-phenylazetidin-1-yl)sulfonyl)phenyl)propanoic acid

The compound (44.5 g) obtained by the method of Step 28 was dissolved by addition of tetrahydrofuran (150 ml) and methanol (150 ml), then an aqueous 2 N sodium hydroxide solution (76 ml) was added dropwise under ice-cooling, and the mixture was stirred for 30 minutes subsequently under ice-cooling. The reaction solution was neutralized by addition of 2 N hydrochloric acid at 0°C, and then the solvent was distilled off under reduced pressure. To the residue, ethyl acetate (500 ml) was added, and the mixture was washed with water and saturated saline sequentially, and then dried over sodium sulfate. The sodium sulfate was filtered off, and the filtrate was concentrated under reduced pressure. To the residue, ethyl acetate (50 ml) and n-hexane (50 ml) were added, and (R)-(+)-1-phenethylamine (9.67 ml) was added thereto, and the mixture was stirred at room temperature. The precipitated solid was collected by filtration to obtain (R)-(+)-1-phenethylamine salt (50.1 g) of the title compound. A portion (43.5 g) of the obtained solid was suspended in ethyl acetate and stirred. To the suspension, an aqueous potassium hydrogen sulfate solution was added, and after the solid was completely dissolved, the mixture was separated in solutions. The organic layer was washed with saturated saline, then the mixture was dried over sodium sulfate. The sodium sulfate was filtered off and the filtrate was concentrated under reduced pressure to obtain the title compound (38.4 g).
¹H-NMR (CDCl₃) δ: 1.40 (9H, s), 3.11-3.20 (1H, m), 3.24-3.33 (1H, m), 4.13 (1H, d, J = 9.0 Hz), 4.14 (1H, d, J = 9.5 Hz), 4.20 (1H, d, J = 8.8 Hz), 4.21 (1H, d, J = 9.0 Hz), 4.51-4.61 (1H, m), 5.01-5.10 (1H, m), 6.28-6.34 (2H, m), 6.75-6.82 (2H, m), 7.27-7.34 (5H, m), 7.48-7.54 (2H, m) , 7.68-7.76 (2H, m) .
MS (m/z) : 593 (M+Na)⁺.

### [Step 30]

### (S)-2-Amino-3-(3-((3-(4-fluorophenoxy)-3-phenylazetidin-1-yl)sulfonyl)phenyl)propanoic acid

To the compound obtained by the method of Step 29 (130 mg), dichloromethane (1.3 ml) and trifluoroacetic acid (1.3 ml) were added, and the mixture was stirred at room temperature for 1 hour. The solvent was distilled off under reduced pressure, and the residue was dissolved by addition of tetrahydrofuran (2 ml) and water (2 ml), then neutralized with a 1 N aqueous sodium hydroxide solution. The precipitated solid was collected by filtration to obtain the title compound (88 mg).
¹H-NMR (CDCl₃) δ: 2.98 (1H, dd, J = 14.2, 7.4 Hz), 3.22 (1H, dd, J = 14.3, 5.3 Hz), 3.49 (1H, dd, J = 7.3, 5.5 Hz), 4.08 (1H, d, J = 9.5 Hz), 4.09 (1H, d, J = 9.5 Hz), 4.30 (2H, d, J = 9.8 Hz), 6.42-6.48 (2H, m), 6.92-7.00 (2H, m), 7.23-7.36 (5H, m), 7.58 (1H, t, J = 7.8 Hz), 7.67-7.73 (2H, m), 7.77-7.80 (1H, m).
MS (m/z) : 493 (M+Na)⁺.

### [Example 42]

### (S)-2-Amino-3-(3-( (3-(3-fluorophenoxy)-3-phenylazetidin-1-yl)sulfonyl)phenyl)propanoic acid

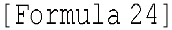

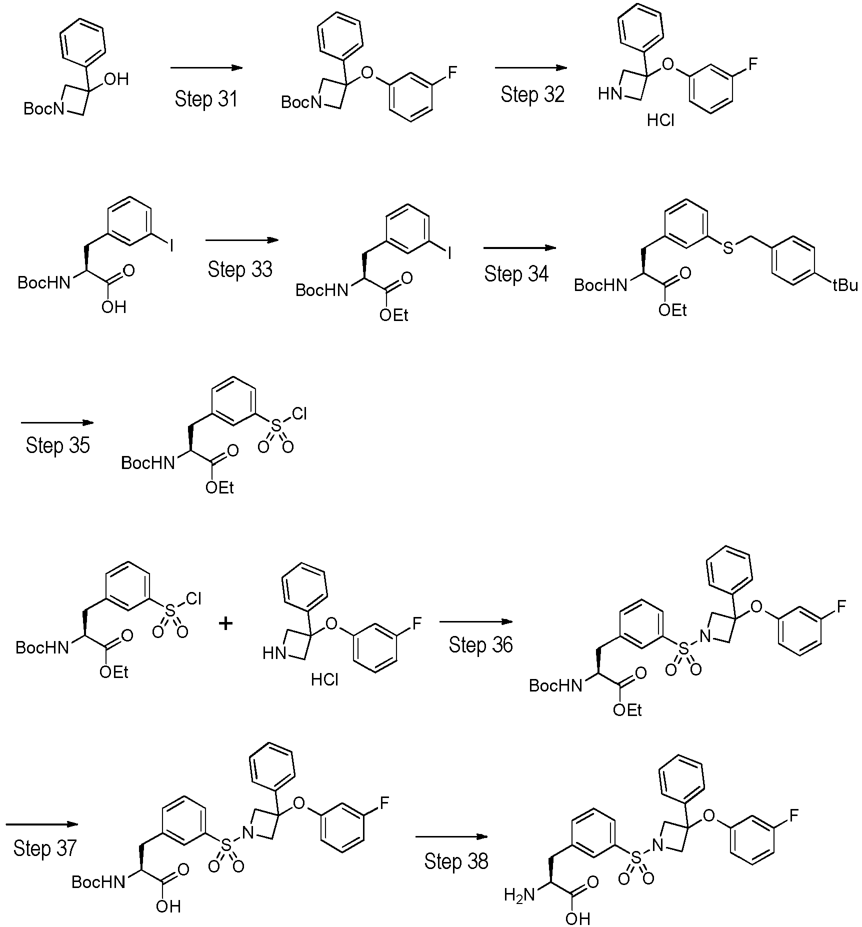

### [Step 31]

### tert-Butyl 3-(3-fluorophenoxy)-3-phenylazetidin-1-carboxylate

To the compound obtained by the method of Step 22 (6.55 g), triphenylphosphine (7.63 g), 3-fluorophenol (5.89 g) and toluene (58 ml) were added, and the mixture was stirred at room temperature to form a homogeneous solution. To the solution under stirring at room temperature, diisopropyl azodicarboxylate (5.84 g) was added, and the mixture was stirred for another 40 minutes, then the solvent was distilled off under reduced pressure. The obtained residue was purified by silica gel column chromatography (n-hexane/ethyl acetate) to obtain the title compound (3.10 g).
¹H-NMR (CDCl₃) δ: 1.47 (9H, s), 4.29 (2H, d, J = 10.0 Hz), 4.36 (2H, d, J = 9.8 Hz), 6.29-6.34 (2H, m), 6.58-6.64 (1H, m), 7.06-7.13 (1H, m), 7.28-7.33 (1H, m), 7.34-7.40 (2H, m), 7.42-7.47 (2H, m).
MS (m/z) : 366 (M+Na)⁺.

### [Step 32]

### 3-(3-Fluorophenoxy)-3-phenylazetidine hydrochloride

To the compound obtained by the method of Step 31 (3.00 g), a solution (20 ml) of 4 N hydrochloric acid/ethyl acetate was added, and the mixture was stirred at room temperature for 1 hour. To the reaction solution, ethyl acetate (20 ml) was added, and the mixture was stirred at room temperature. The solid produced was collected by filtration to obtain the title compound (2.22 g).
¹H-NMR (DMSO-d₆) δ: 4.40 (2H, d, J = 12.5 Hz), 4.61 (2H, d, J = 12.5 Hz), 6.44-6.48 (1H, m), 6.50-6.54 (1H, m), 6.75-6.81 (1H, m), 7.20-7.27 (1H, m), 7.35-7.40 (1H, m), 7.42-7.48 (2H, m), 7.56-7.61 (2H, m), 9.79 (1H, br s). MS (m/z) : 244 (M+H)⁺.

### [Step 33]

### (S)-Ethyl 2-((tert-butoxycarbonyl)amino)-3-(3-iodophenyl)propanoate

To a solution of N-tert-Butoxycarbonyl-L-3-iodophenylalanine (200 g, Cool Pharm Ltd.) in dichloromethane (2 L), ethanol (149 ml), 1-ethyl-3-(3-dimethylaminopropyl)carbodiimide hydrochloride (147 g) and N,N-dimethylaminopyridine (6.25 g) were added, and the mixture was stirred at room temperature for 3 hours. To the reaction solution, 0.5 N hydrochloric acid (1 L) was added, and the mixture was separated in solutions. The aqueous layer was extracted with dichloromethane (200 ml), and the combined organic layer was washed with saturated saline (1 L), and dried over sodium sulfate (250 g) . The sodium sulfate was then filtered off, and the filtrate was concentrated under reduced pressure. To the residue, n-hexane (800 ml) was added, and the precipitated solid was collected by filtration to obtain the title compound (195 g).
¹H-NMR (CDCl₃) δ: 1.25 (3H, t, J = 7.2 Hz), 1.44 (9H, s), 2.99 (1H, dd, J = 13.9, 5.6 Hz),3.07 (1H, dd, J = 13.7, 6.1 Hz), 4.13-4.22 (2H, m), 4.48-4.57 (1H, m), 5.01 (1H, d, J = 6.3 Hz), 7.03 (1H, t, J = 7.8 Hz), 7.12 (1H, br d, J = 7.8 Hz), 7.50 (1H, br s), 7.55-7.60 (1H, m). MS (m/z) : 364 (M-55)⁺.

### [Step 34]

### (S)-Ethyl 2-((tert-butoxycarbonyl)amino)-3-(3-((4-(tert-butyl)benzyl)thio)phenyl)propanoate

To a solution of the compound obtained by the method of Step 33 (125 g) and 4-tert-butylbenzylmercaptan (100 ml) in N-methylpyrrolidone (1.25 L), copper(I) iodide (28.4 g) and potassium carbonate (82 g) were added, and the mixture was stirred at 100°C overnight under an argon atmosphere. The reaction solution was left to cool to room temperature, and then diluted with n-hexane/ethyl acetate, and water was added. The insoluble matters were filtered off through Celite, and the filtrate was separated in solutions. The aqueous layer was extracted with n-hexane/ethyl acetate, and the combined organic layer was washed with water and saturated saline, and then dried over sodium sulfate. The obtained residue was concentrated under reduced pressure and roughly purified by silica gel column chromatography (n-hexane/ethyl acetate) to obtain a crude (144 g) of the title compound.

### [Step 35]

### (S)-Ethyl 2-((tert-butoxycarbonyl)amino)-3-(3-(chlorosulfonyl)phenyl)propanoate

To a solution of the crude (141 g) obtained by the method of Step 34 in acetonitrile (1 L), acetic acid (68.5 ml) and 1,3-dichloro-5,5-dimethylhydantoin (124 g) were added under ice cooling, and the mixture was stirred for 30 minutes. Water was added to the reaction solution and the mixture was concentrated. The mixture was then diluted with n-hexane/ethyl acetate, and washed with water and saturated saline sequentially. The washed product was dried over sodium sulfate, the sodium sulfate was filtered off and the filtrate was concentrated. The obtained residue was purified by silica gel column chromatography (n-hexane/ethyl acetate) to obtain the title compound (63 g).
¹H-NMR (CDCl₃) δ: 1.27 (3H, t, J = 7.2 Hz), 1.43 (9H, s), 3.16 (1H, dd, J = 13.7, 5.9 Hz), 3.30 (1H, dd, J = 13.8, 5.8 Hz), 4.20 (2H, q, J = 7.2 Hz), 4.56-4.65 (1H, m), 5.06-5.14 (1H, m), 7.53-7.57 (2H, m), 7.80 (1H, br s), 7.90-7.96 (1H, m).
MS (m/z) : 336 (M-55)⁺.

### [Step 36]

### (S)-Ethyl 2-((tert-butoxycarbonyl)amino)-3-(3-((3-(3-fluorophenoxy)-3-phenylazetidin-1-yl)sulfonyl)phenyl)propanoate

To a solution of the compound (12.9 g) obtained by the method of Step 35 in dichloromethane (100 ml), N,N-diisopropylethylamine (10.7 ml) and the compound (5.71 g) obtained by the method of Step 32 were added, and the mixture was stirred at room temperature for 1.5 hours. To the reaction solution, a saturated aqueous potassium hydrogen sulfate solution was added, the mixture was separated in solutions, and the aqueous layer was then re-extracted with dichloromethane. The dichloromethane layer was mixed, washed with water and saturated saline sequentially, and then dried over sodium sulfate. The sodium sulfate was filtered off, and the filtrate was concentrated under reduced pressure. The residue was purified by silica gel column chromatography (n-hexane/ethyl acetate) to obtain the title compound (8.66 g) .
¹H-NMR (CDCl₃) δ: 1.26 (3H, t, J = 7.2 Hz), 1.42 (9H, s), 3.14 (1H, dd, J = 13.7, 5.4 Hz), 3.26 (1H, dd, J = 13.6, 5.5 Hz), 4.13-4.25 (6H, m), 4.54-4.62 (1H, m), 5.02 (1H, d, J = 6.5 Hz), 6.12-6.17 (2H, m), 6.56-6.62 (1H, m), 7.01-7.08 (1H, m), 7.26-7.36 (5H, m), 7.46-7.54 (2H, m), 7.64 (1H, br s), 7.73-7.77 (1H, m).
MS (m/z) : 621 (M+Na)⁺.

### [Step 37]

### (S)-2-((tert-Butoxycarbonyl)amino)-3-(3-((3-(3-fluorophenoxy)-3-phenylazetidin-1-yl)sulfonyl)phenyl)propanoic acid

To the compound obtained by the method of Step 36 (8.66 g), tetrahydrofuran (60 ml) and methanol (30 ml) were added. Then, an aqueous 2 N sodium hydroxide solution (14.5 ml) was added dropwise under cooling from -40 to -23°C, and the mixture was gradually warmed and stirred under the temperature of -3°C or lower for 4.5 hours. After 2 N hydrochloric acid (13.5 ml) was added dropwise, the organic solvent was distilled off under reduced pressure. To the residue, ethyl acetate was added, and the mixture was washed with water and saturated saline sequentially, and then dried over sodium sulfate. The sodium sulfate was filtered off and the filtrate was concentrated under reduced pressure to obtain a crude (8.55 g) of the title compound. To this crude, tetrahydrofuran (140 ml), n-hexane (70 ml) and (R)-(+)-1-phenethylamine (1.72 ml) were added, and the mixture was heated and stirred at 70°C. The mixture was stirred at room temperature for 4 hours, then the precipitated solid was collected by filtration to obtain (R)-(+)-1-phenethylamine salt (5.93 g) of the title compound. To a portion (5.90 g) of the obtained solid, ethyl acetate (70 ml) was added, and the suspension was stirred, then dissolved by addition of 0.1 N hydrochloric acid. After separation in solutions, the obtained organic layer was dried over sodium sulfate. The sodium sulfate was filtered off and the filtrate was concentrated under reduced pressure to obtain the title compound (5.11 g).
¹H-NMR (CDCl₃) δ: 1.41 (9H, s), 3.17 (1H, dd, J = 13.7, 5.4 Hz), 3.31 (1H, dd, J = 13.6, 5.3 Hz), 4.15 (1H, d, J = 9.0 Hz), 4.16 (1H, d, J = 9.3 Hz), 4.23 (1H, d, J = 9.0 Hz), 4.23 (1H, d, J = 9.3 Hz), 4.56-4.66 (1H, m), 5.00 (1H, d, J = 7.0 Hz), 6.10-6.15 (2H, m), 6.56-6.62 (1H, m), 7.00-7.07 (1H, m), 7.27-7.35 (5H, m), 7.51-7.55 (2H, m), 7.70-7.77 (2H, m).
MS (m/z) : 593 (M+Na)⁺.

### [Step 38]

### (S)-2-Amino-3-(3-( (3-(3-fluorophenoxy)-3-phenylazetidin-1-yl)sulfonyl)phenyl)propanoic acid

To the compound (5.11 g) obtained by the method of Step 37, 1,4-dioxane (10 ml) and a solution (17 ml) of 4 N hydrochloric acid/1,4-dioxane were added, and the mixture was stirred at room temperature for 2 hours. The solvent was distilled off under reduced pressure, and then ethyl acetate (25 ml) was added, and the mixture was neutralized with a saturated aqueous sodium hydrogen carbonate solution. The neutralized solution was stirred at room temperature, then the solid produced was collected by filtration to obtain the title compound (3.97 g).
¹H-NMR (DMSO-d₆) δ: 2.86 (1H, dd, J = 13.6, 7.3 Hz), 3.16 (1H, dd, J = 13.7, 4.9 Hz), 4.09 (1H, d, J = 9.5 Hz), 4.11 (1H, d, J = 9.8 Hz), 4.33 (1H, d, J = 9.5 Hz), 4.34 (1H, d, J = 9.8 Hz), 6.24-6.34 (2H, m), 6.67-6.73 (1H, m), 7.11-7.19 (1H, m), 7.20-7.35 (5H, m), 7.55 (1H, t, J = 7.7 Hz), 7.64-7.70 (2H, m), 7.77 (1H, br s), signal of one proton was overlapped with H₂O signal. MS (m/z) : 471 (M+H)⁺.

The same procedures as in Example 42 were performed to obtained the compound below. However, for Step 32, the same procedures as in Step 3 were performed.

**[Table 22]**

| Example | Name and Structure | Equipment data |
|---|---|---|
| 43 | (S)-2-Amino-3-(3-((3-(4-fluorophenoxy)-3-(pyridin-2-yl)azetidin-1-yl)sulfonyl)phenyl)propanoic acid | ¹H-NMR (DMSO-d₆) δ: 2.92 (1H, dd, J = 14.2, 7.7 Hz), 3.20 (1H, dd, J = 14.2, 4.9 Hz), 3.40 (1H, dd, J = 7.7, 4.9 Hz), 4.07 (1H, d, J = 9.3 Hz), 4.08 (1H, d, J = 9.3 Hz), 4.48 (1H, d, J = 9.0 Hz), 4.49 (1H, d, J = 9.0 Hz), 6.38-6.45 (2H, m), 6.92-7.00 (2H, m), 7.25-7.33 (2H, m), 7.53 (1H, t, *J=* 7.7 Hz), 7.60-7.67 (2H, m), 7.70-7.76 (2H, m), 8.50-8.54 (1H, m). |
| | 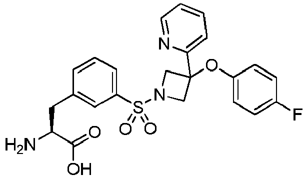 | MS (m/z): 472 (M+H)⁺. |

### [Example 44]

### (2S)-3-(3-((3-(4-Fluorophenoxy)-3-phenylazetidin-1-yl)sulfonyl)phenyl)-2-(((1-(pivaloyloxy)ethoxy)carbonyl)amino)propanoic acid

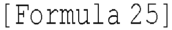

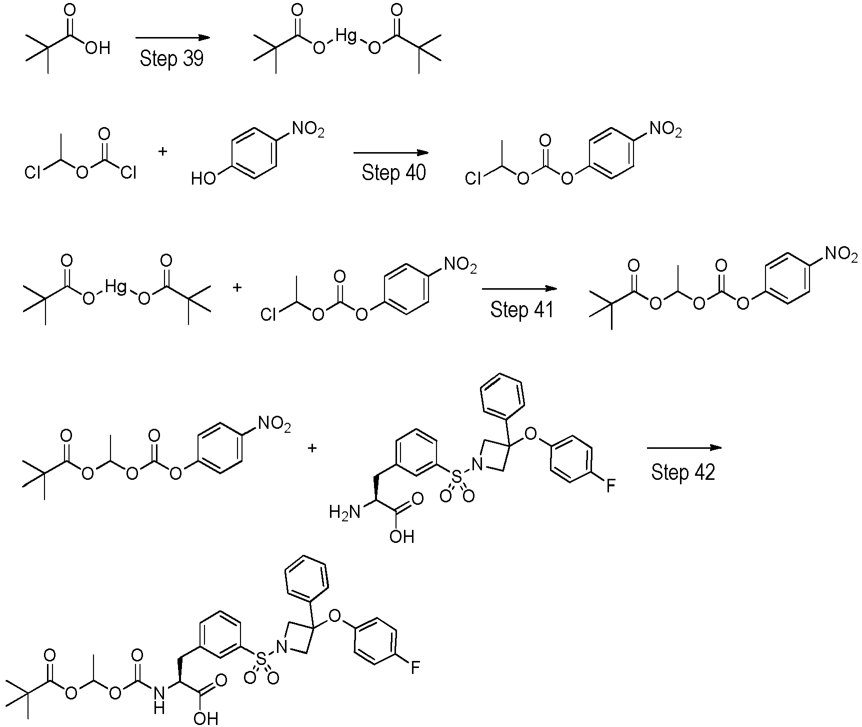

### [Step 39]

### Mercury(II) pivalate

A suspension of mercury(II) oxide (yellow) (32 g) and pivalic acid (30.2 g) in carbon tetrachloride (500 ml) was heated to reflux overnight, and then left to cool to room temperature. The reaction solution was filtered, washed with chloroform, and then the filtrate was concentrated. To the obtained residue, n-hexane was added to form a suspension, and the precipitate was collected by filtration to obtain the title compound (43.0 g).

### [Step 40]

### 1-Chloroethyl(4-nitrophenyl)carbonate

To a solution of 4-nitrophenol (80 g) in pyridine (55.8 ml) and dichloromethane (160 ml), 1-chloroethyl chloroformate (75 ml) was added dropwise at 0°C, and the mixture was stirred at room temperature for 2 hours. The reaction solution was diluted with a mixed solution of n-hexane/ethyl acetate, and washed with an aqueous potassium hydrogen sulfate solution. The aqueous layer was extracted with a mixture of n-hexane/ethyl acetate, and the combined organic layer was washed with saturated saline, dried over sodium sulfate, and then concentrated under reduced pressure. To the obtained residue, n-hexane/ethyl acetate was added, and the precipitate was collected by filtration to obtain the title compound (124 g) .
¹H-NMR (CDCl₃) δ: 1.93 (3H, d, J = 5.8 Hz), 6.51 (1H, q, J = 5.9 Hz), 7.40-7.45 (2H, m), 8.28-8.33 (2H, m) . MS (m/z) : 246 (M+H)⁺.

### [Step 41]

### 1-(((4-Nitrophenoxy)carbonyl)oxy)ethyl pivalate

To pivalic acid (83 ml) under heating at 80°C, the compound obtained by the method of Step 39 (43 g) and the compound obtained by the method of Step 40 (35.0 g) were added little by little, and the mixture was stirred overnight. The precipitate in the reaction solution was filtered off, and the filtrate was diluted with a mixed solution of n-hexane/ethyl acetate, and washed by slowly adding an aqueous 10% sodium carbonate solution at 0°C. The organic layer was further washed with a saturated aqueous sodium hydrogen carbonate solution and saturated saline sequentially, and concentrated. To the residue, n-hexane was added, and the precipitate was collected by filtration to obtain the title compound (29.2 g). ¹H-NMR (CDCl₃) δ: 1.24 (9H, s), 1.62 (3H, d, J = 5.5 Hz), 6.82 (1H, q, J = 5.4 Hz), 7.37-7.42 (2H, m), 8.26-8.31 (2H, m).

### [Step 42]

### (2S)-3-(3-((3-(4-Fluorophenoxy)-3-phenylazetidin-1-yl)sulfonyl)phenyl)-2-(((1-(pivaloyloxy)ethoxy)carbonyl)amino)propanoic acid

To a mixed solution of the compound (52.3 g) of Example 41 and the compound (36.4 g) obtained by the method of Step 41 in tetrahydrofuran (524 ml) and water (262 ml), triethylamine (62 ml) was added at room temperature, and the mixture was stirred at room temperature for 30 minutes. The reaction solution was concentrated under reduced pressure, and ethyl acetate, water, and a saturated aqueous potassium hydrogen sulfate solution were added to the residue to separate in solutions. The obtained organic layer was washed with water and saturated saline sequentially, and dried over magnesium sulfate. The magnesium sulfate was filtered off and the filtrate was concentrated, and the obtained residue was purified by silica gel column chromatography (n-hexane/ethyl acetate, followed by replacement with chloroform/methanol) to obtain a yellow amorphous (62.8 g). To the obtained amorphous, ethyl acetate, a saturated aqueous potassium hydrogen sulfate solution and water were added, and the mixture was stirred at room temperature to separate in solutions. The organic layer was washed by addition of saturated saline and water, and then dried over magnesium sulfate. The magnesium sulfate was filtered off and the solvent was distilled off from the filtrate under reduced pressure to obtain the title compound (60.0 g).
¹H-NMR (CDCl₃) δ: 1.17 (4.5H, s), 1.22 (4.5H, s), 1.43 (1.5H, d, J = 5.4 Hz), 1.45 (1.5H, d, J = 5.4 Hz), 3.22 (1H, td, J = 14.2, 5.5 Hz), 3.29-3.38 (1H, m), 4.09-4.17 (2H, m), 4.20 (1H, d, J = 9.0 Hz), 4.21 (1H, d, J = 9.3 Hz), 4.63-4.71 (1H, m), 5.26 (1H, t, J = 7.4 Hz), 6.28-6.35 (2H, m), 6.74-6.83 (3H, m), 7.27-7.35 (5H, m), 7.49-7.57 (2H, m), 7.65-7.70 (1H, m), 7.73-7.78 (1H, m). MS (m/z) : 643 (M+H)⁺.

The same procedures as in Example 44 were performed to obtain the compounds below. For the raw materials of Step 42, the corresponding Example compounds at the right of the following table were used.

**[Table 23]**

| Example | Name and Structure | Equipment data | Raw material compound of Step 42 |
|---|---|---|---|
| 45 | (2S)-3-(3-((3-(3-Fluorophenoxy)-3-(4-fluorophenyl)azetidin-1-yl)sulfonyl)phenyl)-2-(((1-(isobutyloxy)ethoxy)carbonyl)a mino)propanoic acid | ¹H-NMR (CDCl₃) δ: 1.15 (3H, dd, J = 7.0, 1.8 Hz), 1.18 (3H, d, J = 7.0 Hz), 1.45 (3H, dd, J = 5.3, 3.8 Hz), 2.47-2.60 (1H, m), 3.17-3.27 (1H, m), 3.35 (1H, ddd, J = 14.3, 9.3, 5.7 Hz), 4.10-4.17 (2H, m), 4.17-4.22 (2H, m), 4.64-4.72 (1H, m), 5.24 (1H, dd, J = 12.7, 7.4 Hz), 6.09-6.15 (2H, m), 6.58-6.64 (1H, m), 6.76-6.83 (1H, m), 6.98-7.10 (3H, m), 7.26-7.34 (2H, m), 7.48-7.58 (2H, m), 7.68 (1H, s), 7.76 (1H, dt, J = 6.8, 2.2 Hz). | Example 1 |
| | 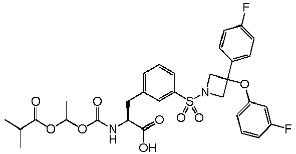 | | |
| | | MS (m/z) : 669 (M+Na)⁺. | |
| 46 | (2S)-2-(((1-((Cyclohexanecarbonyl)oxy)eth oxy)carbonyl)amino)-3-(3-((3-(3 -fluorophenoxy)-3 -(4-(fluorophenyl)azetidin-1-yl)sulfonyl)phenyl)propanoic acid | ¹H-NMR (CDCl₃) δ: 1.16-1.34 (3H, m), 1.36-1.52 (5H, m), 1.57-1.68 (1H, m), 1.69-1.79 (1H, m), 2.22-2.38 (1H, m), 3.18-3.40 (2H, m), 4.09-4.23 (4H, m), 4.63-4.73 (1H, m), 5.20-5.31 (1H, m), 6.10-6.16 (2H, m), 6.57-6.65 (1H, m), 6.77-6.84 (1H, m), 6.98-7.10 (3H, m), 7.27-7.33 (2H, m), 7.48-7.58 (2H, m), 7.67 (1H, s), 7.72-7.80 (1H, m), signals of 3 methylene protons were overlapped with H2O signal. | Example 1 |
| | 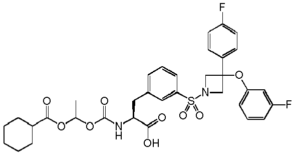 | | |
| | | MS (m/z) : 709 (M+Na)⁺. | |

**[Table 24]**

| | | | |
|---|---|---|---|
| 47 | (2S)-3-(3-((3-3-Fluorophenoxy)-3-(4-fluorophenyl)azetidin-1-yl)sulfonyl)phenyl)-2-(((1-((tetrahydro-2H-pyran-4-carbonyl)oxy)ethoxy)carbonyl)a mino)propanoic acid | ¹H-NMR (CDCl₃) δ: 1.45 (3H, d, J = 5.5 Hz), 2.44-2.66 (1H, m), 3.16-3.51 (4H, m), 3.90-4.01 (2H, m), 4.09-4.22 (4H, m), 4.61-4.72 (1H, m), 5.20-5.33 (1H, m), 6.08-6.16 (2H, m), 6.58-6.64 (1H, m), 6.78-6.85 (1H, m), 6.98-7.10 (3H, m), 7.27-7.33 (2H, m), 7.49-7.57 (2H, m), 7.64-7.70 (1H, m), 7.71-7.78 (1H, m), signals of 4 protons were overlapped with H2O signal. | Example 1 |
| | 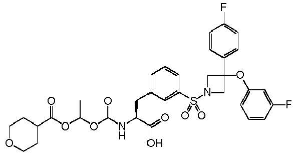 | | |
| | | MS (m/z) : 689 (M+H)⁺. | |
| 48 | (2S)-3-(3-((3-(3-Fluorophenoxy)-3-(4-fluorophenyl)azetidin-1-yl)sulfonyl)phenyl)-2-(((1-((3-methylbutanoyl)oxy)ethoxy)carbo nyl)amino)propanoic acid | ¹H-NMR (CDCl₃) δ: 0.91-1.00 (6H, m), 1.37-1.53 (3H, m), 2.00-2.26 (3H, m), 3.15-3.40 (2H, m), 4.09-4.23 (4H, m), 4.59-4.70 (1H, m), 5.25-5.34 (1H, m), 6.08-6.17 (2H, m), 6.57-6.64 (1H, m), 6.78-6.85 (1H, m), 6.97-7.10 (3H, m), 7.24-7.33 (2H, m), 7.46-7.56 (2H, m), 7.66-7.70 (1H, m), 7.72-7.78 (1H, m). | Example 1 |
| | 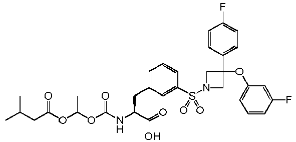 | | |
| | | MS (m/z): 683 (M+Na)⁺. | |
| 49 | (2S)-3-(3-((3 -(3-Fluorophenoxy)-3 -(4-fluorophenyl)azetidin- 1-yl)sulfonyl)phenyl)-2-(((1 - (pivaloyloxy)ethoxy)carbonyl)am ino)propanoic acid | ¹H-NMR (CDCl₃) δ: 1.16 (4.5H, s), 1.20 (4.5H, s), 1.40-1.45 (3H, m), 3.11-3.26 (1H, m), 3.29-3.40 (1H, m), 4.10-4.23 (4H, m), 4.53-4.66 (1H, m), 5.30-5.40 (1H, m), 6.08-6.15 (2H, m), 6.57-6.64 (1H, m), 6.72-6.81 (1H, m), 6.96-7.09 (3H, m), 7.22-7.32 (2H, m), 7.47-7.57 (2H, m), 7.66-7.76 (2H, m). | Example 1 |
| | 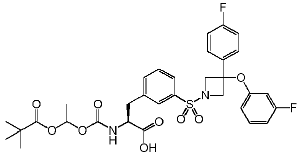 | | |
| | | MS (m/z): 683 (M+Na)⁺. | |

**[Table 25]**

| | | | |
|---|---|---|---|
| 50 | (2S)-2-(((1-(Isobutyryloxy)ethoxy)carbonyl )amino)-3-(3-((3-phenyl-3-(2-(trifluoromethyl)phenoxy)azetid in-1-yl)sulfonyl)phenyl)propanoic acid | ¹H-NMR (CDCl₃) δ: 1.14 (3H, dd, J = 7.0, 2.3 Hz), 1.18 (3H, d, J = 6.8 Hz), 1.44 (3H, t, J = 5.9 Hz), 2.46-2.61 (1H, m), 3.22 (1H, td, J = 14.1, 5.4 Hz), 3.30-3.40 (1H, m), 4.08-4.21 (4H, m), 4.62-4.71 (1H, m), 5.31 (1H, dd, J = 14.7, 7.2 Hz), 6.26-6.35 (2H, m), 6.75-6.85 (3H, m), 7.01 (2H, t, J = 8.7 Hz), 7.24-7.33 (2H, m), 7.48-7.58 (2H, m), 7.65-7.71 (1H, m), 7.72-7.78 (1H, m). | Example 2 |
| | 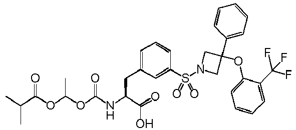 | MS (m/z): 701 (M+Na)⁺. | |
| 51 | (2S)-3-(3-((3-(3-Chlorophenoxy)-3-phenylazetidin-1-yl)sulfonyl)phenyl)-2-(((1-(isobutyryloxy)ethoxy)carbonyl )amino)propanoic acid | 1H-NMR (CDCl₃) δ: 1.13 (3H, d, J = 7.0 Hz), 1.17 (3H, d, J = 7.0 Hz), 1.37-1.47 (3H, m), 2.45-2.60 (1H, m), 3.13-3.26 (1H, m), 3.27-3.40 (1H, m), 4.10-4.18 (2H, m), 4.18-4.26 (2H, m), 4.52-4.67 (1H, m), 5.27-5.54 (1H, m), 6.16-6.22 (1H, m), 6.41-6.45 (1H, m), 6.74-6.83 (1H, m), 6.83-6.89 (1H, m), 7.00 (1H, t, J = 8.2 Hz), 7.24-7.36 (5H, m), 7.47-7.58 (2H, m), 7.69 (1H, s), 7.70-7.76 (1H, m). | Example 3 |
| | 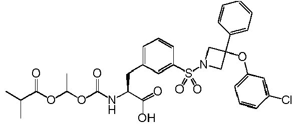 | MS (m/z) : 667 (M+Na)⁺. | |
| 52 | (2S)-3-(3-((3-(2-Fluorophenoxy)-3-(4-fluorophenyl)azesidin-1-yl)sulfonyl)phenyl)-2-(((1-(isobutyryloxy)ethoxy)carbonyl )amino)propanoic acid | ¹H-NMR (CDCl₃) δ: 1.15 (3H, d, J = 7.0 Hz), 1.19 (3H, d, J = 7.0 Hz), 1.43-1.49 (3H, m), 2.48-2.61 (1H, m), 3.19-3.37 (2H, m), 4.15-4.20 (2H, m), 4.24 (2H, d, J = 9.5 Hz), 4.63-4.72 (1H, m), 5.28 (1H, dd, J = 12.5, 7.5 Hz), 6.14 (1H, td, J = 8.2, 1.5 Hz), 6.75-6.89 (3H, m), 6.98-7.06 (3H, m), 7.31-7.39 (2H, m), 7.48-7.55 (2H, m), 7.67 (1H, s), 7.75 (1H, td, J = 4.5, 2.1 Hz). | Example 4 |
| | 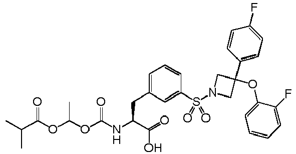 | | |
| | | MS (m/z) : 669 (M+Na)⁺. | |

**[Table 26]**

| | | | |
|---|---|---|---|
| 53 | (2S)-3-(3-((3-(2-Bromophenoxy)-3-phenylazetidin-1-yl)sulfonyl)phenyl)-2-(((1-isobutyryloxy)ethoxy)carbonyl) amino)propanoic acid | ¹H-NMR (CDCl₃) δ: 1.12-1.24 (6H, m), 1.47 (3H, d, J = 5.5 Hz), 2.48-2.64 (1H, m), 3.18-3.40 (2H, m), 4.13-4.22 (2H, m), 4.26 (2H, d, J = 9.0 Hz), 4.60-4.71 (1H, m), 5.20-5.29 (1H, m), 5.98-6.03 (1H, m), 6.73-6.86 (2H, m), 6.89-6.99 (1H, m), 7.24-7.41 (5H, m), 7.45-7.58 (3H, m), 7.67 (1H, br s), 7.73-7.80 (1H, m). | Example 5 |
| | 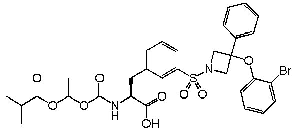 | | |
| | | MS (m/z) : 689 (M+H)⁺. | |
| 54 | (2S)-3-(3-((3-(4-Fluorophenoxy)-3-(4-fluorophenyl)azetidin-1-yl)sulfonyl)phenyl)-2-(((1-(isobutyryloxy)ethoxy)carbonyl amino)propanoic acid | ¹H-NMR (CDCl₃) δ: 1.14 (3H, dd, J = 7.0, 2.3 Hz), 1.18 (3H, d, J = 6.8 Hz), 1.44 (3H, t, J = 5.9 Hz), 2.46-2.61 (1H, m), 3.22 (1H, td, J = 14.1, 5.4 Hz), 3.30-3.40 (1H, m), 4.08-4.21 (4H, m), 4.62-4.71 (1H, m), 5.31 (1H, dd, J = 14.7, 7.2 Hz), 6.26-6.35 (2H, m), 6.75-6.85 (3H, m), 7.01 (2H, t, J = 8.7 Hz), 7.24-7.33 (2H, m), 7.48-7.58 (2H, m), 7.65-7.71 (1H, m), 7.72-7.78 (1H, m). | Example 6 |
| | 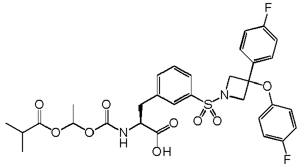 | MS (m/z) : 669 (M+Na)⁺. | |
| 55 | (2S)-3-(3-((3-(2-Chlorophenoxy)-3 -phenylazetidin-1-yl)sulfonyl)phenyl)-2-(((1-(isobutyryloxy)ethoxy)carbonyl amino)propanoic acid | ¹H-NMR (CDCl₃) δ: 1.13 (3H, d, J = 6.8 Hz), 1.17 (3H, d, J = 6.8 Hz), 1.40-1.46 (3H, m), 2.45-2.62 (1H, m), 3.10-3.27 (1H, m), 3.27-3.38 (1H, m), 4.15-4.22 (2H, m), 4.25 (2H, d, J = 9.0 Hz), 4.50-4.63 (1H, m), 5.36 (1H, br s), 6.03 (1H, dd, J = 8.3, 1.5 Hz), 6.74-6.94 (3H, m), 7.25-7.36 (6H, m), 7.46-7.56 (2H, m), 7.68 (1H, d, J = 4.0 Hz), 7.70-7.76 (1H, m). | Example 7 |
| | 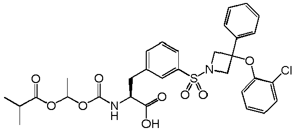 | | |
| | | MS (m/z): 645 (M+H)⁺. | |

**[Table 27]**

| | | | |
|---|---|---|---|
| 56 | (2S)-3-(3-((3-(3-Bromophenoxy)-3-phenylazetidin-1-yl)sulfonyl)phenyl)-2-(((1-isobutyryloxy)ethoxy)carbonyl) amino)propanoic acid | ¹H-NMR (CDCl₃) δ: 1.13 (3H, d, J = 7.0 Hz), 1.18 (3H, d, J = 7.0 Hz), 1.39-1.42 (3H, m), 2.45-2.62 (1H, m), 3.12-3.28 (1H, m), 3.29-3.40 (1H, m), 4.10-4.18 (2H, m), 4.18-4.28 (2H, m), 4.52-4.63 (1H, m), 5.36-5.47 (1H, m), 6.18-6.25 (1H, m), 6.58-6.62 (1H, m), 6.74-6.85 (1H, m), 6.90-6.97 (1H, m), 6.98-7.04 (1H, m), 7.22-7.36 (5H, m), 7.45-7.59 (2H, m), 7.64-7.70 (1H, m), 7.70-7.76 (1H, m). | Example 8 |
| | 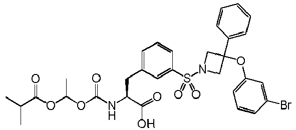 | | |
| | | MS (m/z): 689, 691 (M+H)⁺. | |
| 57 | (2S)-2-(((1-(Isobutyryloxy)ethoxy)carbonyl )amino)-3-(3-((3-phenyl-3-(p-tolyloxy)azetidin-1-yl)sulfonyl)phenyl)propanoic acid | ¹H-NMR (CDCl₃) δ: 1.13-1.22 (6H, m), 1.42-1.49 (3H, m), 2.17-2.22 (3H, m), 2.46-2.63 (1H, m), 3.15-3.39 (2H, m), 4.08-4.15 (2H, m), 4.18-4.25 (2H, m), 4.59-4.69 (1H, m), 5.22-5.32 (1H, m), 6.22-6.30 (2H, m), 6.79-6.86 (1H, m), 6.87-6.92 (2H, m), 7.27-7.37 (5H, m), 7.47-7.58 (2H, m), 7.63-7.68 (1H, m), 7.72-7.79 (1H, m). | Example 9 |
| | 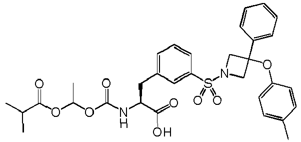 | | |
| | | MS (m/z): 625 (M+H)⁺. | |
| 58 | (2S)-3-(3-((3-(3,4-Difluorophenoxy)-3-phenylazetidin-1-yl)sulfonyl)phenyl)-2-(((1-isobutyryloxy)ethoxy)carbonyl) amino)propanoic acid | ¹H-NMR (CDCl₃) δ: 1.10-1.24 (6H, m), 1.46 (3H, d, J = 5.3 Hz), 2.47-2.65 (1H, m), 3.18-3.30 (1H, m), 3.30-3.43 (1H, m), 4.10-4.17 (2H, m), 4.20 (1H, d, J = 8.8 Hz), 4.22 (1H, d, J = 8.8 Hz), 4.63-4.76 (1H, m), 5.19-5.30 (1H, m), 6.01-6.11 (1H, m), 6.20-6.30 (1H, m), 6.77-6.85 (1H, m), 6.84-6.93 (1H, m), 7.23-7.40 (5H, m), 7.48-7.60 (2H, m), 7.68 (1H, br s), 7.76 (1H, d, J = 7.0 Hz). | Example 10 |
| | 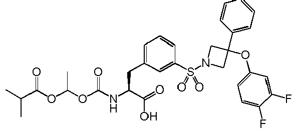 | MS (m/z): 647 (M+H)⁺. | |

**[Table 28]**

| | | | |
|---|---|---|---|
| 59 | (2S)-3-(3-((3-(3-Chloro-4-fluorophenoxy)-3-phenylazetidin-1-yl)sulfonyl)phenyl)-2-(((1-isobutyryloxy)ethoxy)carbonyl) amino)propanoic acid | ¹H-NMR (CDCl₃) δ: 1.16 (3H, d, J = 16.1 Hz), 1.18 (3H, d, J = 16.1 Hz), 1.41-1.49 (3H, m), 2.46-2.64 (1H, m), 3.18-3.30 (1H, m), 3.30-3.44 (1H, m), 4.11-4.17 (2H, m), 4.21 (1H, d, J = 8.8 Hz), 4.22 (1H, d, J = 9.0 Hz), 4.63-4.73 (1H, m), 5.23-5.34 (1H, m), 6.14-6.22 (1H, m), 6.46 (1H, dd, J = 6.0, 3.0 Hz), 6.75-6.91 (2H, m), 7.23-7.39 (5H, m), 7.48-7.59 (2H, m), 7.68 (1H, s), 7.72-7.78 (1H, m). | Example 11 |
| | 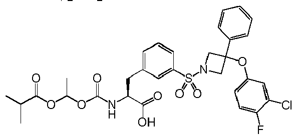 | | |
| | | MS (m/z): 685 (M+Na)⁺. | |
| 60 | (2S)-2-(((1-(Isobutyryloxy)ethoxy)carbonyl )amino)-3-(3-((3-phenyl-3-(4-(trifluoromethyl)phenoxy)azetid in-1-yl)sulfonyl)phenyl)propanoic acid | ¹H-NMR (CDCl₃) δ: 1.11-1.22 (6H, m), 1.42-1.47 (3H, m), 2.46-2.61 (1H, m), 3.17-3.29 (1H, m), 3.29-3.41 (1H, m), 4.14-4.21 (2H, m), 4.22-4.29 (2H, m), 4.61-4.71 (1H, m), 5.19-5.31 (1H, m), 6.44-6.51 (2H, m), 6.76-6.85 (1H, m), 7.27-7.41 (7H, m), 7.49-7.56 (2H, m), 7.66-7.70 (1H, m), 7.73-7.79 (1H, m). | Example 12 |
| | 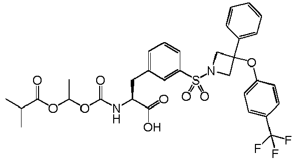 | | |
| | | MS (m/z) : 680 (M+H)⁺. | |
| 61 | (2S)-3-(3-((3-(3-Fluorophenoxy)-3-(2-fluorophenyl)azetidin-1-y1)sulfonyl)phenyl)-2-(((1-isobutyryloxy)ethoxy)carbonyl) amino)propanoic acid | ¹H-NMR (CDCl₃) δ: 1.12-1.22 (6H, m), 1.42-1.48 (3H, m), 2.46-2.62 (1H, m), 3.18-3.38 (2H, m), 4.24 (1H, d, J = 9.0 Hz), 4.25 (1H, d, J = 9.3 Hz), 4.45 (1H, d, J = 8.3 Hz), 4.47 (1H, d, J = 9.3 Hz), 4.62-4.72 (1H, m), 5.21-5.31 (1H, m), 6.16-6.28 (2H, m), 6.60-6.67 (1H, m), 6.76-6.86 (1H, m), 7.03-7.09 (3H, m), 7.19-7.33 (2H, m), 7.44-7.56 (2H, m), 7.65-7.67 (1H, br m), 7.72-7.80 (1H, m). | Example 13 |
| | 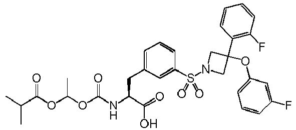 | | |
| | | MS (m/z) : 669 (M+Na)⁺. | |

**[Table 29]**

| | | | |
|---|---|---|---|
| 62 | (2S)-3-(3-((3-(4-Fluorophenoxy)-3-(2-fluorophenyl)azetidin-1-yl)sulfonyl)phenyl)-2-(((1-isobutyryloxy)ethoxy)carbonyl)a mino)propanoic acid | ¹H-NMR (CDCl₃) δ: 1.11-1.24 (6H, m), 1.45 (3H, d, J = 5.3 Hz), 2.45-2.66 (1H, m), 3.17-3.38 (2H, m), 4.25 (1H, d, J = 9.3 Hz), 4.26 (1H, d, J = 9.3 Hz), 4.40 (1H, d, J = 8.8 Hz), 4.42 (1H, d, J = 8.5 Hz), 4.62-4.73 (1H, m), 5.22-5.38 (1H, m), 6.42 (2H, dd, J = 9.2, 4.4 Hz), 6.74-6.88 (3H, m), 6.98-7.11 (2H, m), 7.11-7.20 (1H, m), 7.23-7.37 (1H, m), 7.43-7.57 (2H, m), 7.68 (1H, br s), 7.75 (1H, d, J = 7.5 Hz). | Example 14 |
| | 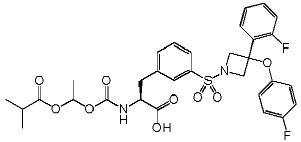 | MS (m/z) : 669 (M+Na)⁺. | |
| 63 | (2S)-3-(3-((3-(3-Carbamoylphenoxy)-3-phenylazetidin-1-yl)sulfonyl)phenyl)-2-(((1-(isobutyryloxy)ethoxy)carbonyl)a mino)propanoic acid | ¹H-NMR (CDCl₃) δ: 1.11 (3H, dd, J = 13.6, 7.0 Hz), 1.19 (3H, t, J = 7.2 Hz), 1.40 (1.5H, d, J = 5.5 Hz), 1.44 (1.5H, d, J = 5.5 Hz), 2.48 (0.5H, dt, J = 14.0, 7.0 Hz), 2.58 (0.5H, dt, J = 14.0, 7.0 Hz), 3.24-3.40 (2H, m), 4.01 (1H, dd, J = 15.9, 9.4 Hz), 4.14-4.27 (2H, m), 4.33 (1H, dd, J = 18.2, 9.4 Hz), 4.60-4.70 (1H, m), 5.25-5.39 (1H, m), 6.39-6.54 (1H, m), 6.72-6.93 (4H, m), 7.12-7.20 (1H, m), 7.20-7.36 (6H, m), 7.43-7.60 (2H, m), 7.68-7.74 (2H, m). | Example 15 |
| | 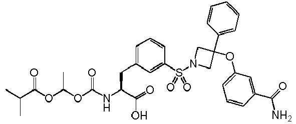 | MS (m/z) : 676 (M+Na)⁺. | |
| 64 | (2S)-3-(3-((3-(4-Carbamoylphenoxy)-3-phenylazetidin-1-yl)sulfonyl)phenyl)-2-(((1-(isobutyryloxy)ethoxy)carbonyl)a mino)propanoic acid | ¹H-NMR (DMSO-d₆) δ: 0.97 (3H, dd, J = 12.5, 7.0 Hz), 1.05 (3H, d, J = 6.8 Hz), 1.19-1.25 (1.5H, m), 1.34 (1.5H, d, J = 5.5 Hz), 2.93-3.08 (1H, m), 3.17-3.26 (1H, m), 4.03-4.16 (3H, m), 4.27 (1H, t, J = 10.6 Hz), 4.37 (1H, t, J = 8.7 Hz), 6.47 (2H, t, J = 8.9 Hz), 6.54 (0.5H, q, J = 5.5 Hz), 6.60 (0.5H, q, J = 5.5 Hz), 7.09-7.20 (3H, m), 7.23-7.33 (3H, m), 7.53 (1H, dd, J = 16.2, 8.2 Hz), 7.60-7.69 (4H, m), 7.72-7.81 (2H, m), signal of one proton was overlapped with DMSO signal. | Example 17 |
| | 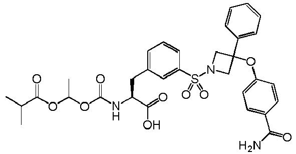 | MS (m/z) : 676 (M+Na)⁺. | |

**[Table 30]**

| | | | |
|---|---|---|---|
| 65 | (2S)-3-(3-((3-(2-Cyanophenoxy)-3-phenylazetidin-1-yl)sulfonyl)phenyl)-2-(((1-(isobutyryloxy)ethoxy)carbonyl )amino)propanoic acid | ¹H-NMR (CDCl₃) δ: 1.13-1.21 (6H, m), 1.46 (3H, d, J = 5.5 Hz), 2.49-2.60 (1H, m), 3.23-3.37 (2H, m), 4.08-4.15 (2H, m), 4.27-4.40 (2H, m), 4.62-4.73 (1H, m), 5.47 (1H, dd, J = 16.6, 7.5 Hz), 6.11 (1H, dd, J = 8.4, 2.6 Hz), 6.81 (1H, q, J = 5.4 Hz), 6.98 (1H, t, J = 7.7 Hz), 7.22-7.28 (1H, m), 7.30-7.42 (5H, m), 7.47-7.58 (3H, m), 7.71-7.76 (2H, br m). | Example 18 |
| | 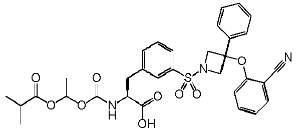 | MS (m/z) : 658 (M+Na)⁺. | |
| 66 | (2S)-3-(3-((3-(4-Bromophenoxy)-3-phenylazetidin-1-yl)sulfonyl)phenyl)-2-(((1-isobutyryloxy)ethoxy)carbonyl) amino)propanoic acid | ¹H-NMR (CDCl₃) δ: 1.12-1.22 (6H, m), 1.42-1.48 (3H, m), 2.44-2.63 (1H, m), 3.14-3.43 (2H, m), 4.11-4.15 (2H, m), 4.21 (1H, d, J = 8.5 Hz), 4.22 (1H, d, J = 8.8 Hz), 4.61-4.73 (1H, m), 5.19-5.31 (1H, m), 6.27 (2H, d, J = 9.0 Hz), 6.77-6.86 (1H, m), 7.16-7.25 (2H, m), 7.28-7.34 (5H, m), 7.47-7.57 (2H, m), 7.67 (1H, br s), 7.73-7.79 (1H, m). | Example 19 |
| | 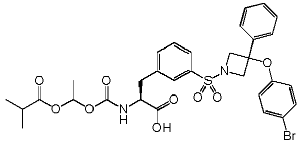 | MS (m/z) : 689 (M+H)⁺. | |
| 67 | (2S)-3-(3-((3-(3-Cyanophenoxy)-3-phenylazetidin-1-yl)sulfonyl)phenyl)-2-(((1-(isobutyryloxy)ethoxy)carbonyl )amino)propanoic acid | ¹H-NMR (CDCl₃) δ: 1.08-1.19 (6H, m), 1.35-1.45 (3H, m), 2.42-2.60 (1H, m), 3.13-3.28 (1H, m), 3.28-3.41 (1H, m), 4.11-4.20 (2H, m), 4.25 (2H, t, J = 9.2 Hz), 4.52-4.64 (1H, m), 6.59-6.67 (2H, m), 6.72-6.83 (1H, m), 7.14-7.35 (8H, m), 7.45-7.59 (2H, m), 7.68-7.75 (2H, m). | Example 20 |
| | 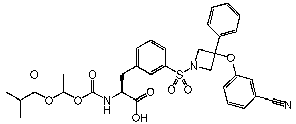 | MS (m/z) : 658 (M+Na)⁺. | |

**[Table 31]**

| | | | |
|---|---|---|---|
| 68 | (2S)-2-(((1-(Isobutyryloxy)ethoxy)carbonyl )amino)-3-(3-((3-phenyl-3-(3-(trifluoromethyl)phenoxy)azetid in-1-yl)sulfonyl)phenyl)propanoic acid | ¹H-NMR (CDCl₃) δ: 1.11-1.20 (6H, m), 1.40-1.53 (3H, m), 2.47-2.60 (1H, m), 3.14-3.38 (2H, m), 4.12-4.20 (2H, m), 4.23-4.29 (2H, m), 4.63-4.72 (1H, m), 5.22-5.30 (1H, m), 6.40-6.45 (1H, m), 6.73 (1H, br s), 6.75-6.82 (1H, m), 7.11-7.22 (2H, m), 7.27-7.36 (5H, m), 7.49-7.57 (2H, m), 7.69 (1H, br s), 7.73-7.78 (1H, m). | Example 21 |
| | 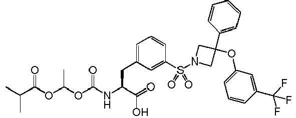 | MS (m/z): 701 (M+Na)⁺. | |
| 69 | (2S)-2-(((1-(Isobutyryloxy)ethoxy)carbonyl )amino)-3-(3-((3-phenoxy-3-phenylazetidin-1-yl)sulfonyl)phenyl)propanoic acid | ¹H-NMR (CDCl₃) δ: 1.12-1.21 (6H, m), 1.42-1.54 (3H, m), 2.48-2.61 (1H, m), 3.17-3.35 (3H, m), 4.10-4.17 (2H, m), 4.20-4.27 (2H, m), 4.60-4.70 (1H, m), 5.19-5.26 (1H, m), 6.34-6.39 (2H, m), 6.77-6.84 (1H, m), 6.85-6.91 (1H, m), 7.07-7.14 (2H, m), 7.27-7.37 (5H, m), 7.49-7.56 (2H, m), 7.64-7.68 (1H, m), 7.74-7.79 (1H, m). | Example 24 |
| | 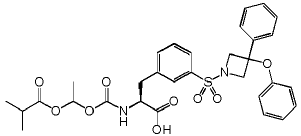 | MS (m/z) : 611 (M+H)⁺. | |
| 70 | (2S)-3-(3-((3-(3-Fluorophenyl)-3-phenoxyazetidin-1-yl)sulfonyl)phenyl)-2-(((1-(isobutyryloxy)ethoxy)carbonyl )amino)propanoic acid | ¹H-NMR (CDCl₃) δ: 1.15 (3H, d, J = 7.0 Hz), 1.19 (3H, d, J = 7.0 Hz), 1.41-1.49 (3H, m), 3.15-3.39 (2H, m), 4.07-4.23 (4H, m), 4.59-4.70 (1H, m), 5.21-5.32 (1H, m), 6.36 (2H, br d, J = 8.3 Hz), 6.74-6.84 (1H, m), 6.90 (1H, br t, J = 7.3 Hz), 6.94-7.04 (2H, m), 7.07-7.19 (3H, m), 7.23-7.35 (1H, m), 7.47-7.57 (2H, m), 7.67 (1H, br s), 7.71-7.79 (1H, m), signal of methine proton was overlapped with water signal. | Example 26 |
| | 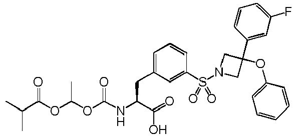 | MS (m/z) : 629 (M+H)⁺. | |

**[Table 32]**

| | | | |
|---|---|---|---|
| 71 | (2S)-3-(3-((3-(2-Fluorophenyl)-3-phenoxyazetidin-1-yl)sulfonyl)phenyl)-2-(((1-(isobutyryloxy)ethoxy)carbonyl )amino)propanoic acid | ¹H-NMR (CDCl₃) δ: 1.10-1.23 (6H, m), 1.40-1.50 (3H, m), 2.45-2.63 (1H, m), 3.16-3.36 (2H, m), 4.25 (2H, d, J = 9.3 Hz), 4.45 (1H, d, J = 8.5 Hz), 4.47 (1H, d, J = 9.0 Hz), 4.62-4.71 (1H, m), 5.21-5.31 (1H, m), 6.45 (2H, d, J = 8.5 Hz), 6.77-6.85 (1H, m), 6.91 (1H, t, J = 7.4 Hz), 7.00-7.07 (2H, m), 7.08-7.16 (2H, m), 7.17-7.32 (2H, m), 7.42-7.54 (2H, m), 7.62-7.69 (1H, m), 7.72-7.79 (1H, m). | Example 27 |
| | 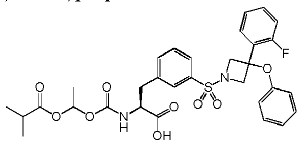 | MS (m/z) : 629 (M+H)⁺. | |
| 72 | (2S)-3-(3-((3-(4-Cyanophenoxy)-3-phenylazetidin-1-yl)sulfonyl)phenyl)-2-(((1-(isobutyryloxy)ethoxy)carbonyl )amino)propanoic acid | ¹H-NMR (CDCl₃) δ: 1.04-1.19 (6H, m), 1.33-1.43 (3H, m), 2.42-2.57 (1H, m), 3.06-3.25 (1H, m), 3.29-3.40 (1H, m), 4.17 (2H, dd, J = 9.2, 4.4 Hz), 4_{.}25 (2H, d, J = 9.0 Hz), 4.45-4.60 (1H, m), 6.47 (2H, d, J = 8.8 Hz), 6.69-6.82 (1H, m), 7.17-7.25 (2H, m), 7.27-7.33 (3H, m), 7.38-7.44 (2H, m), 7.44-7.57 (2H, m), 7.67-7.76 (2H, m). | Example 40 |
| | 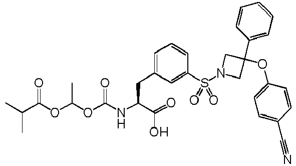 | MS (m/z) : 658 (M+Na)⁺. | |
| 73 | (2S)-2-(((1-((Cyclohexanecarbonyl)oxy)eth oxy)carbonyl)amino)-3-(3-((3-(4-fluorophenoxy)-3-phenylazetidin-1-yl)sulfonyl)phenyl)propanoic acid | ¹H-NMR (CDCl₃) δ: 1.16-1.52 (8H, m), 1.57-1.68 (1H, m), 1.68-1.80 (2H, m), 1_{.}82-1.96 (2H, m), 2.22-2.37 (1H, m), 3_{.}22 (1H, td, J = 13.9, 5.5 Hz), 3.29-3.39 (1H, m), 4.09-4.16 (2H, m), 4.20 (1H, d, J = 9.0 Hz), 4.21 (1H d J = 9.0 Hz) 4.63-4.72 (1H, m), 5.26 (1H, dd, J = 14.7, 7.2 Hz), 6.28-6.34 (2H, m), 6.75-6.84 (3H, m), 7.27-7.35 (5H, m), 7.48-7.57 (2H, m), 7.67 (1H, s), 7.74-7.78 (1H, m). | Example 41 |
| | 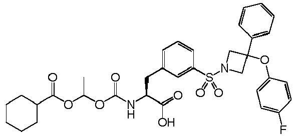 | MS (m/z) : 669 (M+H)⁺. | |

**[Table 33]**

| | | | |
|---|---|---|---|
| 74 | (2S)-3-(3-((3-(4-Fluorophenoxy)-3-phenylazetidin-1-yl)sulfonyl)phenyl)-2-(((1-((3-methylbutanoyl)oxy)ethoxy)car bonyl)amino)propanoic acid | ¹H-NMR (CDCl₃) δ: 0.94 (3H, d, J = 6.5 Hz), 0.97 (3H, d, J = 6.7 Hz), 1.43 (1.5H, d, J = 4.8 Hz), 1.45 (1.5H, d, J = 5.3 Hz), 2.04-2.26 (3H, m), 3.23 (1H, td, J = 14.0, 5.4 Hz), 3.29-3.38 (1H, m), 4.08-4.16 (2H, m), 4.20 (1H, d, J = 9.0 Hz), 4.21 (1H, d, J = 9.0 Hz), 4.64-4.73 (1H, m), 5.23 (1H, dd, J = 11.0, 7.5 Hz), 6.28-6.34 (2H, m), 6.75-6.86 (3H, m), 7.27-7.36 (5H, m), 7.49-7.56 (2H, m), 7.65-7.69 (1H, m), 7.73-7.79 (1H, m). | Example 41 |
| | 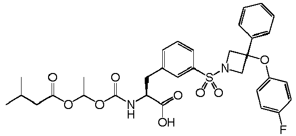 | MS (m/z): 643 (M+H)⁺. | |
| 75 | (2S)-3-(3-((3-(4-Fluorophenoxy)-3-phenylazetidin-1-yl)sulfonyl)phenyl)-2-(((1-(isobutyryloxy)-2-methylpropoxy)carbonyl)amino )propanoic acid | ¹H-NMR (CDCl₃) δ: 0.91-0.99 (6H, m), 1.16 (3H, t, J = 6.9 Hz), 1.20 (3H, dd, J = 7.0, 3.5 Hz), 1.93-2.06 (1H, m), 2.49-2.65 (1H, m), 3.22 (1H, td, J = 13.6, 5.0 Hz), 3.30-3.40 (1H, m), 4.09-4.16 (2H, m), 4.17-4.24 (2H, m), 4.64-4.73 (1H, m), 5.27 (1H, t, J = 7.7 Hz), 6.28-6.34 (2H, m), 6.55-6.59 (1H, m), 6.75-6.83 (2H, m), 7.25-7.36 (5H, m), 7.48-7.58 (2H, m), 7.65-7.70 (1H, m), 7.73-7.78 (1H, m). | Example 41 |
| | 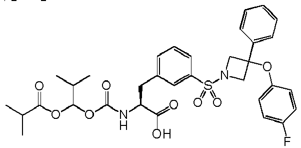 | MS (m/z) : 679 (M+Na)⁺. | |
| 76 | (2S)-3-(3-((3-(3-Fluorophenoxy)-3-phenylazetidin-1-yl)sulfonyl)phenyl)-2-(((1-isobutyryloxy)ethoxy)carbonyl) amino)propanoic acid | ¹H-NMR (CDCl₃) δ: 1.12-1.21 (6H, m), 1.42-1.47 (3H, m), 2.46-2.61 (1H, m), 3.16-3.39 (1H, m), 4.11-4.18 (2H, m), 4.20-4.26 (2H, m), 4.60-4.69 (1H, m), 5.24-5.32 (1H, m), 6.10-6.16 (2H, m), 6.56-6.63 (1H, m), 6.77-6.84 (1H, m), 6.99-7.08 (1H, m), 7.27-7.36 (6H, m), 7.49-7.57 (2H, m), 7.65-7.69 (1H, m), 7.74-7.78 (1H, m). | Example 42 |
| | 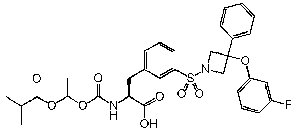 | MS (m/z): 651 (M+Na)⁺. | |

**[Table 34]**

| | | | |
|---|---|---|---|
| 77 | (2S)-3-(3-((3-(3-Fluorophenoxy)-3-phenylazetidin-1-yl)sulfonyl)phenyl)-2-(((1-((3-methylbutanoyl)oxy)ethoxy)car bonyl)amino)propanoic acid | ¹H-NMR (CDCl₃) δ: 0.90-1.00 (6H, m), 1.23-1.28 (2H, m), 1.40-1.48 (3H, m), 2.03-2.13 (1H, m), 2.13-2.24 (1H, m), 3.15-3.38 (2H, m), 4.10-4.19 (2H, m), 4.22 (1H, d, J = 8.8 Hz), 4.23 (1H, d, J = 9.3 Hz), 4.53-4.69 (1H, m), 5.17-5.36 (1H, m), 6.10-6.17 (2H, m), 6.56-6.62 (1H, m), 6.78-6.85 (1H, m), 6.99-7.08 (1H, m), 7.27-7.35 (5H, m), 7.48-7.56 (2H, m), 7.66-7.70 (1H, m), 7.72-7.78 (1H, m). | Example 42 |
| | 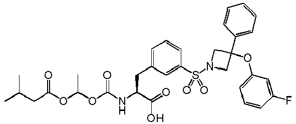 | MS (m/z): 665 (M+Na)⁺. | |
| 78 | (2S)-3-(3-((3-(3-Fluorophenoxy)-3-phenylazetidin-1-yl)sulfonyl)phenyl)-2-((1-(pivaloyloxy)ethoxy)carbonyl)amino)propanoic acid | ¹H-NMR (CDCl₃) δ: 1.17 (4.5H, s), 1.22 (4.5H, s), 1.44 (1.5H, d, J = 5.5 Hz), 1.45 (1.5H, d, J = 5.8 Hz), 3.16-3.38 (2H, m), 4.11-4.18 (2H, m), 4.19-4.26 (2H, m), 4.60-4.69 (1H, m), 5.25-5.31 (1H, m), 6.10-6.16 (2H, m), 6.56-6.62 (1H, m), 6.75-6.82 (1H, m), 6.99-7.08 (1H, m), 7.27-7.36 (5H, m), 7.50-7.56 (2H, m), 7.66-7.69 (1H, m), 7.74-7.78 (1H, m). | Example 42 |
| | 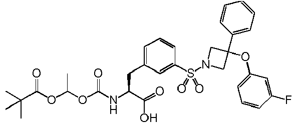 | MS (m/z): 665 (M+Na)⁺. | |
| 79 | (2S)-2-(((1-((Cyclohexanecarbonyl)oxy)eth oxy)carbonyl)amino)-3-(3-((3-(3-fluorophenoxy)-3-phenylazetidin-1-yl)sulfonyl)phenyl)propanoic acid | ¹H-NMR (CDCl₃) δ: 1.17-1.52 (8H, m), 1.57-1.68 (1H, m), 1.68-1.81 (2H, m), 1.82-1.96 (2H, m), 2.22-2.37 (1H, m), 3.16-3.39 (2H, m), 4.11-4.18 (3H, m), 4.22 (1H, d, J = 9.0 Hz), 4.23 (1H, d, J = 8.8 Hz), 4.61-4.71 (1H, m), 5.22-5.32 (1H, m), 6.10-6.16 (2H, m), 6.56-6.63 (1H, m), 6.76-6.84 (1H, m), 6.99-7.09 (1H, m), 7.27-7.36 (5H, m), 7.48-7.58 (2H, m), 7.67 (1H, br s), 7.73-7.79 (1H, m). | Example 42 |
| | 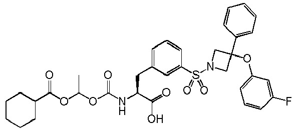 | MS (m/z) : 669 (M+H)⁺. | |

**[Table 35]**

| | | | |
|---|---|---|---|
| 80 | (2S)-3-(3-((3-(3-Fluorophenoxy)-3-phenylazetidin-1-yl)sulfonyl)phenyl)-2-(((1-((tetrahydro-2H-pyran-4-carbonyl)oxy)ethoxy)carbonyl)am ino)propanoic acid | ¹H-NMR (CDCl₃) δ: 1.44 (3H, d, J = 5.5 Hz), 2.46-2.65 (1H, m), 3.15-3.49 (4H, m), 3.89-4.01 (2H, m), 4.13 (1H, d, J = 8.8 Hz), 4.16 (1H, d, J = 8.5 Hz), 4.23 (1H, d, J = 9.0 Hz), 4.24 (1H, d, J = 8.8 Hz), 4.57-4.68 (1H, m), 5.24-5.34 (1H, m), 6.09-6.16 (2H, m), 6.56-6.63 (1H, m), 6.77-6.85 (1H, m), 6.99-7.08 (1H, m), 7.27-7.36 (5H, m), 7.49-7.56 (2H, m), 7.64-7.70 (1H, m), 7.73-7.78 (1H, m), signals of 4 protons were overlapped with H₂O signal. | Example 42 |
| | 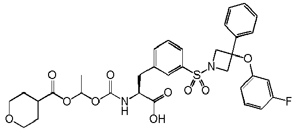 | MS (m/z): 693 (M+Na)⁺. | |
| 81 | (S)-3-(3-((3-(3-Fluorophenoxy)-3-phenylazetidin-1-yl)sulfonyl)phenyl)-2-((((2-(isobutyryloxy)propan-2-yl)oxy)carbonyl)amino)propanoic acid | ¹H-NMR (CDCl₃) δ: 1.14 (6H, d, J = 7.0 Hz), 1.80 (6H, s), 2.47-2.56 (1H, m), 3.18 (1H, dd, J = 14.2, 5.1 Hz), 3.31 (1H, dd, J = 14.1, 5.8 Hz), 4.14 (1H, d, J = 9.3 Hz), 4.15 (1H, d, J = 9.0 Hz), 4.22 (1H, d, J = 9.0 Hz), 4.23 (1H, d, J = 9.0 Hz), 4.55-4.65 (1H, m), 5.21-5.31 (1H, m), 6.10-6.16 (2H, m), 6.56-6.62 (1H, m), 6.99-7.08 (1H, m), 7.27-7.36 (5H, m), 7.51-7.56 (2H, m), 7.69 (1H, br s), 7.73-7.79 (1H, m). | Example 42 |
| | 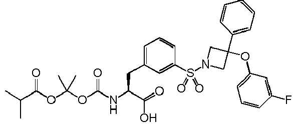 | MS (m/z): 665 (M+Na)⁺. | |
| 82 | (S)-3-(3-((3-(3-Fluorophenoxy)-3-phenylazetidin-1-yl)sulfonyl)phenyl)-2-((((2-pivaloyloxy)propan-2-yl)oxy)carbonyl)amino)propanoic acid | ¹H-NMR (CDCl₃) δ: 1.19 (9H, s), 1.80 (6H, s), 3.19 (1H, dd, J = 14.1, 6.0 Hz), 3.30 (1H, dd, J = 13.8, 5.8 Hz), 4.14 (1H, d, J = 9.3 Hz), 4.15 (1H, d, J = 9.3 Hz), 4.23 (1H, d, J = 9.0 Hz), 4.24 (1H, d, J = 9.3 Hz), 4.56-4.65 (1H, m), 5.19-5.27 (1H, m), 6.10-6.16 (2H, m), 6.56-6.63 (1H, m), 6.99-7.08 (1H, m), 7.28-7.36 (5H, m), 7.51-7.56 (2H, m), 7.69 (1H, br s), 7.73-7.79 (1H, m). | Example 42 |
| | 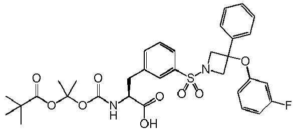 | MS (m/z) : 679 (M+Na)⁺. | |

**[Table 36]**

| | | | |
|---|---|---|---|
| 83 | (2S)-3-(3-((3-(4-Fluorophenoxy)-3-(pyridin-2-yl)sulfonyl)phenyl)-2-(((1-(isobutyryloxy)ethoxy)carbonyl)a mino)propanoic acid | ¹H-NMR (CDCl₃) δ: 1.17 (3H, d, J = 7.0 Hz), 1.22 (3H, dd, J = 6.9, 4.7 Hz), 1.49 (1.5H, d, J = 5.4 Hz), 1.51 (1.5H, d, J = 5.4 Hz), 2.53 (0.5H, dt, J = 14.0, 7.0 Hz), 2.60 (0.5H, dt, J = 14.0, 7.0 Hz), 3.19 (0.5H, dd, J = 13.4, 6.3 Hz), 3.27 (0.5H, dd, J = 13.4, 6.3 Hz), 3.34-3.43 (1H, m), 4.18 (1H, d, J = 8.0 Hz), 4.22-4.33 (2H, m), 4.48 (1H, dd, J = 16.9, 8.4 Hz), 4.65-4.73 (1H, m), 5.45 (1H, dd, J = 28.0, 6.7 Hz), 6.35-6.42 (2H, m), 6.79-6.91 (3H, m), 7.34-7.41 (1H, m), 7.42-7.55 (3H, m), 7.72-7.84 (2H, m), 7.91 (1H, d, J = 7.8 Hz), 8.62-8.67 (1H, m). | Example 43 |
| | 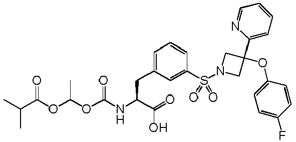 | MS (m/z) : 630 (M+H)⁺. | |

### [Example 84]

### (2S)-2-(((1-Acetoxyethoxy)carbonyl)amino)-3-(3-((3-(4-fluorophenoxy)-3-phenylazetidin-1-yl)sulfonyl)phenyl)propanoic acid

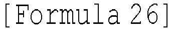

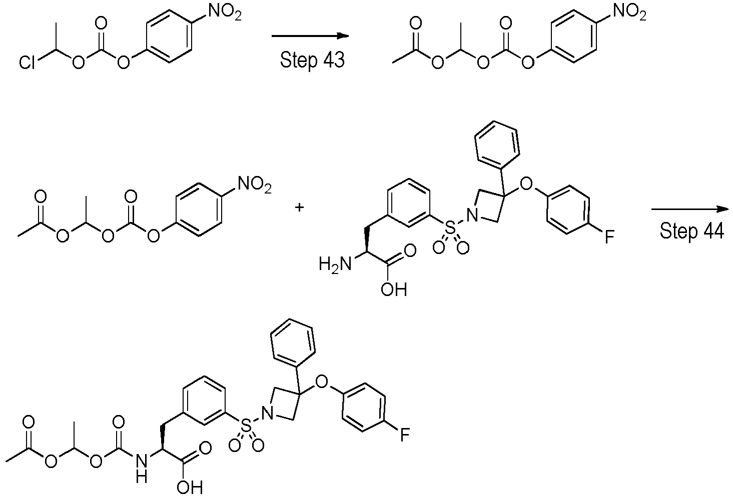

### [Step 43]

### 1-(((4-Nitrophenoxy)carbonyl)oxy)ethyl acetate

A suspension of the compound (150 g) obtained by the method of Step 40 and mercury(II) acetate (233 g) in acetic acid (1 1) was stirred at room temperature for 71 hours, then acetic acid was distilled off under reduced pressure. The residue was suspended in diethyl ether and water, and sodium hydrogen carbonate (180 g) was slowly added to the suspension under stirring. The aqueous layer was extracted with diethyl ether, and the extract was washed with a 0.5 N aqueous sodium hydroxide solution, a saturated aqueous sodium hydrogen carbonate solution, water and saturated saline, and then dried over sodium sulfate. After the sodium sulfate was filtered off, the filtrate was concentrated under reduced pressure. To the residue, ethyl acetate (30 ml) was added, and the mixture was stirred at 70°C to dissolve the residue. n-Hexane (250 ml) was added, and the mixture was stirred at room temperature for 15 hours. The precipitate produced was collected by filtration to obtain the title compound (149 g).
¹H-NMR (CDCl₃) δ: 1.62 (3H, d, J = 5.5 Hz), 2.14 (3H, s), 6.84 (1H, q, J = 5.5 Hz), 7.38-7.43 (2H, m), 8.26-8.31 (2H, m).

### [Step 44]

### (2S)-2-(((1-Acetoxyethoxy)carbonyl)amino)-3-(3-((3-(4-fluorophenoxy)-3-phenylazetidin-1-yl)sulfonyl)phenyl)propanoic acid

To a mixed solution of the compound (136 mg) of Example 41 and the compound (93 mg) obtained by the method of Step 43 in tetrahydrofuran (1.4 ml) and water (0.7 ml), triethylamine (161 µl) was added at room temperature, and the mixture was stirred at room temperature for 5 hours. To the reaction solution, a 10% aqueous sodium hydrogen sulfate solution was added, and the mixture was extracted with ethyl acetate. The organic layer was dried over sodium sulfate. After the sodium sulfate was filtered off, the filtrate was concentrated under reduced pressure. The residue was purified by silica gel column chromatography (n-hexane/ethyl acetate) to obtain the title compound (178 mg).
¹H-NMR (CDCl₃) δ: 1.41-1.48 (3H, m), 2.05 (3H, s), 3.18-3.41 (2H, m), 4.09-4.17 (2H, m), 4.21 (1H, d, J = 8.5 Hz), 4.21 (1H, d, J = 8.5 Hz), 4.63-4.73 (1H, m), 5.27 (1H, dd, J = 14.8, 7.3 Hz), 6.28-6.35 (2H, m), 6.76-6.84 (3H, m), 7.27-7.37 (5H, m), 7.49-7.56 (2H, m), 7.68 (1H, br s), 7.73-7.80 (1H, m).
MS (m/z) : 601 (M+H)⁺.

The same procedures as in Example 84 were performed to obtain the compounds below. For the raw materials of Step 44, the corresponding Example compounds at the right of the following table were used.

**[Table 37]**

| Example | Name and Structure | Equipment data | Raw material compound of Step 44 |
|---|---|---|---|
| 85 | (2S)-2-(((1-Acetoxyethoxy)carbonyl)amino) -3-(3-((3-(3-fluorophenoxy)-3-(4-fluorophenyl)azetidin-1-yl)sulfonyl)phenyl)propanoic acid | ¹H-NMR (CDCl₃) δ: 1.41-1.51 (3H, m), 2.05 (1.5H, s), 2.08 (1.5H, s), 3.17-3.40 (2H, m), 4.09-4.22 (4H, m), 4.62-4.73 (1H, m), 5.22-5.32 (1H, m), 6.09-6.15 (2H, m), 6.58-6.64 (1H, m), 6.76-6.83 (1H, m), 6.99-7.10 (3H, m), 7.27-7.33 (2H, m), 7.50-7.57 (2H, m), 7.68 (1H, br s), 7.73-7.78 (1H, m). | Example 1 |
| | 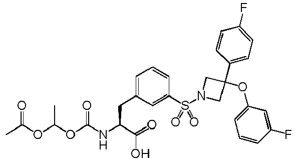 | MS (m/z): 641 (M+Na)⁺. | |
| 86 | (S)-3-(3-((3-(4-Fluorophenoxy)-3-phenylazetidin-1-yl)sulfonyl)phenyl)-2-((((2-pivaloyloxy)propan-2-yl)oxy)carbonyl)amino)propano ic acid | ¹H-NMR (CDCl₃) δ: 1.18 (9H, s), 1.79 (6H, s), 3.19 (1H, dd, J = 13.9, 5.6 Hz), 3.31 (1H, dd, J = 13.9, 5.6 Hz), 4.13 (1H, d, J = 9.0 Hz), 4.14 (1H, d, J = 9.0 Hz), 4.20 (1H, d, J = 9.0 Hz), 4.21 (1H, d, J = 9.0 Hz), 4.57-4.65 (1H, m), 5.28 (1H, br d, J = 7.5 Hz), 6.28-6.35 (2H, m), 6.75-6.83 (2H, m), 7.27-7.36 (5H, m), 7.50-7.55 (2H, m), 7.70 (1H, br s), 7.73-7.78 (1H, m). | Example 41 |
| | 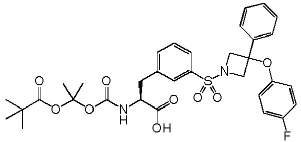 | MS (m/z): 679 (M+Na)⁺. | |

**[Table 38]**

| | | | |
|---|---|---|---|
| 87 | (2S)-2-(((1-Acetoxyethoxy)carbonyl)amino )-3-(3-((3-(3-fluorophenoxy)-3-phenylazetidin-1-yl)sulfonyl)phenyl)propanoic acid | ¹H-NMR (CDCl₃) δ: 1.42 (1.5H, d, J = 5.3 Hz), 1.43 (1.5H, d, J = 5.0 Hz), 2.03 (1.5H, s), 2.08 (1.5H, s), 3.15-3.39 (2H, m), 4.12-4.18 (2H, m), 4.19-4.26 (2H, m), 4.54-4.66 (1H, m), 5.28-5.41 (1H, m), 6.10-6.15 (2H, m), 6.56-6.62 (1H, m), 6.75-6.83 (1H, m), 6.99-7.08 (1H, m), 7.26-7.35 (5H, m), 7.50-7.55 (2H, m), 7.66-7.71 (1H, m), 7.72-7.77 (1H, m). | Example 42 |
| | 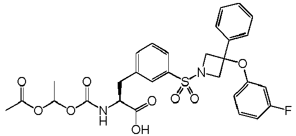 | MS (m/z): 623 (M+Na)⁺. | |
| 88 | (S)-2-((((2-Acetoxypropan-2-yl)oxy)carbonyl)amino)-3-(3-((3-(3-fluorophenoxy)-3-phenylazetidin-1-yl)sulfonyl)phenyl)propanoic acid | ¹H-NMR (CDCl₃) δ: 1.80 (6H, s), 2.03 (3H, s), 3.19 (1H, dd, J = 13.8, 5.5 Hz), 3. 31 (1H, dd, J = 13.8, 5.8 Hz), 4.15 (1H, d, J = 9.0 Hz), 4.16 (1H, d, J = 9 .0 Hz), 4.23 (1H, d, J = 9.0 Hz), 4.24 (1H, d, J = 9.0 Hz), 4.56-4.64 (1H, m), 5.29 (1H, br d, J = 7.0 Hz), 6.10-6.16 (2H, m), 6.56-6.62 (1H, m), 6.99-7.08 (1H, m), 7.27-7.36 (5H, m), 7.50-7.56 (2H, m), 7.69 (1H, br s), 7.73-7.78 (1H, m). | Example 42 |
| | 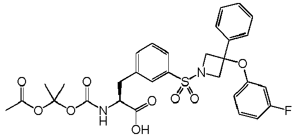 | MS (m/z) : 637 (M+Na)⁺. | |

### [Example 89]

### (S)-2-((((R)-1-Acetoxyethoxy)carbonyl)amino)-3-(3-((3-(3-fluorophenoxy)-3-phenylazetidin-1-yl)sulfonyl)phenyl)propanoic acid

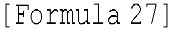

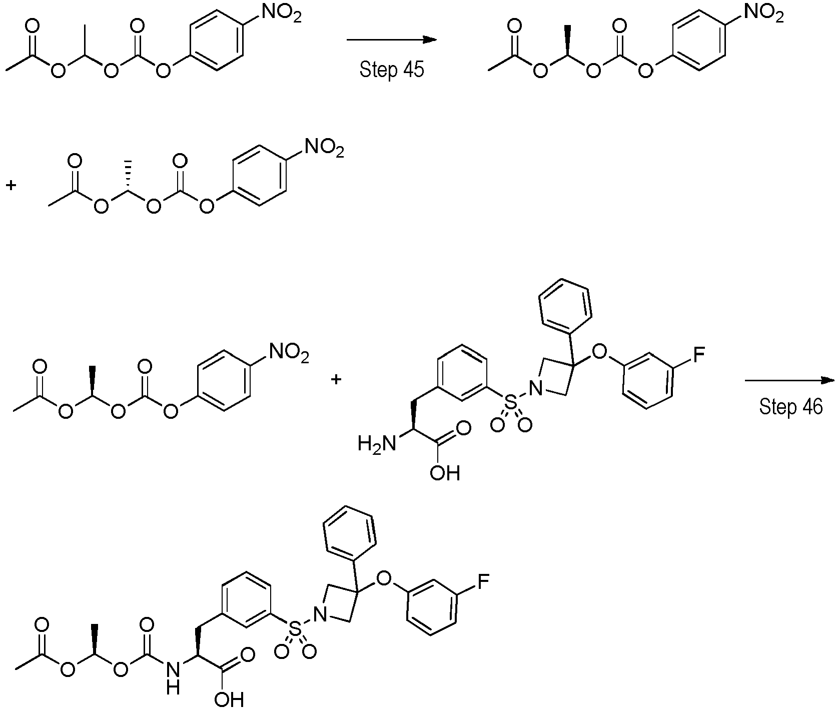

### [Step 45]

### (R)-1-((4-Nitrophenoxy)carbonyl)oxy)ethyl acetate

### (S)-1-((4-Nitrophenoxy)carbonyl)oxy)ethyl acetate

The racemate compound (1.5 g) obtained by the method of Step 43 was purified by chiral column chromatography (CHIRALFLASH IC, particle diameter: 20 µm, size: 30 mmφ x 100 mm, mobile layer: n-hexane/ethyl acetate/acetic acid = 94/6/0.1, flow rate: 12 ml/min, temperature: room temperature, wavelength: 254 nm) in several portions to obtain the title compound (R configuration: 502 mg, S configuration: 550 mg).
¹H-NMR (CDCl₃) δ: 1.62 (3H, d, J = 5.5 Hz), 2.14 (3H, s), 6.84 (1H, q, J = 5.5 Hz), 7.38-7.43 (2H, m), 8.26-8.31 (2H, m).
CHIRALPAK ID, size: 0.46 cmφ x 25 cm, mobile layer: n-hexane/ethyl acetate = 94/6, flow rate: 1.0 ml/min, temperature: 35°C, wavelength: 254 nm, retention time: 19.5 min (R configuration), 17.4 min (S configuration)

### [Step 46]

### (S)-2-((((R)-1-Acetoxyethoxy)carbonyl)amino)-3-(3-((3-(3-fluorophenoxy)-3-phenylazetidin-1-yl)sulfonyl)phenyl)propanoic acid

To a mixed solution of the compound (600 mg) of Example 42 and the compound (R configuration: 354 mg) obtained by the method of Step 45 in tetrahydrofuran (5 ml) and water (1 ml), triethylamine (0.36 ml) was added at room temperature, and the mixture was stirred at room temperature for 4 hours. To the reaction solution, an aqueous 10% potassium hydrogen sulfate solution was added, and the mixture was extracted with ethyl acetate. The organic layer was dried over sodium sulfate. After the sodium sulfate was filtered off, the filtrate was concentrated under reduced pressure. The residue was purified by silica gel column chromatography (n-hexane/ethyl acetate and chloroform/methanol), suspended in hexane/ethyl acetate, and collected by filtration to obtain the title compound (737 mg).
¹H-NMR (CDCl₃) δ: 1.44 (3H, d, J = 5.5 Hz), 2.04 (3H, s), 3.21 (1H, dd, J = 13.8, 5.8 Hz), 3.32 (1H, dd, J = 14.1, 5.5 Hz), 4.13 (1H, d, J = 9.0 Hz), 4.16 (1H, d, J = 9.3 Hz), 4.22 (1H, d, J = 9.0 Hz), 4.24 (1H, d, J = 9.0 Hz), 4.62-4.70 (1H, m), 5.32 (1H, d, J = 7.3 Hz), 6.09-6.16 (2H, m), 6.57-6.62 (1H, m), 6.79 (1H, q, J = 5.4 Hz), 7.01-7.08 (1H, m), 7.27-7.37 (5H, m), 7.49-7.57 (2H, m), 7.68 (1H, br s), 7.74-7.79 (1H, m).
MS (m/z) : 601 (M+H)⁺.

The same procedures as in Example 89 were performed to obtain the compounds below. For the raw materials of Step 46, the corresponding Example compounds at the right of the following table were used.

**[Table 39]**

| Example | Name and Structure | Equipment data | Raw material compound of Step 46 |
|---|---|---|---|
| 90 | (S)-2-((((R)-1-Acetoxyethoxy)carbonyl)amino)-3-(3-((3-(4-fluorophenoxy)-3-phenylazetidin-1-yl)sulfonyl)phenyl)propanoic acid | ¹H-NMR (CDCl₃) δ: 1.44 (3H, d, J = 5.5 Hz), 2.04 (3H, s), 3.22 (1H, dd, J = 13.9, 5.6 Hz), 3.32 (1H, dd, J = 13.9, 5.6 Hz), 4.11 (1H, d, J = 9.5 Hz), 4.14 (1H, d, J = 9.5 Hz), 4.20 (1H, d, J = 9.0 Hz), 4.22 (1H, d, J = 9.0 Hz), 4.66 (1H, dd, J = 13.2, 5.6 Hz), 5.29 (1H, d, J = 7.3 Hz), 6.28-6.35 (2H, m), 6.76-6.83 (3H, m), 7.26-7.36 (5H, m), 7.50-7.56 (2H, m), 7.66-7.70 (1H, m), 7.76 (1H, dt, J = 6.5, 2.2 Hz). | Example 41 |
| | 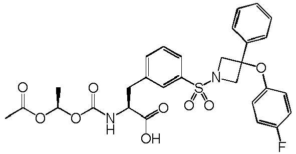 | MS (m/z): 623 (M+Na)⁺. | |
| 91 | (S)-2-((((S)-1-Acetoxyethoxy)carbonyl)amino)-3-(3-((3-(4-fluorophenoxy)-3-phenylazetidin-1- yl)sulfonyl)phenyl)propanoic acid | ¹H-NMR (CDCl₃) δ: 1.44 (3H, d, J = 5.5 Hz), 2.09 (3H, s), 3.25 (1H, dd, J = 14.1, 5.0 Hz), 3.36 (1H, dd, J = 14.1, 5.8 Hz), 4.10-4.17 (2H, m), 4.18-4.24 (2H, m), 4.67 (1H, dd, J = 12.8, 5.8 Hz), 5.26 (1H, d, J = 6.8 Hz), 6.28-6.35 (2H, m), 6.75-6.84 (3H, m), 7.27-7.35 (5H, m), 7.49-7.56 (2H, m), 7.67 (1H, s), 7.73-7.79 (1H, m). | Example 41 |
| | 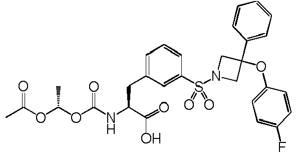 | MS (m/z): 623 (M+Na)⁺. | |
| 92 | (S)-3-(3-((3-(4-Fluorophenoxy)-3-phenylazetidin-1-yl)sulfonyl)phenyl)-2-((((R)-1-(pivaloyloxy)ethoxy)carbonyl)ami no)propanoic acid | ¹H-NMR (CDCl₃) δ: 1.17 (9H, s), 1.43 (3H, d, J = 5.5 Hz), 3.20 (1H, dd, J = 13.8, 5.8 Hz), 3.33 (1H, dd, J = 13.9, 5.9 Hz), 4.12 (1H, d, J = 8.8 Hz), 4.14 (1H, d, J = 9.0 Hz), 4.20 (1H, d, J = 8.8 Hz), 4.21 (1H, d, J = 9.0 Hz), 4.66 (1H, dd, J = 12.9, 5.6 Hz), 5.29 (1H, d, J = 7.3 Hz), 6.28-6.35 (2H, m), 6.74-6.83 (3H, m), 7.27-7.35 (5H, m), 7.49-7.56 (2H, m), 7.68 (1H, s), 7.72-7.78 (1H, m). | Example 41 |
| | 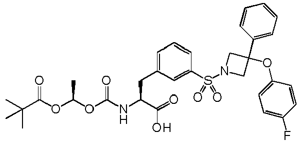 | MS (m/z): 665 (M+Na)⁺. | |

**[Table 40]**

| | | | |
|---|---|---|---|
| 93 | (S)-3-(3-((3-(4-Fluorophenoxy)-3-phenylazetidin-1-yl)sulfonyl)phenyl)-2-((((S)-1-(pivaloyloxy)ethoxy)carbonyl)am ino)propanoic acid | ¹H-NMR (CDCl₃) δ: 1.22 (9H, s), 1.45 (3H, d, J = 5.3 Hz), 3.24 (1H, dd, J = 13.6, 5.0 Hz), 3.35 (1H, dd, J = 13.8, 5.3 Hz), 4.12 (1H, d, J = 8.8 Hz), 4.14 (1H, d, J = 8.5 Hz), 4.20 (1H, d, J = 8.8 Hz), 4.21 (1H, d, J = 9.0 Hz), 4.62-4.70 (1H, m), 5.23-5.30 (1H, m), 6.28-6.34 (2H, m), 6.75-6.83 (3H, m), 7.27-7.35 (5H, m), 7.48-7.57 (2H, m), 7.67 (1H, br s), 7.73-7.77 (1H, m). | Example 41 |
| | 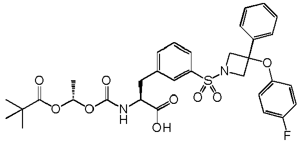 | MS (m/z): 665 (M+Na)⁺. | |
| 94 | (S)-3-(3-((3-(3-Fluorophenoxy)-3-phenylazetidin-1-yl)sulfonyl)phenyl)-2-((((R)-1-(pivaloyloxy)ethoxy)carbonyl)am ino)propanoic acid | ¹H-NMR (CDCl₃) δ: 1.18 (9H, s), 1.44 (3H, d, J = 5.3 Hz), 3.20 (1H, dd, J = 13.8, 5.8 Hz), 3.33 (1H, dd, J = 13.8, 5.8 Hz), 4.13 (1H, d, J = 8.8 Hz), 4.15 (1H, d, J = 8.8 Hz), 4.22 (1H, d, J = 9.0 Hz), 4.24 (1H, d, J = 9.0 Hz), 4.65 (1H, dd, J = 13.1, 5.5 Hz), 5.27 (1H, d, J = 7.5 Hz), 6.10-6.15 (2H, m), 6.56-6.63 (1H, m), 6.78 (1H, q, J = 5.4 Hz), 6.99-7.08 (1H, m), 7.27-7.36 (5H, m), 7.50-7.56 (2H, m), 7.68 (1H, s), 7.74-7.78 (1H, m). | Example 42 |
| | 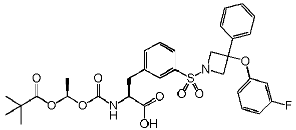 | MS (m/z): 665 (M+Na)⁺. | |
| 95 | (S)-3-(3-((3-(3-Fluorophenoxy)-3-phenylazetidin-1-yl)sulfonyl)phenyl)-2-((((S)-1-(pivaloyloxy)ethoxy)carbonyl)am ino)propanoic acid | ¹H-NMR (CDCl₃) δ: 1.22 (9H, s), 1.45 (3H, d, J = 5.5 Hz), 3.24 (1H, dd, J = 13.7, 4.9 Hz), 3.34 (1H, dd, J = 14.1, 5.8 Hz), 4.14 (1H, d, J = 9.3 Hz), 4.15 (1H, d, J = 9.0 Hz), 4.22 (1H, d, J = 9.0 Hz), 4.23 (1H, d, J = 9.0 Hz), 4.65-4.72 (1H, m), 5.21 (1H, d, J = 7.3 Hz), 6.10-6.16 (2H, m), 6.56-6.62 (1H, m), 6.79 (1H, q, J = 5.5 Hz), 6.99-7.08 (1H, m), 7.28-7.36 (5H, m), 7.50-7.57 (2H, m), 7.67 (1H, br s), 7.75-7.79 (1H, m). | Example 42 |
| | 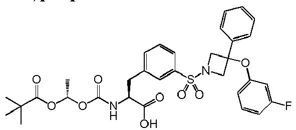 | MS (m/z): 665 (M+Na)⁺. | |

### [Example 96]

### (S)-3-(3-((3-(3-Fluorophenoxy)-3-(4-fluorophenyl)azetidin-1-yl)sulfonyl)phenyl)-2-((((S)-1-(pivaloyloxy)ethoxy)carbonyl)amino)propanoic acid hemi-piperazine salt

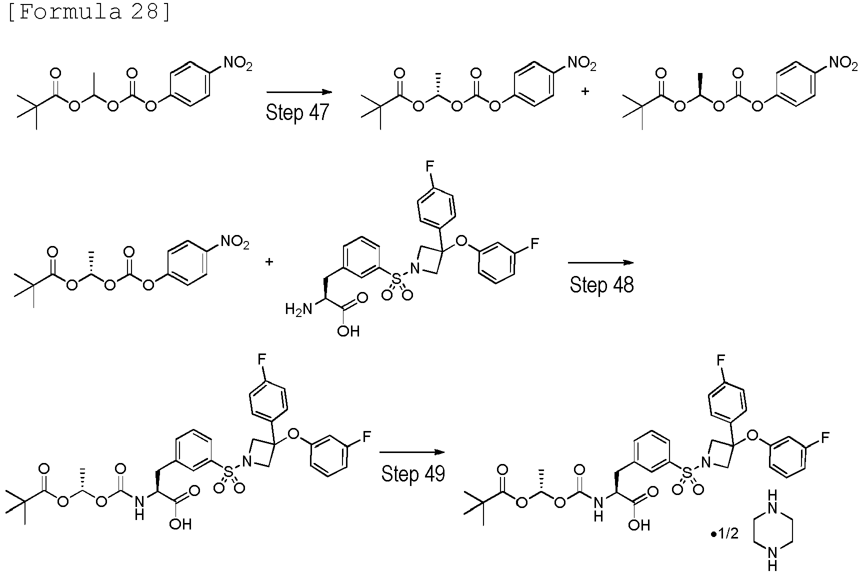

### [Step 47]

### (S)-1-(((4-Nitrophenoxy)carbonyl)oxy)ethyl pivalate

### (R)-1-(((4-Nitrophenoxy)carbonyl)oxy)ethyl pivalate

The racemate (500 mg) obtained by the method of Step 41 was purified by chiral column chromatography (CHIRALFLASH IC, particle diameter: 20 µm, size: 30 mmφ x 100 mm, mobile layer: n-hexane/ethyl acetate = 95/5, flow rate: 12 ml/min, temperature: room temperature, wavelength: 254 nm) in four portions to obtain the title compound (S configuration: 232 mg, R configuration: 242 mg) .
¹H-NMR (CDCl₃) δ: 1.24 (9H, s), 1.62 (3H, d, J = 5.5 Hz), 6.82 (1H, q, J = 5.4 Hz), 7.37-7.42 (2H, m), 8.26-8.31 (2H, m).
CHIRALPAK IC, size: 0.46 cmφ x 25 cm, mobile layer: n-hexane/ethyl acetate = 95/5, flow rate: 1.0 ml/min, temperature: 35°C, wavelength: 254 nm, retention time: 14.4 min (S configuration), 12.2 min (R configuration)

### [Step 48]

### (S)-3-(3-((3-(3-Fluorophenoxy)-3-(4-fluorophenyl)azetidin-1-yl)sulfonyl)phenyl)-2-((((S)-1-(pivaloyloxy)ethoxy)carbonyl)amino)propanoic acid

To a mixed solution of the compound (0.5 g) of Example 1 and the compound (S configuration: 0.350 g) obtained by the method of Step 47 in tetrahydrofuran (5 ml) and water (1 ml), triethylamine (0.571 ml) was added at room temperature, and the mixture was stirred at room temperature overnight. Water was added to the reaction solution and the mixture was extracted with n-hexane/ethyl acetate, and then the combined organic layer was washed with an aqueous potassium hydrogen sulfate solution and saturated saline sequentially, and dried over sodium sulfate. The sodium sulfate was filtered off and the filtrate was concentrated and the obtained residue was purified by silica gel column chromatography (n-hexane/ethyl acetate, followed by replacement with chloroform/methanol) to obtain the title compound (618 mg) .
MS (m/z) : 683 (M+Na)⁺.

### [Step 49]

### (S)-3-(3-((3-(3-Fluorophenoxy)-3-(4-fluorophenyl)azetidin-1-yl)sulfonyl)phenyl)-2-((((S)-1-(pivaloyloxy)ethoxy)carbonyl)amino)propanoic acid hemi-piperazine salt

To a solution of the compound obtained by the method of Step 48 (618 mg) in ethyl acetate (6.2 ml), a solution of 5% piperazine/ethyl acetate (0.806 ml) was added, and the precipitated solid was collected by filtration to obtain the title compound (529 mg).
¹H-NMR (CDCl₃) δ: 1.23 (9H, s), 1.44 (3H, d, J = 5.5 Hz), 3.07-3.14 (4H, m), 3.24 (1H, dd, J = 13.6, 3.8 Hz), 3.36 (1H, dd, J = 13.4, 5.4 Hz), 4.11-4.19 (4H, m), 4.39-4.46 (1H, m), 5.50 (1H, d, J = 6.0 Hz), 6.07-6.13 (2H, m), 6.57-6.64 (1H, m), 6.81 (1H, q, J = 5.4 Hz), 6.97-7.09 (3H, m), 7.20-7.26 (2H, m), 7.48 (1H, t, J = 7.8 Hz), 7.52-7.57 (1H, m), 7.65-7.71 (2H, m).
MS (m/z) : 683 (M+Na)⁺.

The same procedures as in Example 96 were performed to obtain the compounds below. For the raw materials of Step 48, the corresponding Example compounds at the right of the following table were used.

**[Table 41]**

| Example | Name and Structure | Equipment data | Raw material compound of Step 48 |
|---|---|---|---|
| 97 | (S)-3-(3-((3-(3-Fluorophenoxy)-3-phenylazetidin-1-yl)sulfonyl)phenyl)-2-((((S)-1-(pivaloyloxy)ethoxy)carbonyl)a mino)propanoic acid hemi-piperazine salt | ¹H-NMR (CDCl₃) δ: 1.24 (9H, s), 1.44 (3H, d, J = 5.5 Hz), 3.08-3.15 (4H, m), 3.23 (1H, dd, J = 13.7, 3.9 Hz), 3.36 (1H, dd, J = 13.4, 5.4 Hz), 4.12-4.16 (2H, m), 4.17-4.22 (2H, m), 4.40-4.45 (1H, m), 5.50 (1H, d, J = 6.0 Hz), 6.08-6.13 (2H, m), 6.56-6.62 (1H, m), 6.81 (1H, q, J = 5.4 Hz), 6.99-7.07 (1H, m), 7.22-7.34 (5H, m), 7.48 (1H, t, J = 7.5 Hz), 7.52-7.57 (1H, m), 7.65-7.71 (2H, m). | Example 42 |
| | 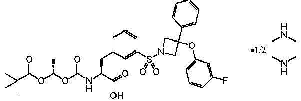 | MS (m/z): 665 (M+Na)⁺. | |
| 98 | (S)-3-(3-((3-(3-Fluorophenoxy)-3-(4-fluorophenyl)azetidin-1-yl)sulfonyl)phenyl)-2-((((R)-1-(pivaloyloxy)ethoxy)carbonyl)a mino)propanoic acid hemi-piperazine salt | ¹H-NMR (CDCl₃) δ: 1.17 (9H, s), 1.44 (3H, d, J = 5.3 Hz), 3.11 (4H, s), 3.21 (1H, dd, J = 13.7, 4.9 Hz), 3.33 (1H, dd, J = 13.7, 5.1 Hz), 4.14 (2H, d, J = 9.3 Hz), 4.17 (1H, d, J = 9.3 Hz), 4.18 (1H, d, J = 9.3 Hz), 4.39-4.45 (1H, m), 5.53 (1H, d, J = 6.3 Hz), 6.08-6.13 (2H, m), 6.57-6.63 (1H, m), 6.80 (1H, q, J = 5.4 Hz), 6.97-7.09 (3H, m), 7.22-7.29 (2H, m), 7.46-7.53 (2H, m), 7.66-7.71 (2H, m). | Example 1 |
| | 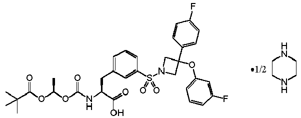 | MS (m/z): 683 (M+Na)⁺. | |
| 99 | (S)-2-((((S)-1-Acetoxyethoxy)carbonyl)amino )-3-(3-((3-(3-fluorophenoxy)-3- phenylazetidin-1-yl)sulfonyl)phenyl)propanoic acid hemi-piperazine salt | ¹H-NMR (CDCl₃) δ: 1.43 (3H, d, J = 5.3 Hz), 2.09 (3H, s), 3.11 (4H, s), 3.23 (1H, dd, J = 13.6, 4.3 Hz), 3.35 (1H, dd, J = 13.6, 5.5 Hz), 4.15 (2H, d, J = 9.0 Hz), 4.20 (2H, d, J = 9.5 Hz), 4.41 (1H, br q, J = 5.4 Hz), 5.56 (1H, br d, J = 6.5 Hz), 6.08-6.14 (2H, m), 6.55-6.62 (1H, m), 6.82 (1H, q, J = 5.4 Hz), 7.04 (1H, td, J = 8.3, 6.7 Hz), 7.22-7.34 (5H, m), 7.45-7.57 (2H, m), 7.64-7.72 (2H, m). | Example 42 |
| | 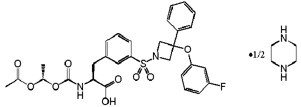 | MS (m/z): 601 (M+H)⁺. | |

### [Example 100]

### (S)-3-(3-((3-(4-Fluorophenoxy)-3-phenylazetidin-1-yl)sulfonyl)phenyl)-2-((((R)-1-(((tetrahydro-2H-pyran-4-carbonyl)oxy)ethoxy)carbonyl)amino)propanoic acid

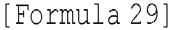

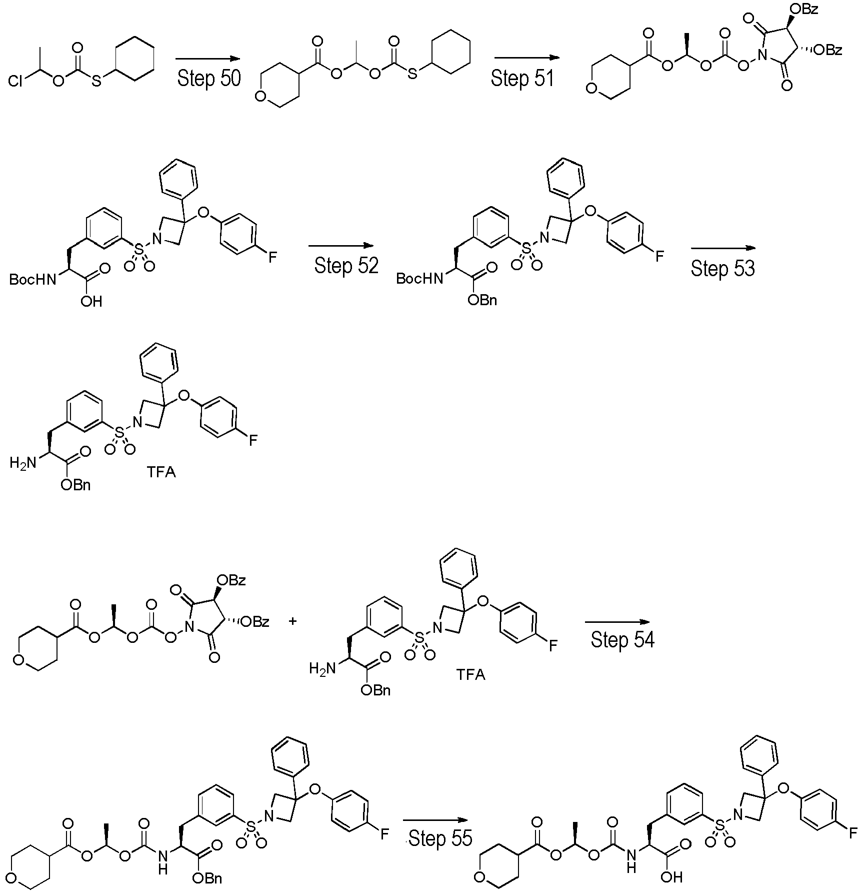

### [Step 50]

### 1-(((Cyclohexylthio)carbonyl)oxy)ethyl tetrahydro-2H-pyran-4-carboxylate

To a solution of a known compound O-(1-chloroethyl) S-cyclohexyl carbothioate (8.28 g) and tetrahydropyran-4-carboxylic acid (14.5 g) in toluene (8 ml), N,N-diisopropylethylamine (9.74 ml) was slowly added, and then the mixture was stirred at 75°C for 19 hours. The reaction solution was diluted with water, and the aqueous layer was extracted with diethyl ether. The organic layer was washed with a saturated aqueous sodium hydrogen carbonate solution and saturated saline, and dried over sodium sulfate. After the sodium sulfate was filtered off, the filtrate was concentrated under reduced pressure. The residue was purified by silica gel column chromatography (n-hexane/ethyl acetate) to obtain the title compound (8.39 g).
¹H-NMR (CDCl₃) δ: 1.20-1.48 (4H, m), 1.50 (3H, d, J = 5.5 Hz), 1.55-1.65 (2H, m), 1.67-1.89 (6H, m), 1.94-2.05 (2H, m), 2.51-2.61 (1H, m), 3.30-3.39 (1H, m), 3.39-3.47 (2H, m), 3.91-3.99 (2H, m), 6.93 (1H, q, J = 5.4 Hz).
MS (m/z) : 339 (M+Na)⁺.

### [Step 51]

### (3S,4S)-2,5-Dioxo-1-((((R)-1-(((tetrahydro-2H-pyran-4-carbonyl)oxy) ethoxy)carbonyl)oxy)pyrrolidin-3,4-diyl dibenzoate

Under an argon atmosphere, sulfuryl chloride (1.70 ml) was added dropwise under ice-cooling to a solution of the compound (4.53 g) obtained by the method of Step 50 in tetrahydrofuran (8 ml). After the mixture was stirred at the same temperature for 1 hour, a solution of a known compound (3S,4S)-1-hydroxy-2,5-dioxopyrrolidin-3,4-diyl dibenzoate (4.58 g) in tetrahydrofuran (8 ml) was added, followed by addition of N-methylmorpholine (3.15 ml) dropwise. The reaction solution was stirred under ice cooling for 2.5 hours, then the insoluble matters were filtered off. The filtrate was diluted with ethyl acetate, washed with water and saturated saline, and dried over sodium sulfate. After the sodium sulfate was filtered off, the filtrate was concentrated under reduced pressure. The residue was suspended in methanol and the precipitate was collected by filtration to obtain a diastereomeric mixture (3.43 g).

The obtained diastereomeric mixture (3.43 g) was suspended in ethyl acetate (10 ml) and dissolved by stirring at 50°C. n-Hexane (6 ml) was added thereto, and the mixture was stirred at room temperature for 4 hours, and the precipitate was collected by filtration. The obtained solid was suspended in ethyl acetate (4 ml) and dissolved by stirring at 50°C. n-Hexane (4 ml) was added thereto, and the mixture was stirred at room temperature for 3 hours, and the precipitate was collected by filtration to obtain the title compound (129 mg, 96.0% d.e.) .
¹H-NMR (CDCl₃) δ: 1.65 (3H, d, J = 5.3 Hz), 1.74-1.92 (4H, m), 2.57-2.67 (1H, m), 3.38-3.48 (2H, m), 3.94 (2H, tt, J = 12.2, 3.8 Hz), 6.00 (2H, br s), 6.91 (1H, q, J = 5.5 Hz), 7.46-7.52 (4H, m), 7.62-7.67 (2H, m), 8.07-8.11 (4H, m) .
MS (m/z) : 578 (M+Na)⁺.
CHIRALPAK ID, size: 0.46 cmφ x 25 cm, mobile layer: n-hexane/ethyl acetate = 70/30, flow rate: 1.0 ml/min, temperature: 30°C, wavelength: 254 nm, retention time: 7.2 min

### [Step 52]

### (S)-Benzyl 2-(tert-butoxycarbonyl)amino)-3-(3-((3-(4-fluorophenoxy)-3-phenylazetidin-1-yl)sulfonyl)phenyl)propanoate

To a solution of the compound (457 mg) obtained by the method of Step 29 in dimethylformamide (2 ml), 1-hydroxy-7-azabenzotriazole (109 mg), 1-ethyl-3-(3-dimethylaminopropyl)carbodiimide hydrochloride (307 mg), benzyl alcohol (0.824 ml), N,N-diisopropylethylamine (0.272 ml) were added, and the mixture was stirred for 2 hours. The reaction solution was extracted with ethyl acetate, and the organic layer was washed with a saturated aqueous sodium hydrogen carbonate solution and saturated saline, then dried over anhydrous sodium sulfate. The sodium sulfate was filtered off, and the filtrate was concentrated under reduced pressure. The residue was purified by silica gel column chromatography (n-hexane/ethyl acetate) to obtain the title compound (488 mg).
¹H-NMR (CDCl₃) δ: 1.41 (9H, s), 3.13 (1H, dd, J = 14.2, 5.4 Hz), 3.26 (1H, dd, J = 13.7, 5.6 Hz), 4.07-4.23 (4H, m), 4.60-4.69 (1H, m), 4.95-5.03 (1H, m), 5.13 (1H, d, J = 12.0 Hz), 5.17 (1H, d, J = 12.0 Hz), 6.32 (1H, d, J = 9.0 Hz), 6.33 (1H, d, J = 9.0 Hz), 6.79 (2H, dd, J = 9.2, 8.2 Hz), 7.19-7.45 (12H, m), 7.63 (1H, s), 7.72 (1H, d, J = 8.0 Hz) .
MS (m/z) : 605 (M-55)⁺.

### [Step 53]

### (S)-Benzyl 2-amino-3-(3-((3-(4-fluorophenoxy)-3-phenylazetidin-1-yl) sulfonyl)phenyl)propanoate trifluoroacetate

To a solution of the compound (153 mg) obtained by the method of Step 52 in dichloromethane (2.5 ml), trifluoroacetic acid (0.5 ml) was added. The mixture was stirred at room temperature for 1.5 hours, then concentrated under reduced pressure to obtain the title compound. The obtained compound was used in the next step without purification.

### [Step 54]

### (R)-1-((((S)-1-(Benzyloxy)-3-(3-((3-(4-fluorophenoxy)-3-phenylazetidin-1-yl)sulfonyl)phenyl)-1-oxopropan-2-yl)carbamoyl)oxy)ethyl tetrahydro-2H-pyran-4-carboxylate

To a mixed solution of the compound (157 mg) obtained by the method of Step 53 in tetrahydrofuran (2 ml)/water (1 ml), triethylamine (0.162 ml) and the compound obtained by the method of Step 51 (126 mg) were added, and the mixture was stirred at room temperature for 2 hours. To the reaction solution, a saturated aqueous potassium hydrogen sulfate solution was added, and then the mixture was diluted with ethyl acetate. The organic layer was washed with water and saturated saline sequentially, and dried over sodium sulfate. After the sodium sulfate was filtered off, the filtrate was concentrated under reduced pressure. The residue was purified by silica gel column chromatography two times (chloroform/ethyl acetate, n-hexane/ethyl acetate) to obtain the title compound (144 mg).
¹H-NMR (CDCl₃) δ: 1.43 (3H, d, J = 5.3 Hz), 1.68-1.85 (4H, m), 2.46-2.56 (1H, m), 3.16 (1H, dd, J = 13.7, 5.4 Hz), 3.27 (1H, dd, J = 13.8, 5.5 Hz), 3.35-3.45 (2H, m), 3.89-3.97 (2H, m), 4.08-4.23 (4H, m), 4.68 (1H, dd, J = 12.9, 5.6 Hz), 5.16 (2H, s), 5.27 (1H, d, J = 7.5 Hz), 6.28-6.36 (2H, m), 6.75-6.83 (3H, m), 7.24-7.45 (12H, m), 7.61 (1H, s), 7.72 (1H, d, J = 8.0 Hz).
MS (m/z) : 783 (M+Na)⁺.

### [Step 55]

### (S)-3-(3-((3-(4-Fluorophenoxy)-3-phenylazetidin-1-yl)sulfonyl)phenyl)-2-((((R)-1-((tetrahydro-2H-pyran-4-carbonyl)oxy)ethoxy)carbonyl)amino)propanoic acid

To a solution of the compound obtained by the method of Step 54 (144 mg) in methanol (2 ml), palladium carbon (38.1 mg) was added, and the mixture was stirred under a hydrogen atmosphere at room temperature for 1.5 hours. The insoluble matters were filtered off, and the filtrate was concentrated under reduced pressure. The residue was dissolved in ethyl acetate (1 ml), then dichloromethane (2 ml) and n-hexane (8 ml) were added thereto. The precipitate was collected by filtration to obtain the title compound (107 mg).
¹H-NMR (CDCl₃) δ: 1.45 (3H, d, J = 5.5 Hz), 1.69-1.86 (4H, m), 2.48-2.58 (1H, m), 3.22 (1H, dd, J = 13.9, 5.6 Hz), 3.32 (1H, dd, J = 13.9, 5.9 Hz), 3.37-3.46 (2H, m), 3.94 (2H, dq, J = 11.5, 3.5 Hz), 4.11 (1H, d, J = 8.7 Hz), 4.14 (1H, d, J = 8.8 Hz), 4.21 (1H, d, J = 8.8 Hz), 4.22 (1H, d, J = 9.0 Hz), 4.67 (1H, dd, J = 13.2, 5.9 Hz), 5.26 (1H, d, J = 7.8 Hz), 6.29-6.35 (2H, m), 6.76-6.85 (3H, m), 7.27-7.36 (5H, m), 7.49-7.57 (2H, m), 7.68 (1H, s), 7.77 (1H, dt, J = 6.8, 2.0Hz).
MS (m/z) : 693 (M+Na)⁺.

The same procedures as in Example 100 above were performed to obtain the compounds below.

**[Table 42]**

| Example | Name and Structure | Equipment data |
|---|---|---|
| 101 | (S)-3-(3-((3-(4-Fluorophenoxy)-3-phenylazetidin-1-yl)sulfonyl)phenyl)-2-((((S)-1-((tetrahydro-2H-pyran-4-carbonyl)oxy)ethoxy)carbonyl)amino)pr opanoic acid | ¹H-NMR (CDCl₃) δ: 1.45 (3H, d, J = 5.3 Hz), 1.72-1.91 (4H, m), 2.51-2.64 (1H, m), 3.25 (1H, dd, J = 13.7, 5.1 Hz), 3.35 (1H, dd, J = 14.3, 6.0 Hz), 3.45 (2H, t, J = 11.3 Hz), 3.91-4.02 (2H, m), 4.12 (1H, d, J = 8.8 Hz), 4.14 (1H, d, J = 9.0 Hz), 4.18-4.25 (2H, m), 4.62-4.71 (1H, m), 5.23-5.32 (1H, m), 6.28-6.35 (2H, m), 6.75-6.86 (3H, m), 7.23-7.37 (5H, m), 7.49-7.57 (2H, m), 7.66 (1H, s), 7.73-7.81 (1H, m). |
| | 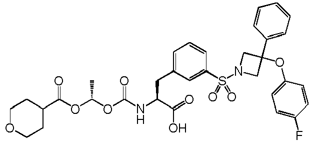 | |
| | | MS (m/z): 693 (M+Na)⁺. |
| 102 | (S)-3-(3-((3-(4-Fluorophenoxy)-3-phenylazetidin-1-yl)sulfonyl)phenyl)-2-((((R)-1-((3-methylbutanoyl)oxy)ethoxy)carbonyl)a mino)propanoic acid | ¹H-NMR (CDCl₃) δ: 0.94 (6H, d, J = 6.5 Hz), 1.44 (3H, d, J = 5.5 Hz), 2.04-2.13 (1H, m), 2.15-2.20 (2H, m), 3.21 (1H, dd, J = 14.1, 6.3 Hz), 3.33 (1H, dd, J = 14.2, 5.9 Hz), 4.11 (1H, d, J = 9.5 Hz), 4.14 (1H, d, J = 9.5 Hz), 4.20 (1H, d, J = 8.9 Hz), 4.22 (1H, d, J = 8.9 Hz), 4.67 (1H, dd, J = 13.3, 6.0 Hz), 5.25 (1H, d, J = 7.5 Hz), 6.28-6.35 (2H, m), 6.76-6.85 (3H, m), 7.27-7.36 (5H, m), 7.49-7.56 (2H, m), 7.68 (1H, s), 7.76 (1H, dt, J = 6.4, 2.0 Hz). |
| | 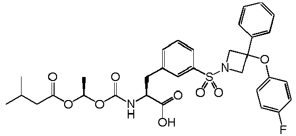 | MS (m/z): 665 (M+Na)⁺. |

### [Example 103]

### (S)-3-(3-((3-(4-Fluorophenoxy)-3-phenylazetidin-1-yl)sulfonyl)phenyl)-2-((((S)-1-(isobutyryloxy)ethoxy)carbonyl)amino)propanoic acid

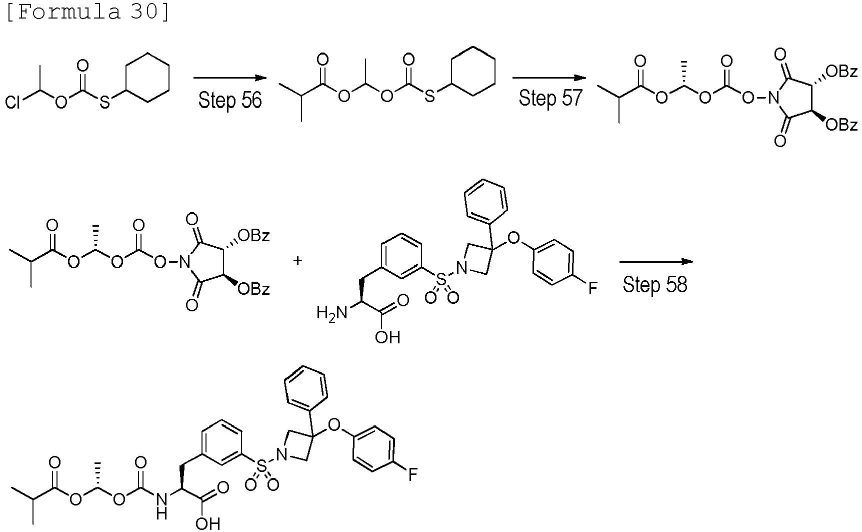

### [Step 56]

### 1-(((Cyclohexylthio)carbonyl)oxy)ethyl isobutyrate

To a solution of N,N-diisopropylethylamine (23.5 ml) in isobutyric acid (8 ml), a solution of a known compound O-(1-chloroethyl)S-cyclohexyl carbothioate (20 g) in isobutyric acid (8 ml) was added dropwise and the mixture was stirred at 75°C for 20 hours. The reaction solution was diluted with ethyl acetate, and washed with saturated aqueous sodium hydrogen carbonate solution and saturated saline sequentially. The organic layer was dried over sodium sulfate, then the sodium sulfate was filtered off, and the filtrate was concentrated under reduced pressure. The obtained residue was purified by silica gel column chromatography (n-hexane/ethyl acetate) to obtain the title compound (19.6 g).
¹H-NMR (CDCl₃) δ: 1.17 (3H, d, J = 7.0 Hz), 1.17 (3H, d, J = 7.0 Hz), 1.23-1.47 (5H, m), 1.49 (3H, d, J = 5.5 Hz), 1.53-1.63 (1H, m), 1.67-1.76 (2H, m), 1.97-2.05 (2H, m), 2.50-2.60 (1H, m), 3.32-3.38 (1H, m), 6.92 (1H, q, J = 5.5 Hz).
MS (m/z) : 297 (M+Na)⁺.

### [Step 57]

### (3R,4R)-1-((((S) - 1(Isobutyryloxy)ethoxy)carbonyl)oxy)-2,5-dioxopyrrolidin-3,4-diyl dibenzoate

To a solution of the compound obtained by the method of Step 56 (20 g) in tetrahydrofuran (70 ml), sulfuryl chloride (8.89 ml) was added under ice-cooling, and the mixture was stirred for 30 minutes. Furthermore, N-methylmorpholine (16.0 ml) and a known compound (3R,4R)-1-hydroxy-2,5-dioxopyrrolidin-3,4-diyl dibenzoate (25.9 g) were added under ice-cooling, and the mixture was stirred for 2 hours. Water was added to the reaction solution, and the mixture was extracted with ethyl acetate, then the combined organic layer was washed with saturated saline, and dried over sodium sulfate. To the concentrated residue, n-hexane/ethyl acetate was added, and the precipitated solid was collected by filtration to obtain a stereoisomeric mixture (13.1 g, about 30% isomer content). To the obtained mixture, ethyl acetate (52 ml) was added, and the mixture was warmed to 80°C to dissolve, and then n-hexane (78 ml) was added thereto. The mixture was left to cool to room temperature. The resulting solid (8 g, about 3% isomer content) was collected by filtration. To the obtained solid (7.23 g), ethyl acetate was added, and the mixture was warmed to 80°C to dissolve, and then n-hexane (45 ml) was slowly added thereto. The mixture was left to cool to room temperature, then the precipitated solid was collected by filtration, washed with n-hexane/ethyl acetate, then dried to obtain the title compound (5.81 g).
¹H-NMR (CDCl₃) δ: 1.20 (3H, d, J = 7.0 Hz), 1.20 (3H, d, J = 7.0 Hz), 1.65 (3H, d, J = 5.3 Hz), 2.55-2.67 (1H, m), 6.02 (2H, br s), 6.89 (1H, q, J = 5.4 Hz), 7.45-7.52 (4H, m), 7.61-7.67 (2H, m), 8.07-8.12 (4H, m).

### [Step 58]

### (S)-3-(3-((3-(4-Fluorophenoxy)-3-phenylazetidin-1-yl)sulfonyl)phenyl)-2-((((S)-1-(isobutyryloxy)ethoxy)carbonyl)amino)propanoic acid

To a solution of the compound of Example 41 (300 mg) in tetrahydrofuran (6 ml) and water (3 ml), triethylamine (0.444 ml) and the compound obtained by the method of Step 57 (327 mg) were added sequentially, and the mixture was stirred for 2 hours. To the reaction solution, an aqueous 0.5 N hydrochloric acid solution was added, and the mixture was extracted with ethyl acetate. The organic layer was washed with saturated saline, and then dried over anhydrous sodium sulfate. The sodium sulfate was filtered off, and the solvent was distilled off under reduced pressure. The obtained residue was purified by silica gel column chromatography (chloroform/ethyl acetate) to obtain the title compound (116 mg).
¹H-NMR (CDCl₃) δ: 1.19 (6H, d, J = 7.0 Hz), 1.45 (3H, d, J = 5.5 Hz), 2.51-2.62 (1H, m), 3.25 (1H, dd, J = 13.9, 4.9 Hz), 3.35 (1H, dd, J = 14.1, 6.0 Hz), 4.12 (1H, d, J = 8.7 Hz), 4.14 (1H, d, J = 8.5 Hz), 4.20 (1H, d, J = 9.0 Hz), 4.21 (1H, d, J = 9.0 Hz), 4.68 (1H, q, J = 6.3 Hz), 5.23 (1H, d, J = 7.3 Hz), 6.28-6.35 (2H, m), 6.75-6.84 (3H, m), 7.27-7.36 (5H, m), 7.49-7.56 (2H, m), 7.67 (1H, s), 7.74-7.79 (1H, m).
MS (m/z) : 651 (M+Na)⁺.

### [Example 104]

### (S)-3-(3-((3-(4-Fluorophenoxy)-3-phenylazetidin-1-yl)sulfonyl)phenyl)-2-((((R)-1-(isobutyryloxy)ethoxy)carbonyl)amino)propanoic acid

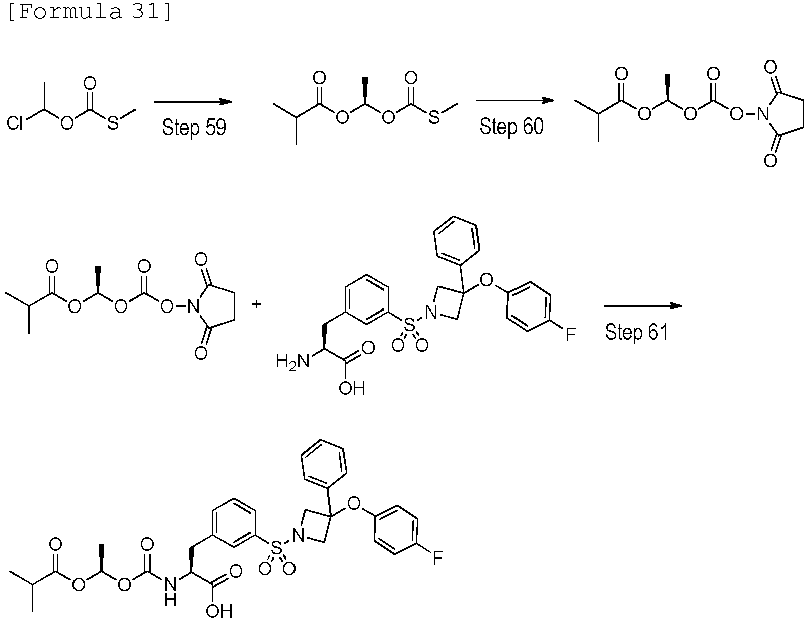

### [Step 59]

### 1-(((((Methylthio)carbonyl)oxy)ethyl isobutyrate

To a solution of N,N-diisopropylethylamine (100 ml) in isobutyric acid (35 ml), a solution of a known compound O-(1-chloroethyl)S-methylcarbothioate (59 g) in isobutyric acid (35 ml) was added dropwise, and the mixture was stirred at 55°C for 12 hours, and then at 80°C for 5 hours. The reaction solution was diluted with n-hexane/ethyl acetate, and washed with a 10% aqueous potassium hydrogen sulfate solution, water, saturated aqueous sodium hydrogen carbonate solution, and saturated saline sequentially. The organic layer was dried over sodium sulfate, then the sodium sulfate was filtered off, and the filtrate was concentrated. The obtained residue (73.3 g) was dissolved in a PBS buffer, and Novozyme 435 (5 g) was added, and the mixture was stirred at room temperature for 20 hours. The reaction solution was diluted with n-hexane/ethyl acetate of 1/1 (about 500 ml), and stirred for 10 minutes, then the solid was filtered off using a high flow supercell. The organic layer of the filtrate was washed with saturated saline, dried over sodium sulfate, then the sodium sulfate was filtered off, and the filtrate was concentrated. The concentrate was used as was, in the next step.
¹H-NMR (CDCl₃) δ: 1.17 (3H, d, J = 7.0 Hz), 1.18 (3H, d, J = 7.0 Hz), 1.51 (3H, d, J = 5.3 Hz), 2.34 (3H, s), 2.50-2.61 (1H, m), 6.94 (1H, q, J = 5.4 Hz).

### [Step 60]

### (R)-1-((((2,5-Dioxopyrrolidin-1-yl)oxy)carbonyl)oxy)ethyl isobutyrate

To a solution of the compound (22.1 g) obtained by the method of Step 59 in dichloromethane (230 ml), N-hydroxysuccinimide (14.9 g) and magnesium monoperoxyphthalate hexahydrate (188 g) were added, and the mixture was stirred at room temperature for 1 hour and 45 minutes. The reaction solution was stirred under ice-cooling for 10 minutes due to heat production, and then the mixture was returned to room temperature and the mixture was stirred for 17 hours. To the reaction solution, dichloromethane (300 ml) was added, and the insoluble matters were filtered off and washed with dichloromethane. The filtrate was washed with water, an aqueous 20% potassium carbonate solution, and saturated saline sequentially, and dried over sodium sulfate. The sodium sulfate was then filtered off, and the solvent was distilled off under reduced pressure. The residue was dissolved in dichloromethane again due to the remaining of impurities, and an aqueous 20% potassium carbonate solution was added thereto. The mixture was stirred for 15 minutes, then the organic layer was fractionated. The organic layer was washed with saturated saline, and dried over sodium sulfate. The sodium sulfate was filtered off and the solvent was distilled off under reduced pressure to obtain the title compound (13.0 g).
¹H-NMR (CDCl₃) δ: 1.18 (3H, d, J = 7.0 Hz), 1.19 (3H, d, J = 7.0 Hz), 1.61 (3H, d, J = 5.5 Hz), 2.53-2.65 (1H, m), 2.83 (4H, s), 6.84 (1H, q, J = 5.4 Hz).

### [Step 61]

### (S)-3-(3-((3-(4-Fluorophenoxy)-3-phenylazetidin-1-yl)sulfonyl)phenyl)-2-((((R)-1-(isobutyryloxy)ethoxy)carbonyl)amino)propanoic acid

To a suspension of the compound of Example 41 (4.50 g) in tetrahydrofuran (50 ml) and water (25 ml), the compound (2.87 g) obtained by the method of Step 60 and triethylamine (4 ml) were added sequentially, and the reaction solution was stirred at room temperature for 5 hours. To the reaction solution, a saturated aqueous potassium hydrogen sulfate solution and water were added, and the mixture was extracted with ethyl acetate. The organic layer was washed with water and saturated saline, and dried over sodium sulfate. The sodium sulfate was filtered off, and the solvent was distilled off under reduced pressure. To the obtained residue, a mixed solvent of n-hexane/ethyl acetate (1/1) was added to form a suspension, and then the insoluble matters were collected by filtration. The obtained solid was purified by silica gel column chromatography (chloroform/methanol). To the obtained solid, a mixed solvent of n-hexane/ethyl acetate (3/1) was added to form a suspension, and then the insoluble matters were collected by filtration to obtain the title compound (3.51 g).
¹H-NMR (CDCl₃) δ: 1.14 (3H, d, J = 7.0 Hz), 1.15 (3H, d, J = 7.0 Hz), 1.44 (3H, d, J = 5.3 Hz), 2.44-2.58 (1H, m), 3.21 (1H, dd, J = 13.8, 6.0 Hz), 3.33 (1H, dd, J = 13.9, 5.9 Hz), 4.12 (1H, d, J = 9.0 Hz), 4.14 (1H, d, J = 9.0 Hz), 4.20 (1H, d, J = 9.0 Hz), 4.21 (1H, d, J = 9.0 Hz), 4.61-4.70 (1H, m), 5.28 (1H, br d, J = 7.5 Hz), 6.27-6.35 (2H, m), 6.75-6.83 (3H, m), 7.25-7.36 (5H, m), 7.48-7.56 (2H, m), 7.68 (1H, br s), 7.72-7.79 (1H, m).
MS (m/z) : 651 (M+Na)⁺.

The same procedures as in Example 104 above were performed to obtain the compound below. For the raw material of Step 61, the corresponding Example compound at the right of the following table was used.

**[Table 43]**

| Example | Name and Structure | Equipment data | Raw material compound of Step 61 |
|---|---|---|---|
| 105 | (S)-3-(3-((3-(3-Fluorophenoxy)-3-phenylazetidin-1-yl)sulfonyl)phenyl)-2-((((R)-1-(isobutyryloxy)ethoxy)carbonyl )amino)propanoic acid | ¹H-NMR (CDCl₃) δ: 1.15 (6H, d, J = 7.0 Hz), 1.44 (3H, d, J = 5.3 Hz), 2.52 (1H, dt, J = 13.8, 6.8 Hz), 3.21 (1H, dd, J = 14.2, 5.9 Hz), 3.33 (1H, dd, J = 14.2, 5.4 Hz), 4.15 (2H, d, J = 9.4 Hz), 4.22 (1H, d, J = 9.0 Hz), 4.24 (1H, d, J = 9.0 Hz), 4.67 (1H, q, J = 6.1 Hz), 5.24 (1H, d, J = 7.5 Hz), 6.09-6.16 (2H, m), 6.56-6.63 (1H, m), 6.80 (1H, q, J = 5.4 Hz), 6.99-7.09 (1H, m), 7.27-7.36 (5H, m), 7.50-7.57 (2H, m), 7.68 (1H, s), 7.74-7.79 (1H, m). | Example 42 |
| | 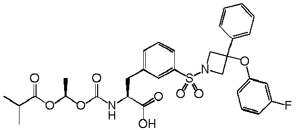 | | |
| | | MS (m/z) : 652 (M+Na)⁺. | |

### [Example 106]

### (S)-3-(3-((3-(3-Fluorophenoxy)-3-phenylazetidin-1-yl)sulfonyl)phenyl)-2-((((S)-1-(isobutyloxy)ethoxy)carbonyl)amino)propanoic acid hemipiperazine salt

The same procedures as in Example 103 were performed to obtain the compound below. However, after Step 58, the procedure of Step 49 of Example 96 was performed. For the raw material of Step 58, the corresponding Example compound at the right of the following table was used.

**[Table 44]**

| Example | Name and Structure | Equipment data | Raw material compound of Step 58 |
|---|---|---|---|
| 106 | (S)-3-(3-((3-(3-Fluorophenoxy)-3-phenylazetidin-1-yl)sulfonyl)phenyl)-2-((((S)-1-(isobutyloxy)ethoxy)carbonyl)a mino)propanoic acid hemipiperazine salt | ¹H-NMR (CDCl₃) δ: 1.20 (3H, d, J = 6.8 Hz), 1.20 (3H, d, J = 6.8 Hz), 1.43 (3H, d, J = 5.3 Hz), 2.57 (1H, dt, J = 13.8, 6.9 Hz), 3.11 (4H, s), 3.23 (1H, dd, J = 13.6, 4.0 Hz), 3.35 (1H, dd, J = 13.4, 5.4 Hz), 4.14 (2H, d, J = 9.0 Hz), 4.20 (2H, d, J = 9.3 Hz), 4.42 (1H, q, J = 4.5 Hz), 5.52 (1H, d, J = 6.0 Hz), 6.07-6.14 (2H, m), 6.55-6.62 (1H, m), 6.83 (1H, q, J = 5.4 Hz), 6.99-7.08 (1H, m), 7.22-7.34 (5H, m), 7.48 (1H, t, J = 7.7 Hz), 7.55 (1H, d, J = 8.0 Hz), 7.65-7.71 (2H, m). | Example 42 |
| | 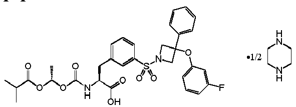 | | |
| | | MS (m/z): 651 (M+Na)⁺. | |

### [Example 107]

### (S)-Ethyl 2-amino-3-(3-((3-(4-fluorophenoxy)-3-phenylazetidin-1-yl)sulfonyl)phenyl)propanoate

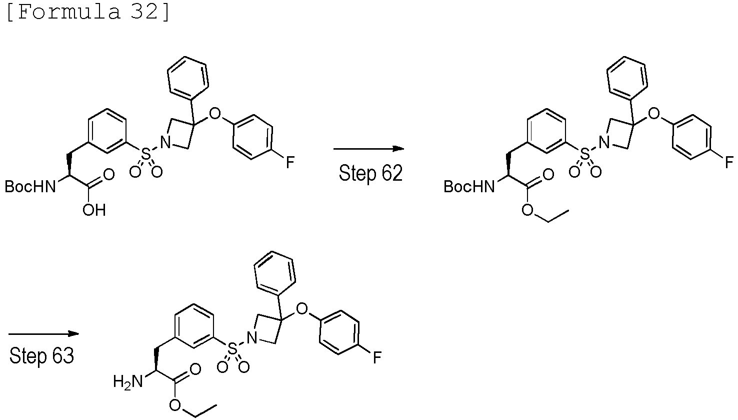

### [Step 62]

### (S)-Ethyl 2-((tert-butoxycarbonyl)amino)-3-(3-((3-(4-fluorophenoxy)-3-phenylazetidin-1-yl)sulfonyl)phenyl)propanoate

To a solution of the compound obtained by the method of Step 29 (100 mg) in N,N-dimethylformamide (1 ml), ethanol (0.102 ml), 1-ethyl-3-(3-dimethylaminopropyl)carbodiimide hydrochloride (67.2 mg) and 1-hydroxy-7-azabenzotriazole (47.7 mg) were added, and the mixture was stirred at room temperature for 3 hours. To the reaction solution, 1 N hydrochloric acid was added, and the reaction solution was extracted with ethyl acetate. The organic layer was washed with saturated saline, and dried over sodium sulfate, then the sodium sulfate was filtered off, and the filtrate was concentrated under reduced pressure. The residue was purified by silica gel column chromatography (n-hexane/ethyl acetate) to obtain the title compound (97 mg) .
¹H-NMR (CDCl₃) δ: 1.26 (3H, t, J = 7.2 Hz), 1.42 (9H, s), 3.14 (1H, dd, J = 13.6, 5.0 Hz), 3.26 (1H, dd, J = 14.1, 5.5 Hz), 4.12-4.23 (6H, m), 4.54-4.63 (1H, m), 5.01 (1H, d, J = 7.3 Hz), 6.30-6.36 (2H, m), 6.76-6.83 (2H, m), 7.28-7.36 (5H, m), 7.45-7.54 (2H, m), 7.64 (1H, br s), 7.72-7.77 (1H, m).
MS (m/z) : 521 (M+Na)⁺.

### [Step 63]

### (S)-Ethyl 2-amino-3-(3-((3-(4-fluorophenoxy)-3-phenylazetidin-1-yl) sulfonyl)phenyl)propanoate

To a solution of the compound obtained by the method of Step 62 (97 mg) in dichloromethane (1 ml), trifluoroacetic acid (1 ml) was added, and the mixture was stirred for 10 minutes. The residue obtained by concentrating the reaction solution was dissolved in ethyl acetate, and washed with 1 N aqueous sodium hydroxide solution and saturated saline sequentially, and then dried over sodium sulfate. The sodium sulfate was filtered off and the filtrate was concentrated to obtain the title compound (79 mg).
¹H-NMR (CDCl₃) δ: 1.26 (3H, t, J = 7.2 Hz), 2.94 (1H, dd, J = 13.6, 7.8 Hz), 3.13 (1H, dd, J = 13.7, 5.1 Hz), 3.67 (1H, dd, J = 7.5, 5.3 Hz), 4.12-4.23 (6H, m), 6.28-6.34 (2H, m), 6.76-6.83 (2H, m), 7.28-7.34 (5H, m), 7.49-7.57 (2H, m), 7.71-7.77 (2H, m).
MS (m/z) : 499 (M+H)⁺.

### [Example 108]

### (S)-Isopropyl 2-amino-3-(3-((3-(4-fluorophenoxy)-3-phenylazetidin-1-yl) sulfonyl)phenyl)propanoate trifluoroacetate

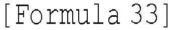

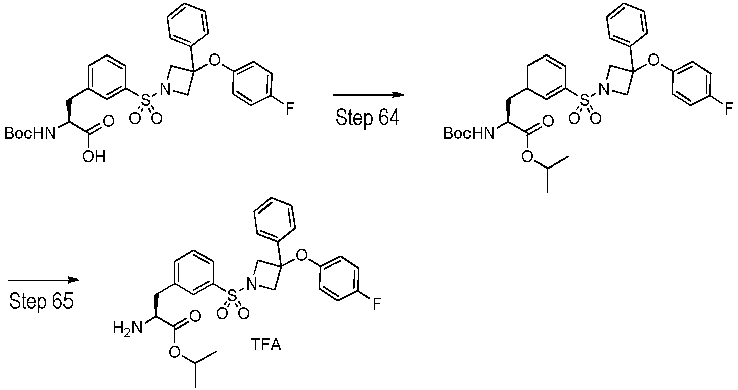

### [Step 64]

### (S)-Isopropyl 2-(tert-butoxycarbonyl)amino)-3-(3-((3-(4-fluorophenoxy)-3-phenylazetidin-1-yl)sulfonyl)phenyl)propanoate

To a solution of the compound obtained by the method of Step 29 (100 mg) in N,N-dimethylformamide (1 ml), isopropyl alcohol (0.135 ml), 1-ethyl-3-(3-dimethylaminopropyl)carbodiimide hydrochloride (67.2 mg) and 1-hydroxy-7-azabenzotriazole (47.7 mg) were added, and the mixture was stirred at room temperature for 3 hours. To the reaction solution, 1 N hydrochloric acid was added, and the mixture was extracted with ethyl acetate. The organic layer was washed with saturated saline, and dried over sodium sulfate, then the sodium sulfate was filtered off, and the filtrate was concentrated under reduced pressure. The residue was purified by silica gel column chromatography (n-hexane/ethyl acetate) to obtain the title compound (57 mg).
¹H-NMR (CDCl₃) δ: 1.23 (3H, d, J = 6.0 Hz), 1.24 (3H, d, J = 6.3 Hz), 1.42 (9H, s), 3.09-3.17 (1H, m), 3.21-3.30 (1H, m), 4.14 (1H, d, J = 9.0 Hz), 4.15 (1H, d, J = 8.5 Hz), 4.19 (1H, d, J = 8.8 Hz), 4.20 (1H, d, J = 9.0 Hz), 4.48-4.59 (1H, m), 4.97-5.05 (2H, m), 6.31-6.36 (2H, m), 6.77-6.83 (2H, m), 7.28-7.36 (6H, m), 7.45-7.53 (2H, m), 7.65 (1H, br s), 7.72-7.76 (1H, m).
MS (m/z) : 635 (M+Na)⁺.

### [Step 65]

### (S)-Isopropyl 2-amino-3-(3-((3-(4-fluorophenoxy)-3-phenylazetidin-1-yl) sulfonyl)phenyl)propanoate trifluoroacetate

To a solution of the compound obtained by the method of Step 64 (57 mg) in dichloromethane (0.6 ml), trifluoroacetic acid (0.6 ml) was added, and the mixture was stirred for 10 minutes. The reaction solution was concentrated and dried under reduced pressure to obtain the title compound (58 mg).
¹H-NMR (CDCl₃) δ: 1.24 (6H, d, J = 6.3 Hz), 3.31 (1H, dd, J = 14.6, 7.8 Hz), 3.38 (1H, dd, J = 14.3, 5.8 Hz), 4.12 (1H, d, J = 9.0 Hz), 4.13 (1H, d, J = 9.0 Hz), 4.17-4.26 (3H, m), 5.01-5.12 (1H, m), 6.26-6.33 (2H, m), 6.75-6.82 (2H, m), 7.27-7.35 (5H, m), 7.52-7.63 (2H, m), 7.73-7.80 (2H, m).
MS (m/z) : 513 (M+H)⁺.

The same procedures as in Example 108 were performed to obtain the compounds below.

**[Table 45]**

| Example | Name and Structure | Equipment data |
|---|---|---|
| 109 | 2-Morpholinoethyl (S)-2-amino-3-(3-((3 -(4-fluorophenoxy)-3 - phenylazetidin-1-yl)sulfonyl)phenyl)propanoate bis(trifluoroacetate) | ¹H-NMR (CDCl₃) δ: 2.77-3.05 (2H, br), 3.30-3.42 (4H, m), 3.45-3.70 (2H, br), 3.82-4.05 (4H, br), 4.10-4.17 (2H, m), 4.21 (2H, d, J = 9.3 Hz), 4.33-4.45 (2H, m), 4.50-4.60 (1H, m), 6.25-6.33 (2H, m), 6.75-6.82 (2H, m), 7.26-7.35 (5H, m), 7.54-7.59 (1H, m), 7.63-7.68 (1H, m), 7.68-7.71 (1H, m), 7.74-7.79 (1H, m). |
| | 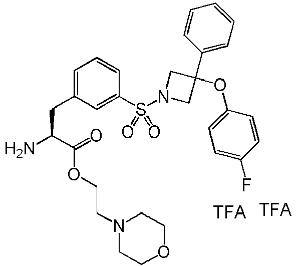 | |
| | | MS (m/z) : 584 (M+H)⁺. |
| 110 | 2-Dimethylaminoethyl (S)-2-amino-3-(3 -((3 -(4-fluorophenoxy)-3 - phenylazetidin-1-yl)sulfonyl)phenyl)propanoate bis(trifluoroacetate) | ¹H-NMR (CDCl₃) δ: 2.85 (3H, br s), 2.87 (3H, br s), 3.30-3.39 (2H, m), 3.39-3.46 (2H, m), 4.08-4.16 (2H, m), 4.21 (2H, d, J = 9.3 Hz), 4.35-4.45 (2H, m), 4.53-4.62 (1H, m), 6.25-6.33 (2H, m), 6.74-6.82 (2H, m), 7.26-7.35 (5H, m), 7.53-7.59 (1H, m), 7.62-7.69 (2H, m), 7.74-7.79 (1H, m). |
| | 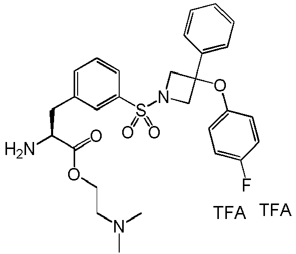 | |
| | | MS (m/z) : 542 (M+H)⁺. |

### [Example 111]

### (2S)-2-(Dimethylamino)ethyl 3-(3-((3-(4-fluorophenoxy)-3-phenylazetidin-1-yl)sulfonyl)phenyl)-2-(((1-(pivaloyloxy)ethoxy)carbonyl)amino)propanoate

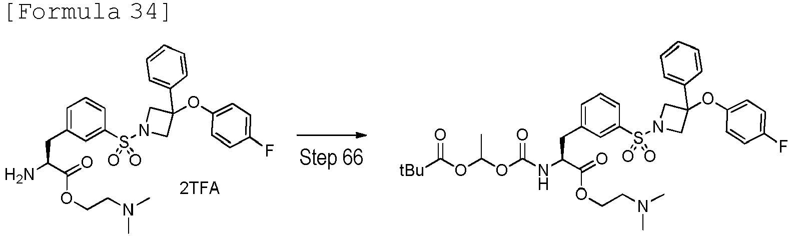

### [Step 66]

### (2S)-2-(Dimethylamino)ethyl 3-(3-((3-(4-fluorophenoxy)-3-phenylazetidin-1-yl)sulfonyl)phenyl)-2-(((1-(pivaloyloxy)ethoxy)carbonyl)amino)propanoate

To a solution of the compound (92 mg) of Example 110 in dichloromethane (1 ml), the compound (74.7 mg) obtained by the method of Step 41 and N,N-diisopropylethylamine (0.204 ml) were added, and the mixture was stirred at room temperature for 2 days. The reaction solution was purified by basic silica gel column chromatography (n-hexane/ethyl acetate) to obtain the title compound (33 mg).
¹H-NMR (CDCl₃) δ: 1.20 (9H, s), 1.40-1.48 (3H, m), 2.26 (6H, d, J = 2.5 Hz), 2.56 (2H, t, J = 5.6 Hz), 3.15-3.35 (2H, m), 4.07-4.29 (6H, m), 4.64-4.72 (1H, m), 5.28-5.37 (1H, m), 6.32 (1H, d, J = 9.0 Hz), 6.34 (1H, d, J = 9.0 Hz), 6.73-6.84 (3H, m), 7.26-7.37 (5H, m), 7.48-7.56 (2H, m), 7.61 (1H, d, J = 9.8 Hz), 7.75 (1H, d, J = 5.8 Hz). MS (m/z) : 714 (M+H)⁺.

The same procedures as in Example 111 were performed to obtain the compound below. For the raw material of Step 66, the corresponding Example compound at the right of the following table were used.

**[Table 46]**

| Example | Name and Structure | Equipment data | Raw material compound of Step 66 |
|---|---|---|---|
| 112 | 1-((((S)-3-(3-((3-(4-Fluorophenoxy)-3-phenylazetidin-1-yl)sulfonyl)phenyl)-1-(2-morpholinoethoxy)-1-oxopropan-2-yl)carbamoyl)oxy)ethyl pivalate | ¹H-NMR (CDCl₃) δ: 1.18, 1.22 (9H, s x2), 1.40-1.48 (3H, m), 2.42-2.52 (4H, m), 2.61 (2H, t, J = 5.8 Hz), 3.14-3.25 (1H, m), 3.26-3.34 (1H, m), 3.65-3.72 (4H, m), 4.10-4.29 (6H, m), 4.61-4.70 (1H, m), 5.22-5.31 (1H, m), 6.29-6.37 (2H, m), 6.73-6.83 (3H, m), 7.27-7.37 (5H, m), 7.46-7.55 (2H, m), 7.56-7.63 (1H, m), 7.72-7.79 (1H, m). | Example 109 |
| | 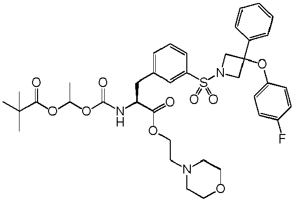 | | |
| | | MS (m/z) : 756 (M+H)⁺. | |

### [Example 113]

### 1-((((S)-1-Ethoxy-3-(3-((3-(4-fluorophenoxy)-3-phenylazetidin-1-yl)sulfonyl)phenyl)-1-oxopropan-2-yl)carbonyl)oxy)ethyl pivalate

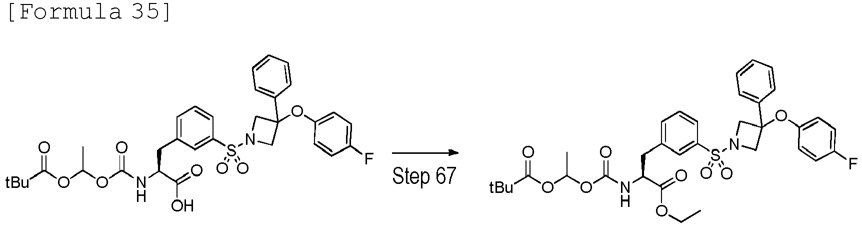

### [Step 67]

### 1-(((1-((((S)-1-Ethoxy-3-(3-((3-(4-fluorophenoxy)-3-phenylazetidin-1-yl)sulfonyl)phenyl)-1-oxopropan-2-yl)carbonyl)oxy)ethyl pivalate

To a solution of the compound of Example 44 (29.0 mg) in N,N-dimethylformamide (1 ml), ethanol (0.026 ml), 1-ethyl-3-(3-dimethylaminopropyl)carbodiimide hydrochloride (17.3 mg) and 1-hydroxy-7-azabenzotriazole (12.3 mg) were added, and the mixture was stirred at room temperature for 2 hours. The reaction solution was diluted with water, and then extracted with ethyl acetate. The organic layer was washed with saturated saline, and dried over sodium sulfate. After the sodium sulfate was filtered off, the filtrate was concentrated under reduced pressure. The residue was purified by silica gel column chromatography (chloroform/methanol) to obtain the title compound (28.1 mg).
¹H-NMR (CDCl₃) δ: 1.20 (9H, d, J = 16.8 Hz), 1.26 (3H, t, J = 7.3 Hz), 1.44 (3H, dd, J = 7.8, 5.5 Hz), 3.19 (1H, td, J = 13.7, 5.0 Hz), 3.29 (1H, dt, J = 13.9, 5.9 Hz), 4.10-4.23 (6H, m), 4.59-4.67 (1H, m), 5.27 (1H, t, J = 8.0 Hz), 6.29-6.37 (2H, m), 6.75-6.84 (3H, m), 7.27-7.36 (5H, m), 7.43-7.54 (2H, m), 7.61 (1H, d, J = 11.0 Hz), 7.72-7.78 (1H, m).
MS (m/z) : 693 (M+Na)⁺.

The same procedures as in Example 113 were performed to obtain the compound below.

**[Table 47]**

| Example | Name and Structure | Equipment data |
|---|---|---|
| 114 | 1-((((S)-3-(3-((3-(4-Fluorophenoxy)-3-phenylazetidin-1-yl)sulfonyl)phenyl)-1-((5-methyl-2-oxo-1,3 -dioxol-4-yl)methoxy)-1-oxopropan-2-yl)carbamoyl)oxy)ethyl pivalate | ¹H-NMR (CDCl₃) δ: 1.18 (4.5H, s), 1.21 (4.5H, s), 1.44 (1.5H, d, J = 5.5 Hz), 1.46 (1.5H, d, J = 5.5 Hz), 2.15 (1.5H, s), 2.15 (1.5H, s), 3.15-3.32 (2H, m), 4.10-4.18 (2H, m), 4.19-4.25 (2H, m), 4.61-4.74 (1H, m), 4.81-4.92 (2H, m), 5.16-5.23 (1H, m), 6.29-6.34 (2H, m), 6.74-6.83 (3H, m), 7.28-7.34 (5H, m), 7.40-7.61 (3H, m), 7.75-7.79 (1H, m). |
| | 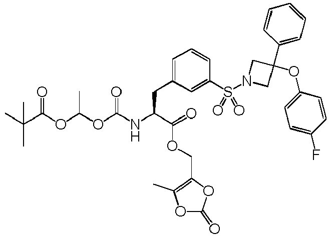 | |
| | | MS (m/z) : 777 (M+Na)⁺. |

### [Example 115]

### (S)-1-((((S)-3-(3-((3-(4-Fluorophenoxy)-3-phenylazetidin-1-yl)sulfonyl)phenyl)-1-(2-hydroxyethoxy)-1-oxopropan-2-yl)carbamoyl)oxy)ethyl pivalate

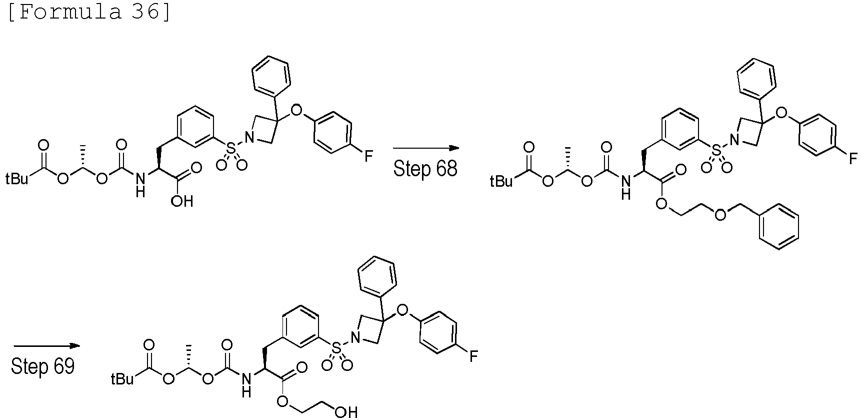

### [Step 68]

### (S)-2-(Benzyloxy)ethyl 3-(3-((3-(4-fluorophenoxy)-3-phenylazetidin-1-yl)sulfonyl)phenyl)-2-((((S)-1-(pivaloyloxy)ethoxy)carbonyl)amino)propanoate

To a solution of the compound of Example 93 (126 mg) in dichloromethane (2 ml), 2-(benzyloxy)ethanol (42 µl), 1-[bis(dimethylamino)methylene]-1H-1,2,3-triazolo[4,5-b]pyridinium 3-oxide hexafluorophosphate (114 mg) and N,N-diisopropylethylamine (51 µl) were added, and the mixture was stirred at room temperature for 6 hours and 30 minutes. The reaction solution was diluted with ethyl acetate, and then washed with a saturated aqueous potassium hydrogen sulfate solution, water, and saturated saline sequentially. After the organic layer was dried over anhydrous sodium sulfate, the sodium sulfate was filtered off, and the solvent was distilled off under reduced pressure. The obtained residue was purified by silica gel column chromatography (n-hexane/ethyl acetate) to obtain the title compound (125 mg).
¹H-NMR (CDCl₃) δ: 1.22 (9H, s), 1.44 (3H, d, J = 5.5 Hz), 3.21 (1H, dd, J = 14.1, 4.5 Hz), 3.31 (1H, dd, J = 13.8, 5.8 Hz), 3.67 (2H, t, J = 4.6 Hz), 4.09-4.21 (4H, m), 4.23-4.38 (2H, m), 4.54 (1H, d, J = 12.0 Hz), 4.57 (1H, d, J = 12.0 Hz), 4.64-4.73 (1H, m), 5.23 (1H, br d, J = 7.3 Hz), 6.28-6.35 (2H, m), 6.74-6.83 (3H, m), 7.25-7.37 (10H, m), 7.44 (1H, t, J = 7.7 Hz), 7.48-7.53 (1H, m), 7.60 (1H, br s), 7.70-7.76 (1H, m).
MS (m/z) : 799 (M+Na)⁺.

### [Step 69]

### (S)-1-((((S)-3-(3-((3-(4-Fluorophenoxy)-3-phenylazetidin-1-yl)sulfonyl)phenyl)-1-(2-hydroxyethoxy)-1-oxopropan-2-yl)carbamoyl)oxy)ethyl pivalate

To a solution of the compound (123 mg) obtained by the method of Step 68 in methanol (2 ml), 10% palladium carbon (50.5 mg) was added, and the replacement with hydrogen was performed. The reaction solution was stirred under a hydrogen atmosphere at room temperature for 5 hours. To the reaction solution, 10% palladium carbon (55.8 mg) was further added, then the mixture was stirred at room temperature under a hydrogen atmosphere for 2 hours. Furthermore, 10% palladium carbon (51.4 mg) was added to the reaction solution, then the reaction solution was stirred at room temperature under a hydrogen atmosphere for 2 hours. The reaction solution was filtered through Celite, and then the solvent was distilled off under reduced pressure. The obtained residue was purified by silica gel column chromatography (n-hexane/ethyl acetate) to obtain the title compound (89.2 mg).
¹H-NMR (CDCl₃) δ: 1.24 (9H, s), 1.48 (3H, d, J = 5.3 Hz), 2.37 (1H, br t, J = 5.4 Hz), 3.29 (1H, dd, J = 13.8, 4.5 Hz), 3.36 (1H, dd, J = 13.8, 5.8 Hz), 3.76-3.89 (2H, m), 4.09-4.24 (5H, m), 4.35 (1H, ddd, J = 11.8, 6.0, 3.0 Hz), 4.61-4.70 (1H, m), 5.29 (1H, br d, J = 6.8 Hz), 6.27-6.35 (2H, m), 6.75-6.85 (3H, m), 7.22-7.36 (5H, m), 7.48-7.57 (2H, m), 7.71-7.79 (2H, m).
MS (m/z) : 687 (M+H)⁺.

The same procedures as in Example 115 were performed to obtain the compound below. For the raw material of Step 68, the corresponding Example compound at the right of the following table were used.

**[Table 48]**

| Example | Name and Structure | Equipment data | Raw material compound of Step 68 |
|---|---|---|---|
| 116 | (R)-1-((((S)-3-(3-((3-(4-Fluorophenoxy)-3-phenylazetidin-1-yl)sulfonyl)phenyl)-1-(2-hydroxyethoxy)-1-oxopropan-2-yl)carbamoyl)oxy)ethyl pivalate | ¹H-NMR (CDCl₃) δ: 1.19 (9H, s), 1.46 (3H, d, J = 5.5 Hz), 2.39 (1H, br t, J = 5.6 Hz), 3.24 (1H, dd, J = 14.1, 5.5 Hz), 3.31 (1H, dd, J = 13.8, 5.8 Hz), 3.78-3.85 (2H, m), 4.09-4.24 (5H, m), 4.35 (1H, ddd, J = 11.8, 5.6, 3.6 Hz), 4.62-4.70 (1H, m), 5.28 (1H, br d, J = 7.5 Hz), 6.28-6.35 (2H, m), 6.75-6.83 (3H, m), 7.25-7.35 (5H, m), 7.47-7.56 (2H, m), 7.71-7.74 (1H, m), 7.76 (1H, dt, J = 7.0, 1.8 Hz). | Example 92 |
| | 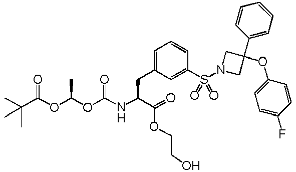 | | |
| | | MS (m/z) : 709 (M+Na)⁺. | |

### [Example 117]

### (4S)-1-Acetoxyethyl 4-(3-((3-(4-fluorophenoxy)-3-phenylazetidin-1-yl)sulfonyl)benzyl)-5-oxoxazolidin-3-carboxylate

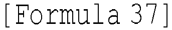

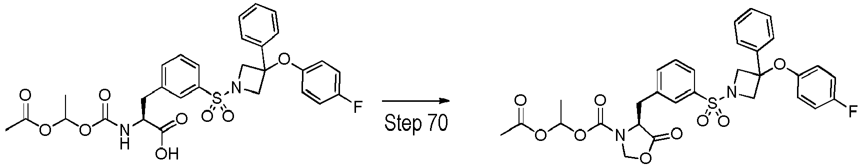

### [Step 70]

### (4S)-1-Acetoxyethyl 4-(3-((3-(4-fluorophenoxy)-3-phenylazetidin-1-yl)sulfonyl)benzyl)-5-oxoxazolidin-3-carboxylate

To a solution of the compound (48.5 mg) of Example 84 in toluene (1 ml), p-toluenesulfonic acid monohydrate (1.50 mg), paraformaldehyde (20.0 mg) and magnesium sulfate (30.0 mg) were added at room temperature. The mixture was stirred under heating and reflux for 1.5 hours. To the reaction solution, a saturated aqueous sodium hydrogen carbonate solution was added, and the mixture was extracted with ethyl acetate. The extract was washed with saturated saline, and dried over sodium sulfate. The sodium sulfate was filtered off, and then the solvent was distilled off under reduced pressure. The obtained residue was purified by silica gel chromatography (n-hexane/ethyl acetate) to obtain the title compound (19.7 mg).
¹H-NMR (CDCl₃) δ: 1.44-1.65 (3H, m), 2.03-2.22 (3H, m), 3.24-3.34 (1H, m), 3.40-3.66 (1H, m), 4.10 (1H, d, J = 8.8 Hz), 4.11 (1H, d, J = 8.8 Hz), 4.18-4.28 (2H, m), 4.31-4.45 (1H, m), 4.46-4.60 (1H, m), 5.06-5.21 (1H, m), 6.26-6.37 (2H, m), 6.80 (2H, t, J = 8.5 Hz), 6.85-6.93 (1H, m), 7.21-7.44 (5H, m), 7.47-7.59 (2H, m), 7.68 (1H, br s), 7.77-7.84 (1H, m).
MS(m/z): 613 (M+H)⁺.

### [Example 118]

### (4S)-1-Acetoxyethyl 4-(3-((3-(4-fluorophenoxy)-3-phenylazetidin-1-yl)sulfonyl)benzyl)-2-methyl-5-oxoxazolidin-3-carboxylate

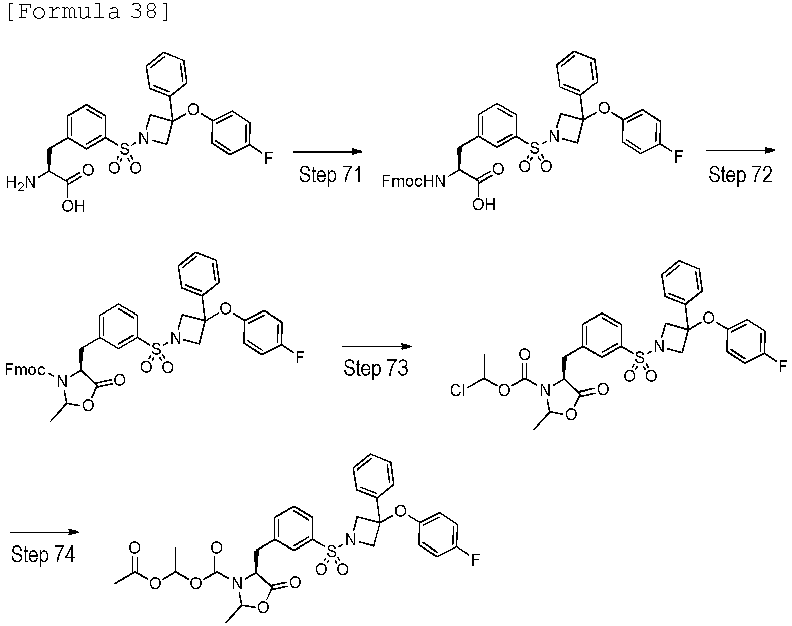

### [Step 71]

### (S)-2-((((9H-Fluoren-9-yl)methoxy)carbonyl)amino)-3-(3-((3-(4-fluorophenoxy)-3-phenylazetidin-1-yl)sulfonyl)phenyl)propanoic acid

To a mixed solution of the compound (567 mg) of Example 41 in acetonitrile (15 ml) and water (15 ml), N,N-diisopropylethylamine (442 µl) and N-(9-fluorenylmethoxycarbonyloxy)succinimide (398 mg) were added at room temperature. The mixture was stirred at room temperature for 2 hours. To the reaction solution, 1 N hydrochloric acid was added, and the mixture was extracted with chloroform. The extract was dried over sodium sulfate. The sodium sulfate was filtered off, and then the solvent was distilled off under reduced pressure. The obtained residue was purified by silica gel chromatography (chloroform/methanol) to obtain the title compound (825 mg).
¹H-NMR (CDCl₃) δ: 3.14-3.38 (2H, m), 4.09-4.21 (6H, m), 4.37-4.52 (2H, m), 4.65-4.77 (1H, m), 5.16-5.24 (1H, m), 6.28 (2H, dd, J = 9.2, 4.1 Hz), 6.75 (2H, t, J = 8.7 Hz), 7.25-7.35 (4H, m), 7.40 (4H, dd, J = 7.4, 7.4 Hz), 7.46-7.51 (1H, m), 7.56 (2H, d, J = 7.5 Hz), 7.68-7.72 (1H, m), 7.77 (4H, d, J = 7.8 Hz).
MS (m/z): 715 (M+Na)⁺.

### [Step 72]

### (4S)-(9H-Fluoren-9-yl)methyl 4-(3-((3-(4-fluorophenoxy)-3-phenylazetidin-1-yl)sulfonyl)benzyl)-2-methyl-5-oxooxazolidin-3-carboxylate

To a solution of the compound (711 mg) obtained by the method of Step 71, metaldehyde (904 mg), N,N-diisopropylethylamine (215 µl) in dichloromethane (11 ml), trimethylsilyl trifluoromethane sulfonate (278 µl) was added at room temperature. The mixture was stirred at room temperature for 4 hours. To the reaction solution, a saturated aqueous sodium hydrogen carbonate solution was added, and then the mixture was extracted with ethyl acetate. The extract was dried over sodium sulfate. The sodium sulfate was filtered off, and then the solvent was distilled off under reduced pressure. The obtained residue was purified by silica gel chromatography (n-hexane/ethyl acetate) to obtain a high polar isomer (66.6 mg) and a low polar isomer (428 mg) of the title compound.

### High polar isomer

¹H-NMR (CDCl₃) δ: 1.46-1.60 (3H, m), 4.07-4.22 (6H, m), 4.23-4.29 (1H, m), 4.83 (1H, dd, J = 10.8, 4.0 Hz), 6.28-6.36 (2H, m), 6.79 (2H, t, J = 8.5 Hz), 7.23-7.45 (14H, m), 7.58 (2H, d, J = 8.0 Hz), 7.74 (4H, dd, J = 13.7, 7.4 Hz) .
MS(m/z): 741 (M+Na)⁺.

### Low polar isomer

¹H-NMR (CDCl₃) δ: 1.46-1.61 (3H, m), 2.29-2.45 (0.5H, m), 2.56-2.76 (0.5H, m), 3.08-3.29 (0.5H, m), 3.62-3.94 (0.5H, m), 4.00-4.35 (3H, m), 4.35-4.72 (1H, m), 4.72-5.09 (2H, m), 6.28 (2H, br s), 6.71-6.93 (3H, m), 7.22-7.47 (14H, m), 7.50-7.65 (2H, m), 7.66-7.82 (3H, m).
MS(m/z): 741 (M+Na)⁺.

### [Step 73]

### (4S)-Chloromethyl 4-(3-((3-(4-fluorophenoxy)-3-phenylazetidin-1-yl)sulfonyl)benzyl)-2-methyl-5-oxoxazolidin-3-carboxylate

To a solution of the low polar isomer (513 mg) obtained by the method of Step 72 in dichloromethane (8 ml), 1,8-diazabicyclo[5.4.0]undec-7-ene (140 µl) was added. The mixture was stirred at room temperature for 30 minutes, then cooled to 0°C. To the reaction solution, 1-chloroethyl chloroformate (311 µl) was added. The mixture was stirred at room temperature for 30 minutes. To the reaction solution, a saturated aqueous sodium hydrogen carbonate solution was added, and the mixture was extracted with ethyl acetate. The extract was dried over sodium sulfate. The sodium sulfate was filtered off, and then the solvent was distilled off under reduced pressure. The obtained residue was purified by silica gel chromatography (n-hexane/ethyl acetate) to obtain a high polar isomer (109 mg) and a low polar isomer (63.4 mg) of the title compound.

### High polar isomer

¹H-NMR (CDCl₃) δ: 1.37-1.55 (3H, m), 1.73-2.03 (3H, m), 3.22-3.64 (2H, m), 4.03-4.33 (4H, m), 4.51-4.67 (1H, m), 4.83-5.02 (0.5H, m), 5.51-5.70 (0.5H, m), 6.27-6.42 (2H, m), 6.57-6.74 (1H, m), 6.75-6.88 (2H, m), 7.24-7.45 (5H, m), 7.45-7.75 (3H, m), 7.76-7.86 (1H, m).
MS(m/z): 604 (M+H)⁺.

### Low polar isomer

¹H-NMR (CDCl₃) δ: 1.41-1.58 (3H, m), 1.79-1.99 (3H, m), 3.22-3.38 (1H, m), 3.61 (0.5H, dd, J = 13.8, 5.0 Hz), 3.77 (0.5H, dd, J = 13.9, 5.4 Hz), 4.04-4.32 (4H, m), 4.52-4.64 (1H, m), 4.80-4.99 (1H, m), 6.24-6.39 (2H, m), 6.63-6.75 (1H, m), 6.75-6.86 (2H, m), 7.23-7.43 (5H, m), 7.44-7.64 (2.5H, m), 7.74-7.87 (1.5H, m).
MS(m/z): 604(M+H)⁺.

### [Step 74]

### (4S)-1-Acetoxyethyl 4-(3-((3-(4-fluorophenoxy)-3-phenylazetidin-1-yl)sulfonyl)benzyl)-2-methoxy-5-oxoxazolidin-3-carboxylate

To a solution of the high polar isomer obtained by the method of Step 73 (40 mg) in dichloromethane (1.5 ml), mercury(II) acetate (27.5 mg) was added at room temperature, and the mixture was stirred at room temperature for 16 hours and 30 minutes. To the reaction solution, a saturated aqueous sodium hydrogen carbonate solution was added, and the mixture was extracted with ethyl acetate. The extract was dried over sodium sulfate. The sodium sulfate was filtered off, and then the solvent was distilled off under reduced pressure. The obtained residue was purified by silica gel chromatography (n-hexane/ethyl acetate) to obtain a high polar isomer (21.4 mg) and a low polar isomer (22.7 mg) of the title compound.

### High polar isomer

¹H-NMR (CDCl₃) δ: 0.66-0.86 (3H, m), 1.40-1.75 (3H, m), 2.11 (3H, br s), 3.21-3.57 (2H, m), 4.15 (1H, d, J = 8.8 Hz), 4.16 (1H, d, J = 8.8 Hz), 4.22 (1H, d, J = 8.5 Hz), 4.22 (1H, d, J = 9.0 Hz), 4.46-4.66 (1H, m), 5.48-5.70 (1H, m), 6.34 (2H, dd, J = 9.0, 4.3 Hz), 6.75-6.84 (2H, m), 6.84-6.94 (1H, m), 7.28-7.43 (5H, m), 7.45-7.60 (2H, m), 7.67 (1H, br s), 7.80 (1H, d, J = 7.3 Hz).
MS (m/z) : 627 (M+H)⁺.

### Low polar isomer

¹H-NMR (CDCl₃) δ: 1.43 (3H, d, J = 5.3 Hz), 1.49-1.65 (3H, m), 2.01-2.22 (3H, m), 3.26 (1H, dd, J = 14.1, 2.3 Hz), 3.43-3.59 (0.5H, m), 3.70-3.86 (0.5H, m), 4.10 (2H, d, J = 8.8 Hz), 4.21 (2H, d, J = 8.5 Hz), 4.56 (1H, br s), 4.77-5.03 (1H, m), 6.24-6.39 (2H, m), 6.73-6.84 (2H, m), 6.93 (1H, q, J = 5.4 Hz), 7.26-7.42 (5H, m), 7.43-7.58 (2H, m), 7.58-7.74 (1H, m), 7.74-7.84 (1H, m).
MS (m/z) : 627 (M+H)⁺.

### [Example 119]

### (4S)-1-Acetoxyethyl 4-(3-((3-(4-fluorophenoxy)-3-phenylazetidin-1-yl)sulfonyl)benzyl)-2-isopropyl-5-oxoxazolidin-3-carboxylate

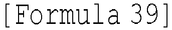

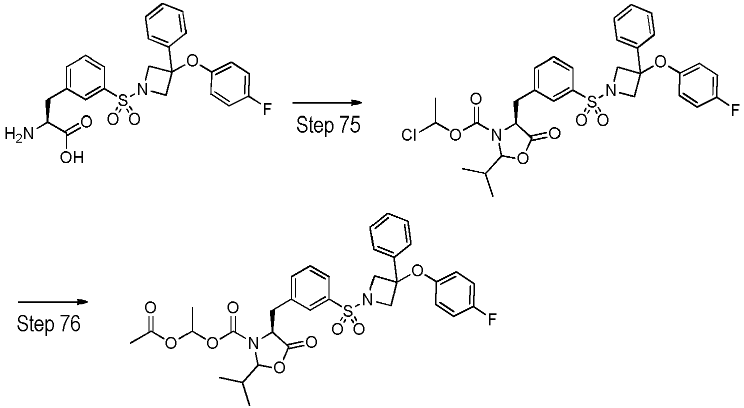

### [Step 75]

### (4S)-1-Chloroethyl 4-(3-((3-(4-fluorophenoxy)-3-phenylazetidin-1-yl)sulfonyl)benzyl)-2-isopropyl-5-oxoxazolidin-3-carboxylate

To a solution of the compound (1 g) of Example 41 in isobutylaldehyde (9 ml), N,N-diisopropylethylamine (371 µl) and trimethylsilyl trifluoromethane sulfonate (845 µl) were added at room temperature. The mixture was stirred at room temperature for 1 hour, then N,N-diisopropylethylamine (2.97 ml) and 1-chloroethyl chloroformate (1.16 ml) were added, and the mixture was stirred at room temperature for 1 hour. To the reaction solution, a saturated aqueous sodium hydrogen carbonate solution was added, and the mixture was extracted with ethyl acetate. The extract was washed with saturated saline, and dried over sodium sulfate. The sodium sulfate was filtered off, and then the solvent was distilled off under reduced pressure. The obtained residue was purified by silica gel chromatography (n-hexane/ethyl acetate) to obtain a high polar isomer (333 mg) and a low polar isomer (93 mg) of the title compound.

### High polar isomer

¹H-NMR (CDCl₃) δ: 0.71-1.03 (6H, m), 1.67-1.96 (4H, m), 3.18-3.67 (2H, m), 4.07-4.30 (4H, m), 4.31-4.65 (1H, m), 5.30-5.55 (1H, m), 6.27-6.37 (2H, m), 6.48-6.57 (1H, m), 6.75-6.83 (2H, m), 7.21-7.40 (5H, m), 7.47-7.63 (2H, m), 7.74-7.84 (2H, m).
MS(m/z): 631(M+H)⁺.

### Low polar isomer

¹H-NMR (CDCl₃) δ: 0.65-1.06 (6H, m), 1.76-1.99 (3H, m), 2.34-2.61 (1H, m), 3.22-3.39 (1H, m), 3.58-3.90 (1H, m), 4.07-4.30 (4H, m), 4.49-4.63 (1H, m), 4.85-5.02 (1H, m), 6.23-6.39 (2H, m), 6.65-6.75 (1H, m), 6.75-6.85 (2H, m), 7.21-7.41 (5H, m), 7.45-7.63 (2H, m), 7.73-7.85 (2H, m). MS(m/z): 631(M+H)⁺.

### [Step 76]

### (4S)-1-Acetoxyethyl 4-(3-((3-(4-fluorophenoxy)-3-phenylazetidin-1-yl)sulfonyl)benzyl)-2-isopropyl-5-oxoxazolidin-3-carboxylate

To a solution of the high polar isomer (115 mg) obtained by the method of Step 75 in dichloromethane (2 ml), mercury(II) acetate (75 mg) was added at room temperature, and the mixture was stirred at room temperature for 22 hours. To the reaction solution, a saturated aqueous sodium hydrogen carbonate solution was added, and the mixture was extracted with ethyl acetate. The extract was dried over sodium sulfate. The sodium sulfate was filtered off, and then the solvent was distilled off under reduced pressure. The obtained residue was purified by silica gel chromatography (n-hexane/ethyl acetate) to obtain a high polar isomer (77 mg) and a low polar isomer (16.9 mg) of the title compound.

### High polar isomer

¹H-NMR (CDCl₃) δ: 0.71-0.81 (3H, m), 0.92 (3H, d, J = 7.0 Hz), 1.36-1.55 (4H, m), 2.09 (3H, d, J = 3.5 Hz), 3.14-3.36 (2H, m), 4.16 (1H, d, J = 9.0 Hz), 4.18 (1H, d, J = 8.8 Hz), 4.22 (1H, d, J = 8.8 Hz), 4.23 (1H, d, J = 9.0 Hz), 4.34-4.46 (1H, m), 5.32-5.50 (1H, m), 6.32 (2H, dd, J = 9.2, 4.4 Hz), 6.73-6.89 (3H, m), 7.26-7.41 (5H, m), 7.50-7.63 (2H, m), 7.74-7.83 (2H, m).
MS (m/z) : 655 (M+H)⁺.

### Low polar isomer

¹H-NMR (CDCl₃) δ: 0.67-0.79 (3H, m), 0.79-0.94 (3H, m), 1.51-1.66 (3H, m), 2.06 (1.5H, br s), 2.16 (1.5H, br s), 2.20-2.33 (0.5H, m), 2.51-2.65 (0.5H, m), 3.20-3.31 (1H, m), 3.51-3.60 (0.5H, m), 3.83 (0.5H, dd, J = 13.7, 5.4 Hz), 4.07-4.16 (2H, m), 4.20 (1H, d, J = 9.0 Hz), 4.24 (1H, d, J = 9.3 Hz), 4.55 (1H, br s), 4.95 (1H, d, J = 16.8 Hz), 6.24-6.38 (2H, m), 6.74-6.84 (2H, m), 6.94 (1H, q, J = 5.4 Hz), 7.27-7.41 (5H, m), 7.51 (2H, t, J = 6.9 Hz), 7.58-7.84 (2H, m).
MS (m/z) : 655 (M+H)⁺.

### [Example 120]

### (4S)-1-Acetoxyethyl 4-(3-((3-(4-fluorophenoxy)-3-phenylazetidin-1-yl)sulfonyl)benzyl)-2,2-dimethyl-5-oxoxazolidin-3-carboxylate

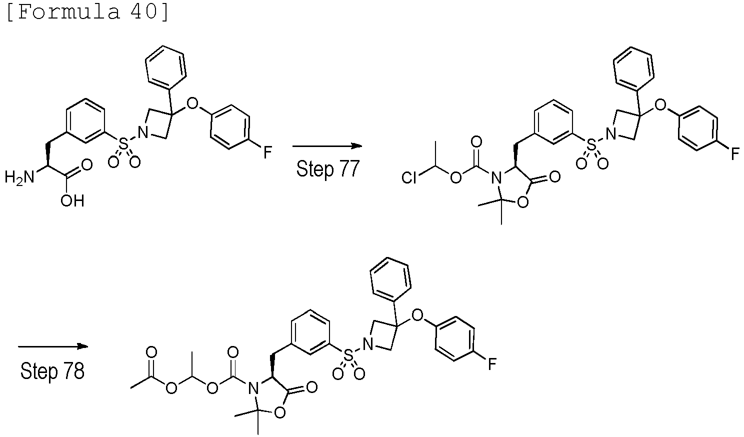

### [Step 77]

### (4S)-Chloroethyl 4-(3-((3-(4-fluorophenoxy)-3-phenylazetidin-1-yl)sulfonyl)benzyl)-2,2-dimethyl-5-oxoxazolidin-3-carboxylate

To a solution of the compound of Example 41 (100 mg) in acetone (2.5 ml), N,N-diisopropylethylamine (37 µl) and trimethylsilyl trifluoromethane sulfonate (84 µl) were added at room temperature. The mixture was stirred at room temperature for 1 hour, then, to the reaction solution, N,N-diisopropylethylamine (300 µl) and 1-chloroethyl chloroformate (116 µl) were added at room temperature, and the mixture was stirred at room temperature for 20 minutes. To the reaction solution, a saturated aqueous sodium hydrogen carbonate solution was added, and the mixture was extracted with ethyl acetate. The extract was washed with saturated saline, and dried over sodium sulfate. The sodium sulfate was filtered off, and then the solvent was distilled off under reduced pressure. The obtained residue was purified by silica gel chromatography (n-hexane/ethyl acetate) to obtain the title compound (73.1 mg).
¹H-NMR (CDCl₃) δ: 0.76-0.99 (3H, m), 1.54-1.72 (3H, m), 1.80-2.02 (3H, m), 3.30-3.74 (2H, m), 4.10-4.18 (2H, m), 4.21 (1H, d, J = 8.8 Hz), 4.26 (1H, d, J = 8.5 Hz), 4.53-4.70 (1H, m), 6.25-6.44 (2H, m), 6.67 (1H, td, J = 12.2, 6.2 Hz), 6.80 (2H, t, J = 8.5 Hz), 7.26-7.69 (8H, m), 7.74-7.86 (1H, m).
MS(m/z): 617 (M+H)⁺.

### [Step 78]

### (4S)-1-Acetoxyethyl 4-(3-((3-(4-fluorophenoxy)-3-phenylazetidin-1-yl)sulfonyl)benzyl)-2,2-dimethyl-5-oxoxazolidin-3-carboxylate

To a solution of the compound (73.1 mg) obtained by the method of Step 77 in acetic acid (1.5 ml), mercury(II) acetate (49.1 mg) was added at room temperature, and the mixture was stirred at room temperature for 13 hours. To the reaction solution, a saturated aqueous sodium hydrogen carbonate solution was added, and the mixture was extracted with ethyl acetate. The extract was washed with saturated saline, and dried over sodium sulfate. The sodium sulfate was filtered off, and then the solvent was distilled off under reduced pressure. The obtained residue was purified by silica gel chromatography (n-hexane/ethyl acetate) to obtain the title compound (73.9 mg).
¹H-NMR (CDCl₃) δ: 0.78-0.99 (3H, m), 1.45-1.72 (6H, m), 2.03-2.24 (3H, m), 3.17-3.81 (2H, m), 4.05-4.30 (4H, m), 4.46-4.70 (1H, m), 6.25-6.43 (2H, m), 6.80 (2H, t, J = 8.5 Hz), 6.86-6.99 (1H, m), 7.25-7.57 (7H, m), 7.57-7.74 (1H, m), 7.75-7.86 (1H, m).
MS(m/z): 641 (M+H)⁺.

### [Test Example 1] Effect of test substance on ³H-thymidine uptake in activated T cells

The effect of test substance on ³H-thymidine uptake by activation was evaluated using T cells from mouse spleen. The spleen was removed from euthanized C57BL/6J mice, and finely sliced, then triturated on a cell strainer (40 µm mesh), and cells were collected by centrifugation. The collected splenocytes were treated with Cold lyser containing 0.17 M NH₄Cl for 5 minutes to hemolytically remove red blood cells and then washed. The resulting cells were used as the splenocytes. From the splenocytes, CD4+ T cells were isolated using an EasySep Mouse CD4+ T cell isolation kit (STEMCELL Technologies Inc.). The CD4+ T cells were seeded into 96-well round-bottom plates. To the plates, test substance or Vehicle was added, and Dynabeads Mouse T-Activator CD3/CD28 (Thermo Fisher Scientific Inc.) was added, then the cells were cultured at 37°C under 5%CO₂. After 24 hours of culture, ³H-Thymidine (PerkinElmer Inc.) diluted with medium was added to each well, then the cells were further cultured at 37°C under 5%CO₂ overnight. To measure ³H-Thymidine uptake into the nuclei of the cells, the cells were collected into filter plates UniFilter-96 GF/B (PerkinElmer Inc.) using a FilterMate Universal Harvester (PerkinElmer Inc.). The filter plates were placed in a dryer and left still for 2 hours or more. Then, scintillation cocktail MicroScint-O (PerkinElmer Inc.) was added to the plates, and radioactivity was measured with TopCount NXT (PerkinElmer Inc.). With the radioactivity of the test substance-free group set as 100% and the radioactivity of the non-activation group as 0%, the relative value (proliferation rate, % of vehicle) of the radioactivity of each well to which test substance was added was calculated. The value of % of vehicle was incorporated into analysis software Prism (Version 5.0, GraphPad Software), the "Nonlinear Regression Log (inhibitor) vs Reaction Variable Inclination" was selected, and then sigmoid fitting with restrictions of Bottom = 0 and Top = 100 was performed to calculate 50% inhibitory concentration (IC₅₀). The results are shown in Table 1. The compounds of the present invention suppressed ³H-thymidine uptake in activated T cells. Since ³H-thymidine uptake is an indicator of cell proliferation, these results indicate that the compounds of the invention suppress proliferation of activated T cells.

**[Table 49]**

| Example No. | IC₅₀ (µM) | Example No. | IC₅₀ (µM) | Example No. | IC₅₀ (µM) |
|---|---|---|---|---|---|
| 1 | 0.0012 | 16 | 0.068 | 31 | 0.21 |
| 2 | 0.015 | 17 | 0.0012 | 32 | 0.011 |
| 3 | 0.0031 | 18 | 0.0029 | 33 | 0.0078 |
| 4 | 0.0026 | 19 | 0.003 | 34 | 0.019 |
| 5 | 0.0032 | 20 | 0.003 | 35 | 0.017 |
| 6 | 0.0012 | 21 | 0.0025 | 36 | 0.078 |
| 7 | 0.0041 | 22 | 0.0087 | 37 | 0.085 |
| 8 | 0.003 | 23 | 0.016 | 38 | 0.034 |
| 9 | 0.0043 | 24 | 0.00049 | 39 | 0.14 |
| 10 | 0.021 | 25 | 0.092 | 40 | 0.0022 |
| 11 | 0.02 | 26 | 0.0025 | 41 | 0.0051 |
| 12 | 0.0096 | 27 | 0.0029 | 42 | 0.0041 |
| 13 | 0.003 | 28 | 0.0023 | 43 | 0.0086 |
| 14 | 0.03 | 29 | 0.18 | | |
| 15 | 0.0029 | 30 | 1.1 | | |

### [Test Example 2]

### Effect of test substance on IFN-α production in mouse splenocytes

Production of IFN-α by TLR9 ligand CpG-ODN D35 stimulation activating pDC was evaluated. Splenocytes were prepared from euthanized C57BL/6J mice in the same way as Test Example 1. Splenocytes were seeded into 96-well round-bottom plates, and test substance was added, and then the plates were preincubated for 30 minutes. Then, CpG-ODN D35 (final concentration 1 µM) was added, and the cells were cultured at 37°C under 5%CO₂. After 24 hours of culture, the culture supernatant was collected, and the IFN-α concentration in the supernatant was measured with an ELISA kit (Mouse IFN-alpha Platinum ELISA Kit, eBioscience). The relative value of the IFN-α concentration of each well when the IFN-α production of the test substance-free group was set as 100% and the amount of the non-stimulated group as 0% (IFN-α production rate, % of vehicle) was calculated. The relative value of the IFN-α concentration was incorporated into analysis software Prism (Version 5.0, GraphPad Software), the "Nonlinear Regression Log (inhibitor) vs Reaction Variable Inclination" was selected for each compound, and then sigmoid fitting with restrictions of Bottom = 0 and Top = 100 was performed to create a graph of the concentration reaction curve and calculate 50% inhibitory concentration (IC₅₀). The results are shown in Figure 1. The compounds of Examples 41 and 42 exhibited concentration-dependent suppression effects, and their IC₅₀ was 21 nM and 13 nM, respectively.

### [Test Example 3] Effect of test substance in mouse acute GVHD model

A mouse acute graft-versus-host disease (GVHD) model was generated in accordance with a previous report (Puliaev R et al. Differential requirement for IFN-gamma in CTL maturation in acute murine graft-versus-host disease. J Immunol. 173, 910-9(2004)). Splenocytes were prepared from euthanized donor mice (C57BL/6N), suspended in PBS without hemolysis of erythrocytes, and administered to host mice, BDF1 mice, from the tail vein (the number of transfer cells was adjusted to be 7 to 8 x 10⁷ cells/0.2 mL/mouse in every test, and matched between individuals within the same test). In the Normal group, 0.2 mL of PBS was administered in place of the cells. The test substance was crushed in an agate mortar, and then suspended in a 0.5% hydroxypropyl cellulose (HPC) solution to prepare a dosage solution. In the Vehicle group, 0.5% HPC was used as a dosage solution. To the host mice, the dosage solution was forcibly orally administered (0.2 mL/mouse) twice daily with a feeding needle from the morning of Day 0 (cell transfer) to the end of the experiment. The tail vein was cut without anesthesia on Day 7 and a small amount of blood was collected with a hematocrit tube (EDTA). Plasma was collected by centrifugation and IFN-γ concentration in plasma was measured with an αLISA kit (Mouse IFN-γ alphaLISA, PerkinElimer, AL501C). The graph of Figure 2 shows the results of the compound of Example 89 when the compound was orally administered twice a day (BID) at 1.5, 5, 15, or 50 mg/kg. For the difference between the Normal group and the Vehicle group, comparison between two groups was performed by Student's t-test. The difference between the Vehicle group and the test substance administration group was analyzed by a parametric multiple-comparison test, Dunnett test. The compound of Example 89 inhibited IFN-γ in plasma in a dose-dependent manner and exhibited significant action at 1.5 mg/kg, BID or more.

Furthermore, as a result of similar tests, the compounds of Examples 44 and 104 exhibited significant suppression effects at an effective dose (the dose of the experimental group having a p-value of 0.05 or less relative to the Vehicle group) of 10 mg/kg, BID and 15 mg/kg, BID or more, respectively.

### [Test Example 4] Effect of test substance on kidney disorder in mouse chronic GVH model

A mouse chronic graft-versus-host (GVH) model was generated in accordance with a previous report (Via CS. Advances in lupus stemming from the parent-into-Fl model. Trends Immunol.31, 236-45(2010)). Splenocytes were prepared from euthanized donor mice (DBA2 mice), suspended in PBS without hemolysis of erythrocytes, and administered to host mice, BDF1 mice, from tail veins (1 x 10⁸ cells/0.2 mL/mouse) at Day 0 and Day 4, twice. In the Normal group, 0.2 mL of PBS was administered in place of the cells. The test substance was crushed in an agate mortar, and then suspended in a 0.5% hydroxypropyl cellulose (HPC) solution to prepare a dosage solution. In the Vehicle group, 0.5% HPC was used as a dosage solution. To the host mice, the dosage solution was forcibly orally administered (0.2 mL/mouse) twice daily with a feeding needle from Day 4 (second cell transfer) to the end of the experiment. Urine was collected on Day 32 under free diet with a metabolic cage. Urine protein concentration was measured with a urine protein test kit (total protein kit microTP test wako, FUJIFILM Wako Pure Chemical Corporation, pyrogallol red method), and urine creatinine concentration was measured using a creatinine test kit (LabAssay(TM) creatinine, FUJIFILM Wako Pure Chemical Corporation, Jaffe method), and the ratio of urine protein to creatinine was calculated for each individual. The graph of Figure 3 shows the results of the compound of Example 89 when the compound was orally administered at 1.5, 5, 15, or 50 mg/kg, BID, and mycophenolate mofetil (MMF: positive control) was orally administered at 30 mg/kg, BID. For the difference between the Normal group and the Vehicle group, the Wilcoxon test (non-parametric, comparison between two groups) was performed, and for the difference between the Vehicle group and the test substance administration group, the analysis was performed by the Shirley-Williams test (non-parametric, multiple comparison with dose response). The compound of Example 89 suppressed the urine protein/creatinine ratio in a dose-dependent manner, and the minimum effective dose was 15 mg/kg, BID.

Furthermore, as a result of similar tests, the effective doses (the dose of the experimental group showing a p-value of 0.05 or less relative to the Vehicle group) of the compounds of Examples 97 and 104 were both 15 mg/kg, BID.

### [Test Example 5] Effect of compound of Example 44 in NZB/W F1 mice that naturally develop SLE-like symptoms and nephritis

To NZB/W F1 mice that naturally develop SLE-like symptoms and nephritis, test substance was forcibly orally administered (0.2 mL/mouse) twice daily with a feeding needle, starting at the age of 12 weeks. The compound of Example 44 was orally administered at 30, 100 mg/kg, BID, and MMF was orally administered at 60 mg/kg, QD. The N was set to 20 in both of the Vehicle group and the compound of Example 44 administration group, and set to 15 in the MMF administration group. The test substance was crushed in an agate mortar, and then suspended in a 0.5% hydroxypropyl cellulose (HPC) solution to prepare a dosage solution. In the Vehicle group, 0.5% HPC was used as a dosage solution. Mice were observed daily, and those that reached a dying state such as weight loss and dyspnea were euthanized, and the survival rate was evaluated. The number of survival days was analyzed by the Kaplan-Meier method and the log rank test was performed. In the Vehicle group, deaths were observed from the age of 29 weeks, and the survival rate at the end of the experiment (at the age of 44 weeks) was 35%. In contrast, in the Example 44 (30, 100 mg/kg) group and the MMF group, no death was observed until the end of the experiment, resulting in significant improvement of survival rate.

### [Test Example 6] Pharmacokinetic test of prodrug compounds in mouse

For prodrug compounds, the compounds of Examples 44, 103, and 104 (active compound of these is the compound of Example 41) and the compounds of Examples 89, 94, and 97 (active compound of these is the compound of Example 42), the plasma concentrations of the active compounds after oral administration to mice were measured, and various PK parameters were calculated. The method was as follows:

### (Method)

To C57BL/6J mice (females, non-fasting), the Example compounds were administered and blood sampling was performed under the following conditions.

### Administration route and dosage amount (number of cases, administration medium)

Intravenous (IV): 1 mg/kg (n = 2, 10% HP-β-CD dissolved in Saline/DMSO = 95/5, v/v)
Oral (PO): 10 mg/kg (n = 2, 0.5% HPC dissolved in water) Blood sampling time point
IV: 0.083, 0.33 1, 2, 4, 7 and 24 h
PO: 0.33, 1, 2, 4, 7 and 24 h

After blood sampling, plasma was obtained by centrifugation, and to 10 µL of the obtained plasma, an equal amount of DMSO was added. Then, to the mixture, 200 µL of 100 ng/mL antipyrine acetonitrile solution was added as an internal standard (IS) solution, and then the mixed solution was centrifuged at 4,000 rpm for 5 minutes. Then, to 50 µL of the obtained supernatant, 50 µL of 0.1% aqueous formic acid solution was added and mixed to form a LC-MS/MS measurement sample. Furthermore, an equal amount of DMSO solution of test substance prepared at 1, 10, 100, 1000, 10000 nM or 1, 10, 100, 1000, 10000 ng/mL was added to a blank plasma and the mixture was processed as described above to form calibration curve samples.
The obtained samples were measured and quantitatively analyzed using MassLynx (version 4.1, Waters Corporation) under the following conditions.

### - LC conditions

### Acquity Ultra Performance LC (Waters Corporation)

Column: ACQUITY UPLC BEH C18 1.7 µm, 2.1 x 50 mm (Waters Corporation)
Mobile phase A: 0.1 vol% Formic Acid-Distilled Water
(Kanto Chemical Co., Inc.)
Moblie phase B: Acetonitrile (Kanto Chemical Co., Inc.)

### Gradient:

**[Table 50]**

| Time (min) | %A | %B |
|---|---|---|
| 0 | 90.0 | 10.0 |
| 0.50 | 1.0 | 99.0 |
| 0.99 | 1.0 | 99.0 |
| 1.00 | 90.0 | 10.0 |

Flow rate: 0.5 mL/min
Injection volume: 5 µL

### - MS conditions

### Acquity TQ Detector (Waters Corporation)

Source temperature: 150°C
Desolvation temperature: 450°C
Capilary: 3.9 kV
Extractor: 2 V
RF Lens: 0.5 V
Collision gas flow: 0.20 mL/min

### Monitored ions:

**[Table 51]**

| Example No. | Q1 (m/z*) | Q3 (m/z*) | Cone (V) | Collision (V) |
|---|---|---|---|---|
| 41 (Active compound) | 471.0 | 130.2 | 20 | 30 |
| 42 (Active compound) | 471.1 | 130.2 | 20 | 40 |
| Antipyrine (IS) | 189.0 | 55.8 | 30 | 30 |

Q1: Precursor ion
Q3: Product ion
*: mass-to-charge ratio

From the plasma concentration measurements of the obtained active compound, the following PK parameters were calculated using Moment (Microsoft Excel). AUClast: Area under plasma concentration-time curve to the last quantifiable time point
Cmax: Maximum plasma concentration
BA: Bioavailability

The PK parameters of the active compound (compound of Example 41) after PO administration of the compound of Example 44, 103, or 104 (prodrug compound) to mice at 10 mg/kg are shown in [Table 52].

**[Table 52]**

| Example No. | AUClast (ng·h/mL) | Cmax (ng/mL) | BA (%) |
|---|---|---|---|
| 44 | 2232.3 | 1476.6 | 30.7 |
| 103 | 2590.9 | 986.6 | 35.6 |
| 104 | 2615.6 | 1411.3 | 35.9 |

Note that the AUClast after IV administration of the compound of Example 41 to mice at 1 mg/kg was 728.4 ng h/mL. Using this value, the BA above was calculated.

Furthermore, the PK parameters of the active compound (compound of Example 42) after PO administration of the compound of Example 89, 94, or 97 (prodrug compound) to mice at 10 mg/kg are shown in [Table 53].

**[Table 53]**

| Example No. | AUClast (ng·h/mL) | Cmax (ng/mL) | BA (%) |
|---|---|---|---|
| 89 | 3177.8 | 752.6 | 63.7 |
| 94 | 2543.4 | 937.5 | 51.0 |
| 97 | 2991.1 | 1025.7 | 59.9 |

Note that the AUClast after IV administration of the compound of Example 42 to mice at 1 mg/kg was 499.2 ng h/mL. Using this value, the BA above was calculated.

As shown, it was demonstrated that prodrug compounds of the present invention exhibit a sufficient bioavailability of the active compound and have a good PK profile.

### Industrial applicability

The compound represented by general formula (I) of the present invention or a pharmaceutically acceptable salt thereof is useful as a therapeutic or prophylactic agent for a disease whose symptoms are prevented, ameliorated or reduced by suppressing proliferation of activated T cells or suppressing production of IFN-α by activated pDC. Examples of such disease include diseases involving the immune system, particularly autoimmune diseases. Specific examples of the diseases involving the immune system include systemic lupus erythematosus (SLE); a renal lesion, a central nerve disorder, a pulmonary disorder or vasculitis due to SLE; multiple myositis; ulcerative colitis; Sjogren's syndrome; rejection with organ transplantation; graft-versus-host disease (GVHD); dry eye; rheumatoid arthritis; Crohn's disease; psoriasis; dermatomyositis; atopic dermatitis; systemic scleroderma; and type I diabetes mellitus. Preferred diseases are SLE; a renal lesion, a central nerve disorder, a pulmonary disorder or vasculitis due to SLE; multiple myositis; ulcerative colitis; Sjogren's syndrome; graft-versus-host disease (GVHD) and psoriasis. Particularly preferred diseases are SLE, a renal lesion due to SLE (also referred to as lupus nephritis), and the like. The compound represented by general formula (I) of the present invention or a pharmaceutically acceptable salt thereof suppresses the function of immune cells by suppressing proliferation of activated T cells or suppressing production of IFN-α by activated pDC, and exerts an effect on the treatment and prevention of diseases involving the immune system described above.

## Claims

1. A compound represented by general formula (I): 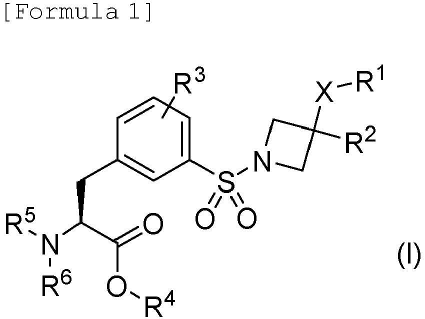 wherein
X indicates a C₁-C₃ alkylene group, an oxygen atom, a sulfur atom, a group represented by formula -NH-, a group represented by formula -N(C₁-C₃ alkyl)-, or a single bond;
R¹ indicates a C₃-C₆ cycloalkyl group, a phenyl group, or an aromatic heterocyclic ring group,
wherein the aromatic heterocyclic ring is a 5- to 6-membered aromatic heterocyclic ring having in the ring 1 to 4 heteroatoms independently selected from an oxygen atom, a sulfur atom, and a nitrogen atom, and
the phenyl group or the aromatic heterocyclic ring group may have 1 to 5 substituents independently selected from substituent group a below;
R² indicates a hydrogen atom, a C₁-C₆ alkyl group, a C₁-C₆ alkyl group substituted with 1 to 3 halogen atoms, a C₃-C₆ cycloalkyl group, a phenyl group, or an aromatic heterocyclic ring group,
wherein the aromatic heterocyclic ring is a 5- to 6-membered aromatic heterocyclic ring having in the ring 1 to 4 heteroatoms independently selected from an oxygen atom, a sulfur atom, and a nitrogen atom, and
the phenyl group or the aromatic heterocyclic ring group may have 1 to 5 substituents independently selected from substituent group b below;
R³ indicates a hydrogen atom or a halogen atom;
R⁴ indicates a hydrogen atom, a C₁-C₆ alkyl group, a C₃-C₆ cycloalkyl group, or is taken together with R⁵ or R⁶ to represent a C₁-C₆ alkylene group,
wherein the C₁-C₆ alkyl group, the C₃-C₆ cycloalkyl group, and the C₁-C₆ alkylene group may have 1 to 3 substituents independently selected from substituent group c below;
R⁵ and R⁶ each independently indicate a hydrogen atom, or a group represented by formula A: 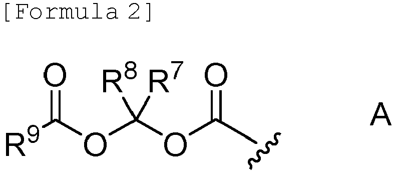 wherein
R⁷ and R⁸ each independently indicate a hydrogen atom, or a C₁-C₆ alkyl group;
R⁹ indicates a C₁-C₆ alkyl group, a C₃-C₆ cycloalkyl group, a phenyl group, or a heterocyclic ring group,
wherein the heterocyclic ring shows a saturated or unsaturated 5- to 6-membered heterocyclic ring having in the ring 1 to 4 heteroatoms selected from an oxygen atom, a sulfur atom, and a nitrogen atom, and
the C₁-C₆ alkyl group, the C₃-C₆ cycloalkyl group, the phenyl group, or the heterocyclic ring group may have 1 to 3 substituents independently selected from the substituent group c below,
substituent group a: a halogen atom, a C₁-C₆ alkyl group, a C₁-C₆ alkyl group substituted with 1 to 3 halogen atoms, a cyano group, a carbamoyl group, a deuterium atom, and a phenyl group, wherein the phenyl group is optionally substituted with 1 to 3 substituents independently selected from the group consisting of a C₁-C₆ alkyl group and a halogen atom;
substituent group b: a halogen atom and a deuterium atom;
substituent group c: a halogen atom, a C₁-C₆ alkyloxy group, a C₃-C₆ cycloalkyloxy group, a C₁-C₆ alkyloxy group substituted with 1 to 3 halogen atoms, a hydroxyl group, a C₁-C₇ acyl group, an amino group, a mono-C₁-C₆ alkylamino group, a di-(C₁-C₆ alkyl)amino group, a morpholino group, a cyano group, a carbamoyl group, a mono-C₁-C₆ alkylcarbamoyl group, a di-(C₁-C₆ alkyl)carbamoyl group, a phenyl group, and a medoxomil group (5-methyl-2-oxo-1,3-dioxol-4-yl group),
or a pharmaceutically acceptable salt thereof.

2. The compound according to claim 1 or a pharmaceutically acceptable salt thereof, wherein, in the general formula (I), X indicates a C₁-C₃ alkylene group, an oxygen atom, or a single bond.

3. The compound according to claim 1 or 2 or a pharmaceutically acceptable salt thereof, wherein, in the general formula (I), R¹ represents a C₃-C₆ cycloalkyl group, a phenyl group, or a pyridyl group, wherein the phenyl group and the pyridyl group may have 1 to 5 substituents independently selected from the substituent group a.

4. The compound according to any one of claims 1 to 3 or a pharmaceutically acceptable salt thereof, wherein, in the general formula (I), R² indicates a hydrogen atom, a C₁-C₆ alkyl group, a C₁-C₆ alkyl group substituted with 1 to 3 halogen atoms, a C₃-C₆ cycloalkyl group, a phenyl group which may have 1 to 5 substituents independently selected from the substituent group b, an unsubstituted pyridyl group, or an unsubstituted thiazolyl group.

5. The compound according to any one of claims 1 to 4 or a pharmaceutically acceptable salt thereof, wherein, in the general formula (I), R³ indicates a hydrogen atom.

6. The compound according to any one of claims 1 to 5 or a pharmaceutically acceptable salt thereof, wherein, in the general formula (I), R⁴ indicates a hydrogen atom or a C₁-C₆ alkyl group which may have 1 to 3 substituents independently selected from substituent group c1 below, or is taken together with R⁵ or R⁶ to represent a methylene, methylmethylene, dimethylmethylene or isopropylmethylene group,
substituent group c1: a hydroxyl group, a mono-C₁-C₆ alkylamino group, a di-(C₁-C₆ alkyl)amino group, a morpholino group, and a medoxomil group (5-methyl-2-oxo-1,3-dioxol-4-yl group).

7. The compound according to any one of claims 1 to 6 or a pharmaceutically acceptable salt thereof, wherein, in the general formula (I), one of R⁵ and R⁶ is a hydrogen atom and the other is formula A, or both R⁵ and R⁶ are hydrogen atoms.

8. The compound according to any one of claims 1 to 7 or a pharmaceutically acceptable salt thereof, wherein, in the general formula (I), one of R⁵ and R⁶ is a hydrogen atom and the other is formula A, or both R⁵ and R⁶ are hydrogen atoms, and one of R⁷ and R⁸ in the formula A is a hydrogen atom and the other is a C₁-C₆ alkyl group.

9. The compound according to any one of claims 1 to 8 or a pharmaceutically acceptable salt thereof, wherein, in the general formula (I), one of R⁵ and R⁶ is a hydrogen atom and the other is formula A, or both R⁵ and R⁶ are hydrogen atoms, wherein R⁹ in the formula A indicates a C₁-C₆ alkyl group, a C₃-C₆ cycloalkyl group, a 4-oxanyl group, or a 3-oxanyl group.

10. The compound according to claim 1 or a pharmaceutically acceptable salt thereof, wherein, in the general formula (I),
X indicates an oxygen atom;
R¹ indicates a phenyl group which may have 1 to 2 substituents independently selected from substituent group a1:
substituent group a1: a halogen atom, a C₁-C₆ alkyl group, and a C₁-C₆ alkyl group substituted with 1 to 3 halogen atoms;
R² indicates a phenyl group optionally substituted with 1 to 2 halogen atoms;
R³ indicates a hydrogen atom;
R⁴ indicates a hydrogen atom;
one of R⁵ and R⁶ is a hydrogen atom and the other is formula A, or both R⁵ and R⁶ are hydrogen atoms,
wherein one of R⁷ and R⁸ in the formula A indicates a hydrogen atom, the other indicates a C₁-C₃ alkyl group, and R⁹ indicates a C₁-C₆ alkyl group,
or a pharmaceutically acceptable salt thereof.

11. The compound according to claim 1 or a pharmaceutically acceptable salt thereof, wherein, in the general formula (I),
X indicates an oxygen atom;
R¹ indicates a 3-fluorophenyl group;
R² indicates an unsubstituted phenyl group;
R³ indicates a hydrogen atom;
R⁴ indicates a hydrogen atom or a C₁-C₆ alkyl group which may have 1 to 3 substituents independently selected from substituent group c1 below, or is taken together with R⁵ or R⁶ to represent a methylene, methylmethylene, dimethylmethylene or isopropylmethylene group,
substituent group c1: a hydroxyl group, a mono-C₁-C₆ alkylamino group, a di-(C₁-C₆) amino group, a morpholino group, and a medoxomil group (5-methyl-2-oxo-1,3-dioxol-4-yl group);
one of R⁵ and R⁶ is a hydrogen atom and the other is formula A, or both R⁵ and R⁶ are hydrogen atoms,
wherein one of R⁷ and R⁸ in the formula A indicates a hydrogen atom and the other indicates a C₁-C₆ alkyl group, and R⁹ indicates a C₁-C₆ alkyl group, a C₃-C₆ cycloalkyl group, a 4-oxanyl group, or a 3-oxanyl group,
or a pharmaceutically acceptable salt thereof.

12. The compound according to claim 1 or a pharmaceutically acceptable salt thereof, wherein the compound is any one selected from the following group:
(S)-2-Amino-3-(3-((3-(3-fluorophenoxy)-3-(4-fluorophenyl)azetidin-1-yl) sulfonyl)phenyl)propanoic acid;
(S)-2-Amino-3-(3-((3-(4-fluorophenoxy)-3-phenylazetidin-1-yl)sulfonyl)phenyl)propanoic acid;
(S)-2-Amino-3-(3-((3-(3-fluorophenoxy)-3-phenylazetidin-1-yl)sulfonyl)phenyl)propanoic acid;
(2S)-3-(3-((3-(4-fluorophenoxy)-3-phenylazetidin-1-yl)sulfonyl)phenyl)-2-(((1-(pivaloyloxy)ethoxy)carbonyl)amino)propanoic acid;
(S)-2-((((R)-1-acetoxyethoxy)carbonyl)amino)-3-(3-((3-(3-fluorophenoxy)-3-phenylazetidin-1-yl)sulfonyl)phenyl)propanoic acid;
(S)-2-((((R)-1-acetoxyethoxy)carbonyl)amino)-3-(3-((3-(4-fluorophenoxy)-3-phenylazetidin-1-yl)sulfonyl)phenyl)propanoic acid;
(S)-2-((((S)-1-acetoxyethoxy)carbonyl)amino)-3-(3-((3-(4-fluorophenoxy)-3-phenylazetidin-1-yl)sulfonyl)phenyl)propanoic acid;
(S)-3-(3-((3-(3-fluorophenoxy)-3-phenylazetidin-1-yl)sulfonyl)phenyl)-2-((((R)-1-(pivaloyloxy)ethoxy)carbonyl)amino)propanoic acid;
(S)-3-(3-((3-(3-fluorophenoxy)-3-phenylazetidin-1-yl)sulfonyl)phenyl)-2-((((S)-1-(pivaloyloxy)ethoxy)carbonyl)amino)propanoic acid;
(S)-3-(3-((3-(3-fluorophenoxy)-3-(4-fluorophenyl)azetidin-1-yl)sulfonyl)phenyl)-2-((((R)-1-(pivaloyloxy)ethoxy)carbonyl)amino)propanoic acid;
(S)-2-((((S)-1-acetoxyethoxy)carbonyl)amino)-3-(3-((3-(3-fluorophenoxy)-3-phenylazetidin-1-yl)sulfonyl)phenyl)propanoic acid;
(S)-3-(3-((3-(4-fluorophenoxy)-3-phenylazetidin-1-yl)sulfonyl)phenyl)-2-((((S)-1-(isobutyryloxy)ethoxy)carbonyl)amino)propanoic acid;
(S)-3-(3-((3-(4-fluorophenoxy)-3-phenylazetidin-1-yl)sulfonyl)phenyl)-2-((((R)-1-(isobutyryloxy)ethoxy)carbonyl)amino)propanoic acid;
(S)-3-(3-((3-(3-fluorophenoxy)-3-phenylazetidin-1-yl)sulfonyl)phenyl)-2-((((R)-1-(isobutyryloxy)ethoxy)carbonyl)amino)propanoic acid; and
(S)-3-(3-((3-(3-fluorophenoxy)-3-phenylazetidin-1-yl)sulfonyl)phenyl)-2-((((S)-1-(isobutyloxy)ethoxy)carbonyl)amino)propanoic acid.

13. The compound according to claim 1 or a pharmaceutically acceptable salt thereof, wherein the compound is (S)-2-amino-3-(3-((3-(4-fluorophenoxy)-3-phenylazetidin-1-yl)sulfonyl)phenyl)propanoic acid.

14. The compound according to claim 1 or a pharmaceutically acceptable salt thereof, wherein the compound is (S)-2-amino-3-(3-((3-(3-fluorophenoxy)-3-phenylazetidin-1-yl)sulfonyl)phenyl)propanoic acid.

15. The compound according to claim 1 or a pharmaceutically acceptable salt thereof, wherein the compound is (2S)-3-(3-((3-(4-fluorophenoxy)-3-phenylazetidin-1-yl)sulfonyl)phenyl)-2-(((1-(pivaloyloxy)ethoxy)carbonyl)amino)propanoic acid.

16. The compound according to claim 1 or a pharmaceutically acceptable salt thereof, wherein the compound is (S)-2-((((R)-1-acetoxyethoxy)carbonyl)amino)-3-(3-((3-(3-fluorophenoxy)-3-phenylazetidin-1-yl)sulfonyl)phenyl)propanoic acid.

17. The compound according to claim 1 or a pharmaceutically acceptable salt thereof, wherein the compound is (S)-3-(3-((3-(3-fluorophenoxy)-3-phenylazetidin-1-yl)sulfonyl)phenyl)-2-((((S)-1-(pivaloyloxy)ethoxy)carbonyl)amino)propanoic acid.

18. The compound according to claim 1 or a pharmaceutically acceptable salt thereof, wherein the compound is (S)-3-(3-((3-(4-fluorophenoxy)-3-phenylazetidin-1-yl)sulfonyl)phenyl)-2-((((S)-1-(isobutyryloxy)ethoxy)carbonyl)amino)propanoic acid.

19. The compound according to claim 1 or a pharmaceutically acceptable salt thereof, wherein the compound is (S)-3-(3-((3-(4-fluorophenoxy)-3-phenylazetidin-1-yl)sulfonyl)phenyl)-2-((((R)-1-(isobutyryloxy)ethoxy)carbonyl)amino)propanoic acid.

20. A medicament comprising the compound according to any one of claims 1 to 19 or a pharmaceutically acceptable salt thereof.

21. An activated T cell-proliferation suppressor comprising the compound according to any one of claims 1 to 19 or a pharmaceutically acceptable salt thereof.

22. An interferon α-production suppressor comprising the compound according to any one of claims 1 to 19 or a pharmaceutically acceptable salt thereof.

23. A therapeutic or prophylactic agent for a disease whose symptoms are prevented, ameliorated or reduced by suppressing proliferation of activated T cells, or suppressing production of interferon α by activated plasmacytoid dendritic cells (pDC), the agent comprising the compound according to any one of claims 1 to 19 or a pharmaceutically acceptable salt thereof.

24. A method for treating or preventing a disease whose symptoms are prevented, ameliorated or reduced by suppressing proliferation of activated T cells, or suppressing production of interferon α by activated plasmacytoid dendritic cells (pDC), the method comprising administering the compound according to any one of claims 1 to 19 or a pharmaceutically acceptable salt thereof to a patient.

25. The compound according to any one of claims 1 to 19 or a pharmaceutically acceptable salt thereof for use in the treatment or prevention of a disease whose symptoms are prevented, ameliorated or reduced by suppressing proliferation of activated T cells, or suppressing production of interferon α by activated plasmacytoid dendritic cells (pDC).

26. The therapeutic or prophylactic agent according to claim 23, wherein the disease is an autoimmune disease.

27. The method for treating or preventing according to claim 24, wherein the disease is an autoimmune disease.

28. The compound or a pharmaceutically acceptable salt according to claim 25, wherein the disease is an autoimmune disease.

29. A therapeutic or prophylactic agent for systemic lupus erythematosus; a renal lesion, a central nerve disorder, a pulmonary disorder or vasculitis due to systemic lupus erythematosus; multiple myositis; ulcerative colitis; Sjogren's syndrome; graft-versus-host disease (GVHD) or psoriasis, the agent comprising the compound according to any one of claims 1 to 19 or a pharmaceutically acceptable salt thereof.

30. A method for treating or preventing systemic lupus erythematosus; a renal lesion, a central nerve disorder, a pulmonary disorder or vasculitis due to systemic lupus erythematosus; multiple myositis; ulcerative colitis; Sjogren's syndrome; graft-versus-host disease (GVHD) or psoriasis, the method comprising administering the compound according to any one of claims 1 to 19 or a pharmaceutically acceptable salt thereof to a patient.

31. The compound according to any one of claims 1 to 19 or a pharmaceutically acceptable salt thereof for use in the treatment or prevention of systemic lupus erythematosus; a renal lesion, a central nerve disorder, a pulmonary disorder or vasculitis due to systemic lupus erythematosus; multiple myositis; ulcerative colitis; Sjogren's syndrome; graft-versus-host disease (GVHD) or psoriasis.
